(19) Europäisches Patentamt
European Patent Office
Office européen des brevets

(11) EP 3 197 428 B1

(12) EUROPEAN PATENT SPECIFICATION

(45) Date of publication and mention of the grant of the patent:
**20.02.2019 Bulletin 2019/08**

(21) Application number: **15778234.3**

(22) Date of filing: **25.09.2015**

(51) Int Cl.:
***A61K 8/31*** (2006.01) ***A61K 8/34*** (2006.01)
***A61Q 1/06*** (2006.01) ***A61K 8/81*** (2006.01)
***A61K 8/894*** (2006.01) ***A61Q 1/04*** (2006.01)
***A61K 8/89*** (2006.01) ***A61K 8/892*** (2006.01)
***A61K 8/895*** (2006.01) ***A61K 8/92*** (2006.01)
***A45D 34/04*** (2006.01)

(86) International application number:
**PCT/EP2015/072170**

(87) International publication number:
**WO 2016/046400 (31.03.2016 Gazette 2016/13)**

(54) **EMULSION COMPRISING A SILICONE-BASED DENDRITIC FILM-FORMING POLYMER AND A SILICONE GUM, TREATMENT PROCESS USING THE SAME AND SUITABLE DEVICE**

EMULSION MIT SILIKONBASIERTEM DENDRITISCHEN FILMBILDENDEN POLYMER UND EINEM SILIKONGUMMI, BEHANDLUNGSVERFAHREN MIT VERWENDUNG DAVON UND DAFÜR GEEIGNETE VORRICHTUNG

ÉMULSION COMPRENANT UN POLYMÈRE FILMOGÈNE DENDRITIQUE À BASE DE SILICONE ET UNE GOMME DE SILICONE, PROCÉDÉ DE TRAITEMENT L'UTILISANT ET DISPOSITIF APPROPRIÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.09.2014 FR 1459075**
**25.09.2014 FR 1459090**

(43) Date of publication of application:
**02.08.2017 Bulletin 2017/31**

(73) Proprietor: **L'Oréal**
**75008 Paris (FR)**

(72) Inventors:
- **LAHOUSSE, Florence**
**F-94320 Thiais (FR)**
- **CORDELETTE, Pascaline**
**F-91430 Igny (FR)**

(74) Representative: **Wattremez, Catherine**
**L'Oréal**
**Service DIPI**
**9 Rue Pierre Dreyfus**
**92110 Clichy (FR)**

(56) References cited:
**EP-A1- 1 020 135** **EP-A1- 1 312 280**
**WO-A1-2013/100207** **FR-A1- 2 878 738**
**US-A1- 2004 247 373**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 197 428 B1

## Description

The present invention relates to a care and/or makeup cosmetic composition in the form of an emulsion comprising an aqueous phase, at least one silicone-based dendritic film-forming polymer, at least one non-volatile oil and at least one silicone gum. The present invention also relates to a makeup and/or care process, preferably for the lips, which consists in applying said composition, and also to a device comprising it and suitable for the application thereof.
The development of compositions for making up and/or caring for the lips, in particular fluid compositions such as liquid lipsticks, which are stable and endowed with satisfactory properties in terms of application (glidance on application, ease of spreading and fineness of the deposit), but also in terms of the makeup effect of the deposit on the lips, for instance the absence of migration of the deposit, preferably without becoming tacky, is an ongoing objective.
Generally, formulations corresponding to liquid galenical formulations conventionally comprise oils, which in particular provide gloss, optionally waxes for structuring the compositions, fillers, in particular for thickening the composition, film-forming polymers, and dyestuffs.
These formulations must be easy to apply on the lips, precisely and as a uniform coat. In addition, the deposit is not expected to migrate, which would result in the outline of the lips being made imprecise.
With the conventional lipstick compositions of this type, it is noted that the deposit is relatively thick, thereby giving it a more or less tacky nature, in particular induced by the use of these oils and of the polymers present. This feature may be reflected especially by adhesion of the made-up lips to each other, causing the user an unpleasant sensation in terms of comfort.
Another difficulty encountered with liquid lipsticks lies in the fact that the compositions must be sufficiently fluid to be easily applied, but not too fluid, so as to avoid degrading the stability of the composition (pigment sedimentation) and the ease of application (running). Compositions, in particular liquid compositions, which produce a fine deposit that does not migrate and that has virtually no tacky nature are thus sought. Compositions that are comfortable to deposit without losing staying power are also sought.
At least one of these objectives is achieved by the present invention, which relates to a composition comprising:

a. at least one film-forming polymer chosen from vinyl polymers comprising at least one carbosiloxane dendrimer-based unit, present in an active material content ranging from 1.5% to 15% by weight, relative to the total weight of the composition,
b. at least 2% by weight, relative to the weight of the composition, of at least one non-volatile oil,
c. at least one silicone gum chosen from polyorganosiloxanes with a weight-average molecular mass of greater than or equal to 400000 g/mol.
d. water.

The invention also relates to a process for making up and/or caring for the lips, which consists in applying said composition, and also to a device comprising the composition and suitable for applying said composition.
Application devices that are particularly suited to this composition will be described with reference to the appended drawings, in which:

- Figure 1 is a sectional view of an application device according to a first embodiment;
- Figure 2 is an exploded view of an application device according to a second embodiment;
- Figure 3 is a sectional view of the device of Figure 2.

The film deposited on the lips thus has the advantage of being very thin and, in certain cases, of being virtually imperceptible to the user.
It is also comfortable in the sense that it is supple on the lips and leaves them with great freedom of movement. The comfort is also reflected by the absence of a sensation of dryness, while at the same time conserving good performance qualities in terms of absence of migration of the composition and of staying power of the composition on the lips. It should also be noted that this comfort nature afforded by the deposit is persistent.
In addition, and this represents a very desired advantage, this deposit is not tacky.
Throughout the application, the wording "comprising a" or "containing a" means "comprising at least one" or "containing at least one", unless otherwise specified. The expressions "at least one" and "several" are used without distinction.
Unless otherwise indicated, the limits indicated for a range are included in that range.
The cosmetic composition for making up and/or caring for the lips according to the invention is in the form of an emulsion, especially a water-in-oil or oil-in-water emulsion, preferably in the form of a water-in-oil emulsion.
Advantageously, the composition according to the invention is in the form of a liquid emulsion.
The term "liquid" means a fluid texture, the viscosity of which at 25°C is more particularly between 0.005 and

15 Pa.s, preferably between 0.01 and 10 Pa.s and even more advantageously between 0.05 and 8 Pa.s.

Preferably, the viscosity at 25°C of a composition according to the invention is between 0.1 and 6 Pa.s.

**Protocol for measuring the viscosity:**

The viscosity measurement is generally performed at 25°C, using a Rheomat RM180 viscometer equipped with a No. 2 or 3 spindle, the measurement being performed after 10 minutes of rotation of the spindle in the composition (after which time stabilization of the viscosity and of the spin speed of the spindle are observed), at 200 rpm.

The composition may be in the form of a direct (oil-in-water) or inverse (water-in-oil) emulsion.

According to one preferred embodiment of the invention, the composition is in the form of an inverse (water-in-oil) emulsion.

Irrespective of the sense of the emulsion, the composition according to the invention comprises at least 10% by weight of water and preferably from 20% to 60% by weight, relative to the total weight of the composition.

Other characteristics and details of the invention will emerge more clearly on reading the description and the examples that follow.

**FILM-FORMING POLYMER**

As indicated previously, the composition according to the invention comprises at least one film-forming polymer chosen from vinyl polymers comprising at least one carbosiloxane dendrimer-based unit.

The term "film-forming polymer" is intended to mean a polymer that is capable of forming, by itself or in the presence of an auxiliary film-forming agent, a continuous deposit on a support, in particular on keratin materials.

The vinyl polymer(s) have a backbone and at least one side chain, which comprises a carbosiloxane dendrimer-based unit having a carbosiloxane dendrimer structure.

The term "carbosiloxane dendrimer structure" in the context of the present invention represents a molecular structure bearing branched groups of high molecular masses, said structure having high regularity in the radial direction starting from the bond to the backbone. Such carbosiloxane dendrimer structures are described in the form of a highly branched siloxane-silylalkylene copolymer in Japanese patent application JP 9-171 154.

A vinyl polymer according to the invention may contain carbosiloxane dendrimer-based units that may be represented by the general formula (I) below:

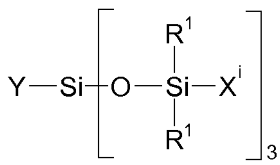
(I)

in which:

- $R^1$ represents an aryl group containing from 5 to 10 carbon atoms or an alkyl group containing from 1 to 10 carbon atoms;
- $X^i$ represents a silylalkyl group which, when i = 1, is represented by formula (II):

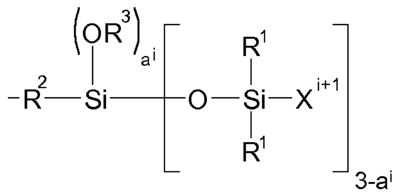
(II)

in which:

- $R^1$ is as defined above in formula (I),
- $R^2$ represents an alkylene radical containing from 2 to 10 carbon atoms,
- $R^3$ represents an alkyl group containing from 1 to 10 carbon atoms,

- $X^{i+i}$ is chosen from: a hydrogen atom, an alkyl group containing from 1 to 10 carbon atoms, an aryl group containing from 5 to 10 carbon atoms and a silylalkyl group defined above of formula (II) with i = i + 1,
- i is an integer from 1 to 10 which represents the generation of said silylalkyl group, and
- $a^i$ is an integer from 0 to 3;

- Y represents a radical-polymerizable organic group chosen from:

  - organic groups containing a methacrylic group or an acrylic group, said organic groups being represented by the formulae:

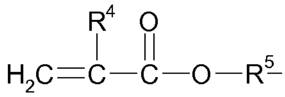

or

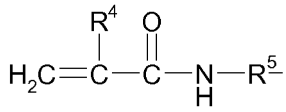

in which:

* $R^4$ represents a hydrogen atom or an alkyl group containing from 1 to 10 carbon atoms; and
* $R^5$ represents an alkylene group containing from 1 to 10 carbon atoms, such as a methylene group, an ethylene group, a propylene group or a butylene group, methylene and propylene groups being preferred; and

  - organic groups containing a styryl group of formula:

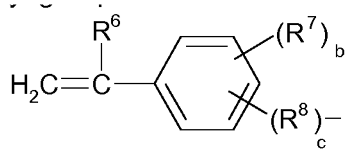

in which:

* $R^6$ represents a hydrogen atom or an alkyl group containing from 1 to 10 carbon atoms, such as a methyl group, an ethyl group, a propyl group or a butyl group, the methyl group being preferred;
* $R^7$ represents an alkyl group containing from 1 to 10 carbon atoms;
* $R^8$ represents an alkylene group containing from 1 to 10 carbon atoms, such as a methylene group, an ethylene group, a propylene group or a butylene group, the ethylene group being preferred;
* b is an integer from 0 to 4; and
* c is 0 or 1, such that, if c is 0, $-(R^8)_c-$ represents a bond.

According to one embodiment, $R^1$ may represent an aryl group containing from 5 to 10 carbon atoms or an alkyl group containing from 1 to 10 carbon atoms. The alkyl group may preferably be represented by a methyl group, an ethyl group, a propyl group, a butyl group, a pentyl group, an isopropyl group, an isobutyl group, a cyclopentyl group or a cyclohexyl group. The aryl group may preferably be represented by a phenyl group and a naphthyl group. The methyl and phenyl groups are more particularly preferred, and the methyl group is preferred among all.

According to one embodiment, $R^2$ represents an alkylene group containing from 2 to 10 carbon atoms, in particular a linear alkylene group, such as an ethylene, propylene, butylene or hexylene group; or a branched alkylene group, such as a methylmethylene, methylethylene, 1-methylpentylene or 1,4-dimethylbutylene group. The ethylene, methylethylene, hexylene, 1-methylpentylene and 1,4-dimethylbutylene groups are preferred among all.

According to one embodiment, $R^3$ is chosen from methyl, ethyl, propyl, butyl and isopropyl groups.

In formula (II), i indicates the number of generations and thus corresponds to the number of repeats of the silylalkyl group.

For example, when the generation number is equal to 1, the carbosiloxane dendrimer may be represented by the general formula shown below, in which Y, $R^1$, $R^2$ and $R^3$ are as defined above, $R^{12}$ represents a hydrogen atom or is identical to $R^1$; $a^1$ is identical to $a^i$. Preferably, the total average number of groups $OR^3$ in a molecule is within the range from 0 to 7.

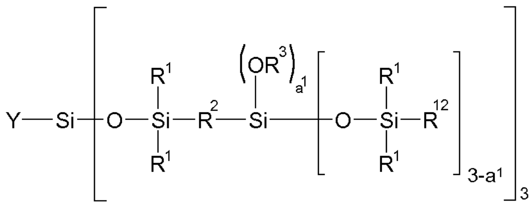

When the generation number is equal to 2, the carbosiloxane dendrimer may be represented by the general formula below, in which Y, $R^1$, $R^2$, $R^3$ and $R^{12}$ are the same as defined above; $a^1$ and $a^2$ represent the $a^i$ of the indicated generation. Preferably, the total average number of groups $OR^3$ in a molecule is within the range from 0 to 25.

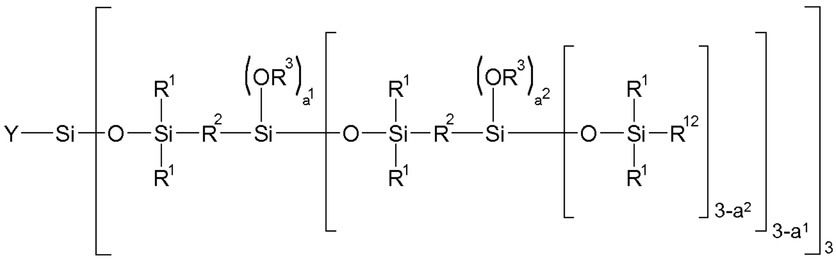

When the generation number is equal to 3, the carbosiloxane dendrimer is represented by the general formula below, in which Y, $R^1$, $R^2$, $R^3$ and $R^{12}$ are the same as defined above; $a^1$, $a^2$ and $a^3$ represent the $a^i$ of the indicated generation. Preferably, the total average number of groups $OR^3$ in a molecule is within the range from 0 to 79.

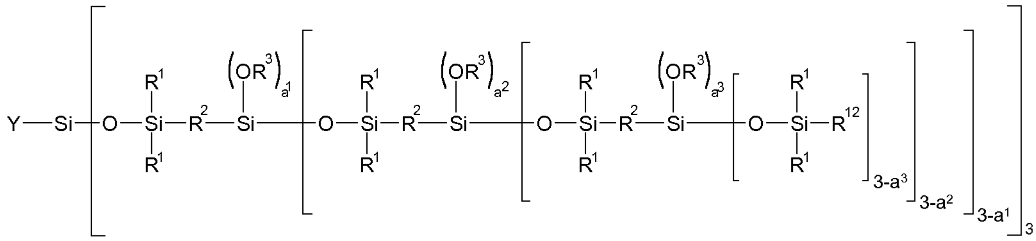

A vinyl polymer bearing at least one carbosiloxane dendrimer-based unit has a molecular side chain containing a carbosiloxane dendrimer structure, and may be derived from the polymerization of:

(A) from 0 to 99.9 parts by weight of a vinyl monomer; and
(B) from 100 to 0.1 parts by weight of a carbosiloxane dendrimer containing a radical-polymerizable organic group, represented by general formula (I) as defined above.

The monomer of vinyl type that is the component (A) in the vinyl polymer bearing at least one carbosiloxane dendrimer-based unit is a monomer of vinyl type that contains a radical-polymerizable vinyl group.
There is no particular limitation as regards such a monomer.
The following are examples of this monomer of vinyl type: methyl methacrylate, ethyl methacrylate, n-propyl methacrylate, isopropyl methacrylate or a methacrylate of lower alkyl analogue; glycidyl methacrylate; butyl methacrylate, butyl acrylate, n-butyl methacrylate, isobutyl methacrylate, tert-butyl acrylate, tert-butyl methacrylate, n-hexyl methacrylate, cyclohexyl methacrylate, 2-ethylhexyl acrylate, 2-ethylhexyl methacrylate, octyl methacrylate, lauryl methacrylate, stearyl acrylate, stearyl methacrylate or a higher methacrylate analogue; vinyl acetate, vinyl propionate or a vinyl ester of a lower fatty acid analogue; vinyl caproate, vinyl 2-ethylhexoate, vinyl laurate, vinyl stearate or a higher fatty acid ester analogue; styrene, vinyltoluene, benzyl methacrylate, phenoxyethyl methacrylate, vinylpyrrolidone or similar vinylaromatic monomers; methacrylamide, N-methylolmethacrylamide, N-methoxymethylmethacrylamide, isobutoxymethoxymethacrylamide, N,N-dimethylmethacrylamide or similar monomers of vinyl type containing amide groups; hydroxyethyl methacrylate, hydroxypropyl alcohol methacrylate or similar monomers of vinyl type containing hydroxyl groups; acrylic acid, methacrylic acid, itaconic acid, crotonic acid, fumaric acid, maleic acid or similar monomers of vinyl type containing a carboxylic acid group; tetrahydrofurfuryl methacrylate, butoxyethyl methacrylate, ethoxydiethylene glycol methacrylate, polyethylene glycol methacrylate, polypropylene glycol monomethacrylate, hydroxybutyl vinyl ether, cetyl

vinyl ether, 2-ethylhexyl vinyl ether or a similar monomer of vinyl type with ether bonds; methacryloxypropyltrimethoxysilane, polydimethylsiloxane containing a methacrylic group on one of its molecular ends, polydimethylsiloxane containing a styryl group on one of its molecular ends, or a similar silicone compound containing unsaturated groups; butadiene; vinyl chloride; vinylidene chloride; methacrylonitrile; dibutyl fumarate; anhydrous maleic acid; anhydrous succinic acid; methacryl glycidyl ether; an organic salt of an amine, an ammonium salt, and an alkali metal salt of methacrylic acid, of itaconic acid, of crotonic acid, of maleic acid or of fumaric acid; a radical-polymerizable unsaturated monomer containing a sulfonic acid group such as a styrenesulfonic acid group; a quaternary ammonium salt derived from methacrylic acid, such as 2-hydroxy-3-methacryloxypropyltrimethylammonium chloride; and a methacrylic acid ester of an alcohol containing a tertiary amine group, such as a methacrylic acid ester of diethylamine.

Multifunctional monomers of vinyl type may also be used. The following represent examples of such compounds: trimethylolpropane trimethacrylate, pentaerythrityl trimethacrylate, ethylene glycol dimethacrylate, tetraethylene glycol dimethacrylate, polyethylene glycol dimethacrylate, 1,4-butanediol dimethacrylate, 1,6-hexanediol dimethacrylate, neopentyl glycol dimethacrylate, trimethylolpropanetrioxyethyl methacrylate, tris(2-hydroxyethyl)isocyanurate dimethacrylate, tris(2-hydroxyethyl)isocyanurate trimethacrylate, polydimethylsiloxane capped with styryl groups containing divinylbenzene groups on both ends, or similar silicone compounds containing unsaturated groups.

A carbosiloxane dendrimer, which is the component (B), may be represented by formula (I) as defined above.

The following represent the preferred examples of group Y of formula (I): an acryloxymethyl group, a 3-acryloxypropyl group, a methacryloxymethyl group, a 3-methacryloxypropyl group, a 4-vinylphenyl group, a 3-vinylphenyl group, a 4-(2-propenyl)phenyl group, a 3-(2-propenyl)phenyl group, a 2-(4-vinylphenyl)ethyl group, a 2-(3-vinylphenyl)ethyl group, a vinyl group, an allyl group, a methallyl group and a 5-hexenyl group.

A carbosiloxane dendrimer according to the present invention may be represented by the formulae having the average structures below:

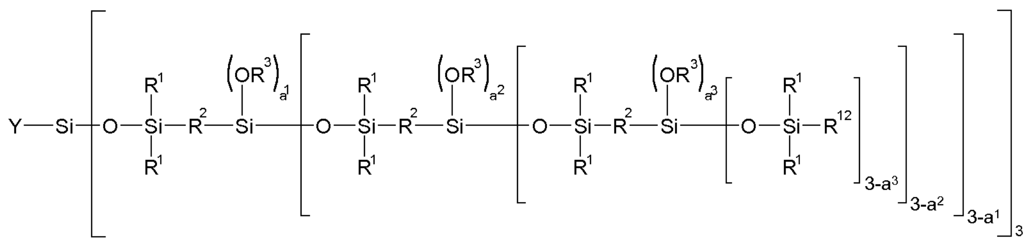

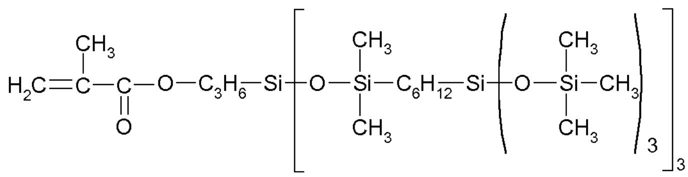

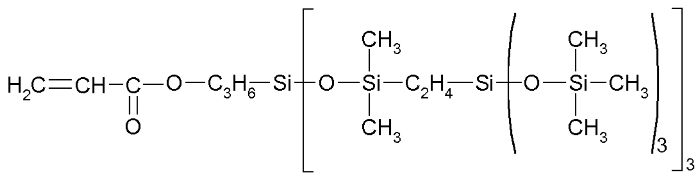

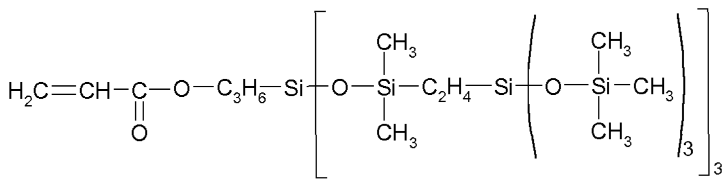

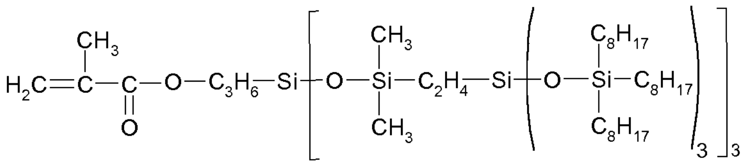

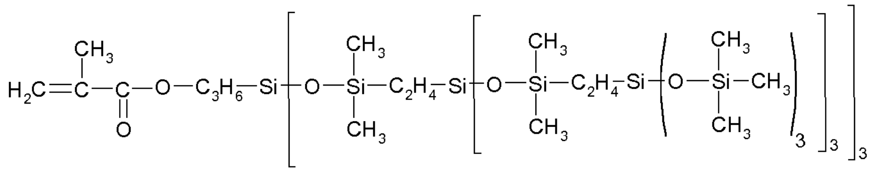

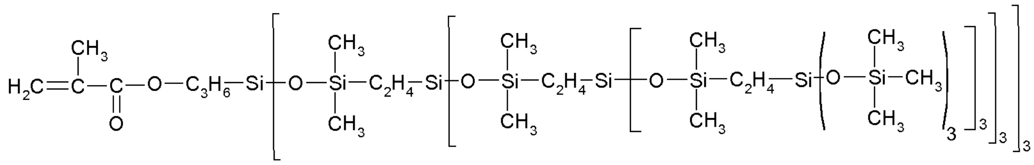

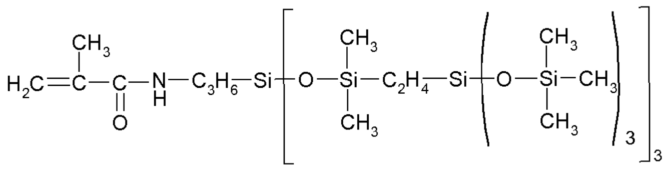

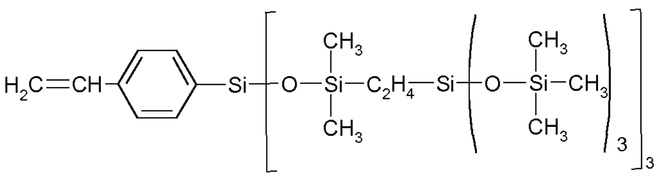

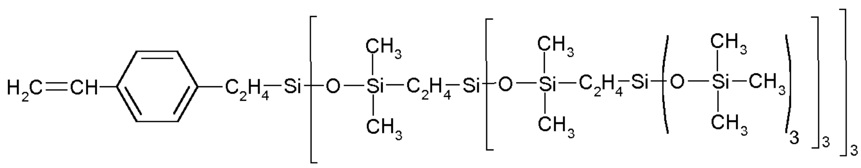

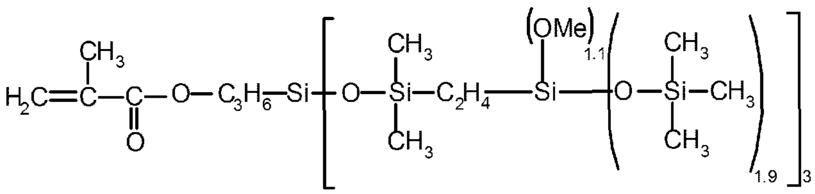

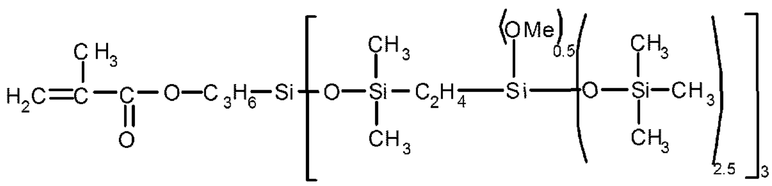

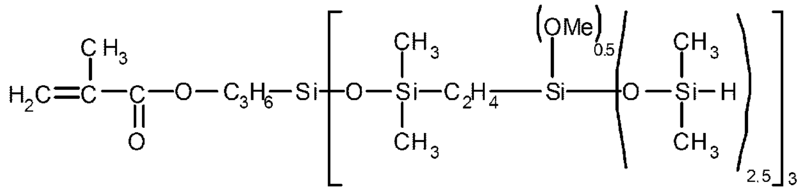

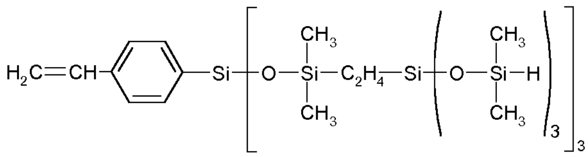

Thus, according to one embodiment, the carbosiloxane dendrimer of the composition according to the present invention is represented by the following formula:

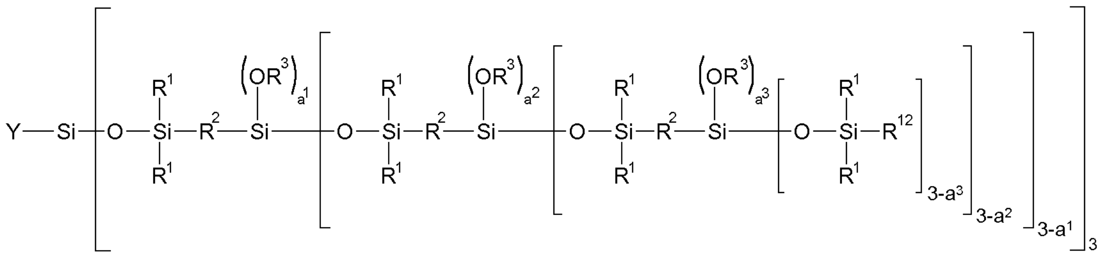

in which:

- Y, $R^1$, $R^2$ and $R^3$ are as defined in formulae (I) and (II) above;
- $a^1$, $a^2$ and $a^3$ correspond to the definition of $a^i$ according to formula (II); and
- $R^{12}$ is H, an aryl group containing from 5 to 10 carbon atoms or an alkyl group containing from 1 to 10 carbon atoms.

According to one embodiment, the carbosiloxane dendrimer of the composition according to the present invention is represented by one of the following formulae:

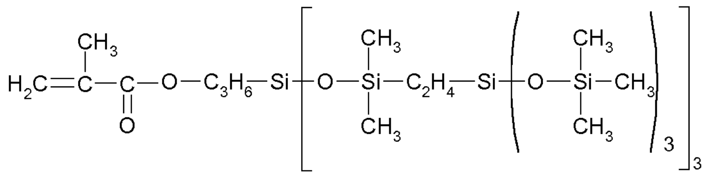

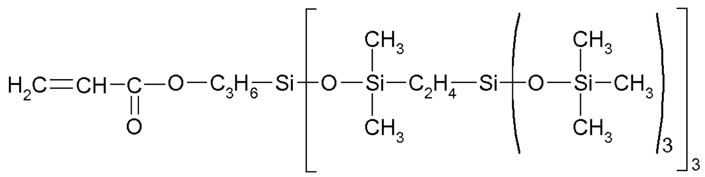

The vinyl polymer comprising the carbosiloxane dendrimer according to the invention may be manufactured according to the process for manufacturing a branched silalkylene siloxane described in Japanese patent application Hei 9-171 154.

For example, it may be produced by subjecting an organosilicon compound containing a hydrogen atom linked to a silicon atom, represented by the following general formula (IV):

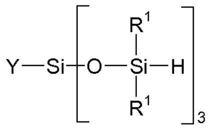
(IV)

Y and $R^1$ being as defined above in formula (I),
and an organosilicon compound containing an alkenyl group, to a hydrosilylation reaction.

In the above formula, the organosilicon compound may be represented by 3-methacryloxypropyltris(dimethylsiloxy)silane, 3-acryloxypropyltris(dimethylsiloxy)silane and 4-vinylphenyltris(dimethylsiloxy)silane. The organosilicon compound that contains an alkenyl group may be represented by vinyltris(trimethylsiloxy)silane, vinyltris(dimethylphenylsiloxy)silane, and 5-hexenyltris(trimethylsiloxy)silane.

The hydrosilylation reaction is performed in the presence of a chloroplatinic acid, a complex of vinylsiloxane and of platinum, or a similar transition metal catalyst.

A vinyl polymer bearing at least one carbosiloxane dendrimer-based unit may be chosen from polymers such that the carbosiloxane dendrimer-based unit is a carbosiloxane dendritic structure represented by formula (III):

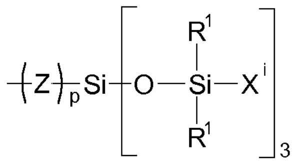
(III)

in which

- Z is a divalent organic group,
- p is 0 or 1,
- $R^1$ is as defined above in formula (IV) and
- $X^i$ is a silylalkyl group represented by formula (II) as defined above.

In a vinyl polymer bearing at least one carbosiloxane dendrimer-based unit, the polymerization ratio between the components (A) and (B), in terms of the weight ratio between (A) and (B), is within a range from 0/100 to 99.9/0.1, or even from 0.1/99.9 to 99.9/0.1 and preferably within a range from 1/99 to 99/1. A ratio between the components (A) and (B) of 0/100 means that the compound becomes a homopolymer of component (B).

A vinyl polymer bearing at least one carbosiloxane dendrimer-based unit may be obtained by copolymerization of the components (A) and (B), or by polymerization of the component (B) alone.

The polymerization may be a free-radical polymerization or an ionic polymerization, but free-radical polymerization is preferred.

The polymerization may be performed by bringing about a reaction between the components (A) and (B) in a solution for a period of from 3 to 20 hours in the presence of a radical initiator at a temperature of from 50°C to 150°C.

A suitable solvent for this purpose is hexane, octane, decane, cyclohexane or a similar aliphatic hydrocarbon; benzene, toluene, xylene or a similar aromatic hydrocarbon; diethyl ether, dibutyl ether, tetrahydrofuran, dioxane or ethers; acetone, methyl ethyl ketone, methyl isobutyl ketone, diisobutyl ketone or similar ketones; methyl acetate, ethyl acetate, butyl acetate, isobutyl acetate or similar esters; methanol, ethanol, isopropanol, butanol or similar alcohols; octamethylcyclotetrasiloxane, decamethylcyclopentasiloxane, hexamethyldisiloxane, octamethyltrisiloxane or a similar organosiloxane oligomer.

A radical initiator may be any compound known in the art for standard free-radical polymerization reactions. The specific examples of such radical initiators are 2,2'-azobis(isobutyronitrile), 2,2'-azobis(2-methylbutyronitrile), 2,2'-azobis(2,4-dimethylvaleronitrile) or similar compounds of azobis type; benzoyl peroxide, lauroyl peroxide, tert-butyl peroxybenzoate, tert-butyl peroxy-2-ethylhexanoate or a similar organic peroxide. These radical initiators may be used alone or in a combination of two or more. The radical initiators may be used in an amount of from 0.1 to 5 parts by weight per 100 parts by weight of the components (A) and (B). A chain-transfer agent may be added. The chain-transfer agent may be 2-mercaptoethanol, butyl mercaptan, n-dodecyl mercaptan, 3-mercaptopropyltrimethoxysilane, a polydimethylsiloxane containing a mercaptopropyl group or a similar compound of mercapto type; methylene chloride, chloroform, carbon tetrachloride, butyl bromide, 3-chloropropyltrimethoxysilane or a similar halogenated compound.
In the manufacture of the polymer of vinyl type, after the polymerization, the unreacted residual vinyl monomer may be

removed under conditions of heating under vacuum.

To facilitate the preparation of starting material for cosmetic products, the number-average molecular weight of the vinyl polymer bearing a carbosiloxane dendrimer may be chosen within the range between 3000 and 2 000 000 and preferably between 5000 and 800 000. It may be a liquid, a gum, a paste, a solid, a powder, or any other form. The preferred forms are solutions consisting of the dilution of a dispersion or of a powder in solvents.

The vinyl polymer may be a dispersion of a polymer of vinyl type bearing a carbosiloxane dendrimer structure in its side molecular chain, in a liquid such as a silicone oil, an organic oil, an alcohol or water.

The silicone oil may be a dimethylpolysiloxane having the two molecular ends capped with trimethylsiloxy groups, a copolymer of methylphenylsiloxane and of dimethylsiloxane having the two molecular ends capped with trimethylsiloxy groups, a copolymer of methyl-3,3,3-trifluoropropylsiloxane and of dimethylsiloxane having the two molecular ends capped with trimethylsiloxy groups, or similar unreactive linear silicone oils, and also hexamethylcyclotrisiloxane, octamethylcyclotetrasiloxane, decamethylcyclopentasiloxane, dodecamethylcyclohexasiloxane or a similar cyclic compound. In addition to the unreactive silicone oils, modified polysiloxanes containing functional groups such as silanol groups, amino groups and polyether groups on the ends or within the molecular side chains may be used.

The organic oils may be isododecane, liquid paraffin, isoparaffin, hexyl laurate, isopropyl myristate, myristyl myristate, cetyl myristate, 2-octyldodecyl myristate; isopropyl palmitate, 2-ethylhexyl palmitate, butyl stearate, decyl oleate, 2-octyldodecyl oleate, myristyl lactate, cetyl lactate, lanolin acetate, stearyl alcohol, cetostearyl alcohol, oleyl alcohol, avocado oil, almond oil, olive oil, cocoa oil, jojoba oil, gum oil, sunflower oil, soybean oil, camelia oil, squalane, castor oil, cottonseed oil, coconut oil, egg yolk oil, polypropylene glycol monooleate, neopentyl glycol 2-ethylhexanoate or a similar glycol ester oil; triglyceryl isostearate, the triglyceride of a fatty acid of coconut oil, or a similar oil of a polyhydric alcohol ester; polyoxyethylene lauryl ether, polyoxypropylene cetyl ether or a similar polyoxyalkylene ether.

The alcohol may be any type that is suitable for use in combination with a cosmetic product starting material. For example, it may be methanol, ethanol, butanol, isopropanol or similar lower alcohols.

A solution or a dispersion of the alcohol should have a viscosity within the range from 10 to $10^9$ mPa at 25°C. To improve the sensory use properties in a cosmetic product, the viscosity should be within the range from 100 to 5 x $10^8$ mPa.s.

The solutions and dispersions may be readily prepared by mixing a vinyl polymer bearing at least one carbosiloxane dendrimer-based unit with a silicone oil, an organic oil, an alcohol or water. The liquids may be present in the polymerization step. In this case, the unreacted residual vinyl monomer should be completely removed by heat treatment of the solution or dispersion under atmospheric pressure or reduced pressure.

In the case of a dispersion, the dispersity of the polymer of vinyl type may be improved by adding a surfactant.

Such an agent may be hexylbenzenesulfonic acid, octylbenzenesulfonic acid, decylbenzenesulfonic acid, dodecylbenzenesulfonic acid, cetylbenzenesulfonic acid, myristylbenzenesulfonic acid or anionic surfactants of the sodium salts of these acids; octyltrimethylammonium hydroxide, dodecyltrimethylammonium hydroxide, hexadecyltrimethylammonium hydroxide, octyldimethylbenzylammonium hydroxide, decyldimethylbenzylammonium hydroxide, dioctadecyldimethylammonium hydroxide, beef tallow-trimethylammonium hydroxide, coconut oil-trimethylammonium hydroxide, or a similar cationic surfactant; a polyoxyalkylene alkyl ether, a polyoxyalkylenealkylphenol, a polyoxyalkylene alkyl ester, the sorbitol ester of polyoxyalkylene, polyethylene glycol, polypropylene glycol, an ethylene oxide additive of diethylene glycol trimethylnonanol, and nonionic surfactants of polyester type, and also mixtures.

In the dispersion, a mean particle diameter of the polymer of vinyl type may be within a range of between 0.001 and 100 microns and preferably between 0.01 and 50 microns. The reason for this is that, outside the recommended range, a cosmetic product mixed with the emulsion will not have a nice enough feel on the lips or to the touch, nor sufficient spreading properties nor a pleasant feel.

A vinyl polymer contained in the dispersion or the solution may have a concentration within a range of between 0.1% and 95% by weight and preferably between 5% and 85% by weight. However, to facilitate the handling and the preparation of the mixture, the range should preferably be between 10% and 75% by weight.

A vinyl polymer that is suitable for use in the invention may also be one of the polymers described in the examples of patent application EP 0 963 751.

According to one preferred embodiment, a vinyl polymer grafted with a carbosiloxane dendrimer may be the product of polymerization of:

(A1) from 0 to 99.9 part by weight of one or more acrylate or methacrylate monomers; and

(B1) from 100 to 0.1 part by weight of an acrylate or methacrylate monomer of a tris[tri(trimethylsiloxy)silylethyldimethylsiloxy]silylpropyl carbosiloxane dendrimer.

The monomers (A1) and (B1) correspond respectively to specific monomers (A) and (B).

According to one embodiment, a vinyl polymer bearing at least one carbosiloxane dendrimer-based unit may comprise a tris[tri(trimethylsiloxy)silylethyldimethylsiloxy]silylpropyl carbosiloxane dendrimer-based unit corresponding

to one of the formulae:

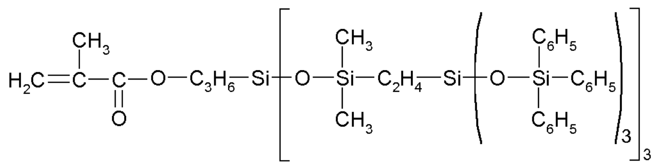

or

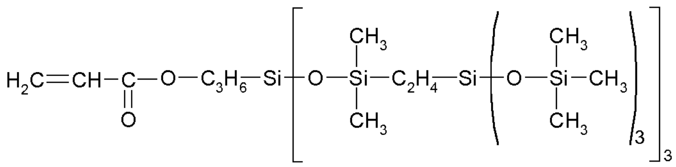

According to one preferred mode, a vinyl polymer bearing at least one carbosiloxane dendrimer-based unit used in the invention comprises at least one butyl acrylate monomer.

According to one embodiment, a vinyl polymer may also comprise at least one fluoro organic group.

Structures in which the polymerized vinyl units constitute the backbone and carbosiloxane dendritic structures and also fluoro organic groups are attached to side chains are particularly preferred.

The fluoro organic groups may be obtained by replacing with fluorine atoms all or some of the hydrogen atoms of methyl, ethyl, propyl, isopropyl, butyl, isobutyl, tert-butyl, pentyl, neopentyl, hexyl, cyclohexyl, heptyl, octyl, nonyl, decyl, undecyl, dodecyl, tridecyl, tetradecyl, hexadecyl and octadecyl groups and other alkyl groups of 1 to 20 carbon atoms, and also alkyloxyalkylene groups of 6 to 22 carbon atoms.

The groups represented by the formula $-(CH_2)_x-(CF_2)_y-R^{13}$ are suggested as examples of fluoroalkyl groups obtained by substituting fluorine atoms for hydrogen atoms of alkyl groups. In the formula, the index "x" is 0, 1, 2 or 3, and "y" is an integer from 1 to 20. $R^{13}$ is an atom or a group chosen from a hydrogen atom, a fluorine atom, $-CH(CF_3)_2-$ or $CF(CF_3)_2$. Such fluorine-substituted alkyl groups are exemplified by linear or branched polyfluoroalkyl or perfluoroalkyl groups represented by the formulae shown below:
$-CF_3$, $-C_2F_5$, $-nC_3F_7$, $-CF(CF_3)_2$, $-nC_4F_9$, $CF_2CF(CF_3)_2$, $-nC_5F_{11}$, $-nC_6F_{13}$, $-nC_8F_{17}$, $CH_2CF_3$, $-(CH(CF_3)_2$, $CH_2CH(CF_3)_2-CH_2(CF_2)_2F$, $-CH_2(CF_2)_3F$, $-CH_2(CF_2)_4F, CH_2(CF_2)_6F$, $CH_2(CF_2)_8F$, $-CH_2CH_2CF_3$, $-CH_2CH_2(CF_2)_2F$, $-CH_2CH_2(CF_2)_3F$, $-CH_2CH_2(CF_2)_4F$, $-CH_2CH_2(CF_2)_6F$, $-CH_2CH_2(CF_2)_8F$, $-CH_2CH_2(CF_2)_{10}F$, $-CH_2CH_2(CF_2)_{12}F$, $CH_2CH_2(CF_2)_{14}F$, $-CH_2CH_2(CF_2)_{16}F$, $-CH_2CH_2CH_2CF_3$, $-CH_2CH_2CH_2(CF_2)_2F$, $-CH_2CH_2CH_2(CF_2)_2H$, $-CH_2(CF_2)_4H$ and $-CH_2CH_2(CF_2)_3H$.

The groups represented by $-CH_2CH_2-(CF_2)_m-CFR^{14}-[OCF_2CF(CF_3)]_n-OC_3F_7$ are suggested as fluoroalkyloxyfluoroalkylene groups obtained by substituting fluorine atoms for hydrogen atoms of alkyloxyalkylene groups. In the formula, the index "m" is 0 or 1, "n" is 0, 1, 2, 3, 4 or 5, and $R^{14}$ is a fluorine atom or $CF_3$. Such fluoroalkyloxyfluoroalkylene groups are exemplified by the perfluoroalkyloxyfluoroalkylene groups represented by the formulae shown below: $-CH_2CH_2CF(CF_3)-[OCF_2CF(CF_3)]_n-OC_3F_7$, $-CH_2CH_2CF_2CF_2-[OCF_2CF(CF_3)]_n-OC_3F_7$.

The number-average molecular weight of the vinyl polymer used in the present invention may be between 3000 and 2 000 000 and more preferably between 5000 and 800 000.

This type of fluorinated vinyl polymer may be obtained by addition:

- of a vinyl monomer (M2) without a fluoro organic group,
- on a vinyl monomer (M1) containing fluoro organic groups, and
- a carbosiloxane dendrimer (B) as defined above, of general formula (I) as defined above, by subjecting them to a copolymerization.

Thus, according to one embodiment, a composition of the invention may comprise a vinyl polymer bearing at least one carbosiloxane dendrimer-based unit and being derived from the copolymerization of a vinyl monomer (M1) as defined above, optionally of a vinyl monomer (M2) as defined above, and of a carbosiloxane dendrimer (B) as defined above, said vinyl polymer having a copolymerization ratio between the monomer (M1) and the monomer (M2) of from 0.1% to 100%/99.9% to 0% by weight, and a copolymerization ratio between the sum of the monomers (M1) and (M2) and the monomer (B) of from 0.1% to 99.9%/99.9% to 0.1% by weight.

The vinyl monomers (M1) containing fluoro organic groups in the molecule are preferably monomers represented

by the general formula:

$(CH^2)=CR^{15}COOR^f$.

In this formula, $R^{15}$ is a hydrogen atom or a methyl group and $R^f$ is a fluoro organic group exemplified by the fluoroalkyl and fluoroalkyloxyfluoroalkylene groups described above. The compounds represented by the formulae presented below are suggested as specific examples of the component (M1). In the formulae present below, "z" is an integer from 1 to 4.

$CH_2$=$CCH_3COO$-$CF_3$, $CH_2$=$CCH_3COO$-$C_2F_5$, $CH_2$=CCH3COO-n$C_3F_7$, $CH_2$=$CCH_3COO$-CF($CF_3$)$_2$, $CH_2$=$CCH_3COO$-n$C_4F_9$, $CH_2$=$CCH_3COO$-CF($CF_3$)$_2$, $CH_2$=$CCH_3COO$-n$C_5F_{11}$, $CH_2$=$CCH_3COO$-n$C_6F_{13}$, $CH_2$=$CCH_3COO$-n$C_8F_{17}$, $CH_2$=$CCH_3COO$-$CH_2CF_3$, $CH_2$=$CCH_3COO$-CH($CF_3$)$_2$, $CH_2$=$CCH_3COO$-$CH_2$CH($CF_3$)$_2$, $CH_2$=$CCH_3COO$-$CH_2$ ($CF_2$)$_2$F, $CH_2$=$CCH_3COO$-$CH_2$($CF_2$)$_2$F, $CH_2$=$CCH_3COO$-$CH_2$($CF_2$)$_4$F, $CH_2$=$CCH_3COO$-$CH_2$($CF_2$)$_6$F, $CH_2$=$CCH_3COO$-$CH_2$($CF_2$)$_8$F, $CH_2$=$CCH_3COO$-$CH_2CH_2CF_3$, $CH_2$=$CCH_3COO$-$CH_2CH_2$($CF_2$)$_2$F, $CH_2$=$CCH_3COO$-$CH_2CH_2$($CF_2$)$_3$F, $CH_2$=$CCH_3COO$-$CH_2CH_2$($CF_2$)$_4$F, $CH_2$=$CCH_3COO$-$CH_2CH_2$($CF_2$)$_6$F, $CH_2$=$CCH_3COO$-$CH_2CH_2$($CF_2$)$_8$F, $CH_2$=$CCH_3COO$-$CH_2CH_2$($CF_2$)$_{10}$F, $CH_2$=$CCH_3COO$-$CH_2CH_2$($CF_2$)$_{12}$F, $CH_2$=$CCH_3COO$-$CH_2CH_2$($CF_2$)$_{14}$F, $CH_2$=$CCH_3COO$-$CH_2$-$CH_2$-($CF_2$)$_{16}$F, $CH_2$=$CCH_3COO$-$CH_2CH_2CH_2CF_3$, $CH_2$=$CCH_3COO$-$CH_2CH_2CH_2$($CF_2$)$_2$F, $CH_2$=$CCH_3COO$-$CH_2CH_2CH_2$($CF_2$)$_2$H, $CH_2$=$CCH_3COO$-$CH_2$($CF_2$)$_4$H, $CH_2$=$CCH_3COO$-($CF_2$)$_3$H, $CH_2$=$CCH_3COO$-$CH_2CH_2$CF($CF_3$)-[O$CF_2$-CF($CF_3$)]z-O$C_3F_7$, $CH_2$=$CCH_3COO$-$CH_2CH_2CF_2CF_2$-[O$CF_2$-CF($CF_3$)]z-O$C_3F_7$, $CH_2$=CH-COO-$CF_3$, CH2=CHCOO-$C_2F_5$, $CH_2$=CHCOO-n$C_3F_7$, $CH_2$=CHCOO-CF($CF_3$)$_2$, $CH_2$=CHCOO-n$C_4F_9$, $CH_2$=CHCOO-$CF_2$CF($CF_3$)$_2$, $CH_2$=CHCOO-n$C_5F_{11}$, $CH_2$=CHCOO-n$C_6F_{13}$, $CH_2$=CHCOO-n$C_8F_{17}$, $CH_2$=CHCOO-$CH_2CF_3$, $CH_2$=CH-COO-CH($CF_3$)$_2$, $CH_2$=CHCOO-$CH_2$CH($CF_3$)$_2$, $CH_2$=CHCOO-$CH_2$($CF_2$)$_2$F, $CH_2$=CHCOO-$CH_2$($CF_2$)$_3$F, $CH_2$=CHCOO-$CH_2$($CF_2$)$_4$F, $CH_2$=CHCOO-$CH_2$($CF_2$)$_6$F, $CH_2$=CHCOO-$CH_2$($CF_2$)$_8$F, $CH_2$=CHCOO-$CH_2CH_2CF_3$, $CH_2$=CHCOO-$CH_2CH_2$ ($CF_2$)$_2$F, $CH_2$=CHCOO-$CH_2CH_2$($CF_2$)$_3$F, $CH_2$=CHCOO-$CH_2CH_2$($CF_2$)$_4$F, $CH_2$=CHCOO-$CH_2CH_2$($CF_2$)$_6$F, $CH_2$=CHCOO-$CH_2CH_2$($CF_2$)$_8$F, $CH_2$=HCOO-$CH_2CH_2$($CF_2$)$_{10}$F, $CH_2$-CHCOO-$CH_2CH_2$-($CF_2$)$_{12}$F, $CH_2$=CHCOO-$CH_2CH_2$($CF_2$)$_{14}$F, $CH_2$=CHCOO-$CH_2CH_2$($CF_2$)$_{16}$F, $CH_2$=CHCOO-$CH_2CH_2CH_2CF_3$, $CH_2$=CHCOO-$CH_2CH_2CH_2$($CF_2$)$_2$F, $CH_2$=CHCOO-$CH_2CH_2CH_2$(CF)$_2$H, $CH_2$=CHCOO-$CH_2$($CF_2$)$_4$H, $CH_2$=CHCOO-$CH_2CH_2$($CF_2$)$_3$H, $CH_2$=CHCOO-$CH_2CH_2$CF($CF_3$)-, [O$CF_2$-CF($CF_3$)]$_z$-O$C_3F_7$, $CH_2$=CHCOO-$CH_2CH_2CF_2CF_2$($CF_3$)-[O$CF_2$-CF($CF_3$)]$_2$-O$C_3F_7$.

Among these, the vinyl polymers represented below are preferred:

$CH_2$=CHCOO-$CH_2CH_2$($CF_2$)$_6$F, $CH_2$=CHCOO-$CH_2CH_2$($CF_2$)$_8$F, $CH_2$=$CCH_3COO$-$CH_2CH_2$($CF_2$)$_6$F, $CH_2$=$CCH_3COO$-$CH_2CH_2$($CF_2$)$_8$F, $CH_2$=CHCOO-$CH_2CF_3$, $CH_2$=$CCH_3COO$-$CH_2CF_3$.

The vinyl polymers represented by the formulae presented below are particularly preferred:

$CH_2$=CHCOO-$CH_2CF_3$,
$CH_2$=CCHCOO-$CH_2CF_3$.

The vinyl monomers (M2) not containing any fluoro organic groups in the molecule may be any monomers containing radical-polymerizable vinyl groups which are exemplified, for example, by methyl acrylate, methyl methacrylate, ethyl acrylate, ethyl methacrylate, n-propyl acrylate, n-propyl methacrylate, isopropyl acrylate, isopropyl methacrylate, and other lower alkyl acrylates or methacrylates; glycidyl acrylate, glycidyl methacrylate; n-butyl acrylate, n-butyl methacrylate, isobutyl acrylate, isobutyl methacrylate, tert-butyl acrylate, tert-butyl methacrylate, n-hexyl acrylate, n-hexyl methacrylate, cyclohexyl acrylate, cyclohexyl methacrylate, 2-ethylhexyl acrylate, 2-ethylhexyl methacrylate, octyl acrylate, octyl methacrylate, lauryl acrylate, lauryl methacrylate, stearyl acrylate, stearyl methacrylate, and other higher acrylates and methacrylates; vinyl acetate, vinyl propionate and other lower fatty acid vinyl esters; vinyl butyrate, vinyl caproate, vinyl 2-ethylhexanoate, vinyl laurate, vinyl stearate, and other higher fatty acid esters; styrene, vinyltoluene, benzyl acrylate, benzyl methacrylate, phenoxyethyl acrylate, phenoxyethyl methacrylate, vinylpyrrolidone, and other vinyl aromatic monomers; dimethylaminoethyl acrylate, dimethylaminoethyl methacrylate, diethylaminoethyl acrylate, diethylaminoethyl methacrylate, and other aminovinyl monomers, acrylamide, methacrylamide, N-methylolacrylamide, N-methylolmethacrylamide, N-methoxymethylacrylamide, N-methoxymethylmethacrylamide, isobutoxymethoxyacrylamide, isobutoxymethoxymethacrylamide, N,N-dimethylacrylamide, N,N-dimethylmethacrylamide, and other vinylamide monomers; hydroxyethyl acrylate, hydroxyethyl methacrylate, acrylic acid hydroxypropyl alcohol, methacrylic acid hydroxypropyl alcohol, and other hydroxyvinyl monomers; acrylic acid, methacrylic acid, itaconic acid, crotonic acid, fumaric acid, maleic acid, and other vinylcarboxylic acid monomers; tetrahydrofurfuryl acrylate, tetrahydrofurfuryl methacrylate, butoxyethyl acrylate, butoxyethyl methacrylate, ethoxydiethylene glycol acrylate, ethoxydiethylene glycol methacrylate, polyethylene glycol acrylate, polyethylene glycol methacrylate, polypropylene glycol monoacrylate, polypropylene glycol monomethacrylate, hydroxybutyl vinyl ether, cetyl vinyl ether, 2-ethylhexyl vinyl ether, and other vinyl monomers containing ether bonds; acryloxypropyltrimethoxysilane, methacryloxypropyltrimethoxysilane, polydimethylsiloxanes containing acryl or methacryl groups at one of the ends, polydimethylsiloxanes containing alkenylaryl groups at one of the

ends and other silicone compounds containing unsaturated groups; butadiene; vinyl chloride; vinylidene chloride, acrylonitrile, methacrylonitrile; dibutyl fumarate; maleic anhydride; dodecylsuccinic anhydride; acryl glycidyl ether, methacryl glycidyl ether, 3,4-epoxycyclohexylmethyl acrylate, 3,4-epoxycyclohexylmethyl methacrylate, alkali metal salts, ammonium salts and organic amine salts of acrylic acid, of methacrylic acid, of itaconic acid, of crotonic acid, of fumaric acid, of maleic acid and of other radical-polymerizable unsaturated carboxylic acids, radical-polymerizable unsaturated monomers containing sulfonic acid groups, such as styrene sulfonic acid and also the alkali metal salts thereof, the ammonium salts thereof and the organic amine salts thereof; the quaternary ammonium salts derived from acrylic acid or methacrylic acid, such as 2-hydroxy-3-methacryloxypropyltrimethylammonium chloride, methacrylic acid esters of a tertiary amine alcohol, such as the diethylamine ester of methacrylic acid and quaternary ammonium salts thereof.

In addition, it is also possible to use as vinyl monomers (M2) the polyfunctional vinyl monomers illustrated, for example, by trimethylolpropane triacrylate, trimethylolpropane trimethacrylate, pentaerythrityl triacrylate, pentaerythrityl trimethacrylate, ethylene glycol diacrylate, ethylene glycol dimethacrylate, tetraethylene glycol diacrylate, tetraethylene glycol dimethacrylate, polyethylene glycol diacrylate, polyethylene glycol dimethacrylate, 1,4-butanediol diacrylate, 1,4-butanediol dimethacrylate, 1,6-hexanediol diacrylate, 1,6-hexanediol dimethacrylate, neopentyl glycol diacrylate, neopentyl glycol dimethacrylate, trimethylolpropanetrioxyethyl acrylate, trimethylolpropanetrioxyethyl methacrylate, tris(2-hydroxyethyl)isocyanurate diacrylate, tris(2-hydroxyethyl)isocyanurate dimethacrylate, tris(2-hydroxyethyl)isocyanurate triacrylate, tris(2-hydroxyethyl)isocyanurate trimethacrylate, polydimethylsiloxane in which the two ends of the molecular chain are blocked with alkenylaryl groups, and other silicone compounds containing unsaturated groups.

As regards the ratio mentioned above in which (M1) and (M2) are copolymerized, the weight ratio between (M1) and (M2) is preferably within the range 1:99 to 100:0.

Y can be chosen, for example, from organic groups containing acrylic or methacrylic groups, organic groups containing an alkenylaryl group, or alkenyl groups containing from 2 to 10 carbon atoms.

The organic groups containing acrylic or methacrylic groups and the alkenylaryl groups are as defined above.

Among the compounds (B), mention may, for example, be made of the following compounds:

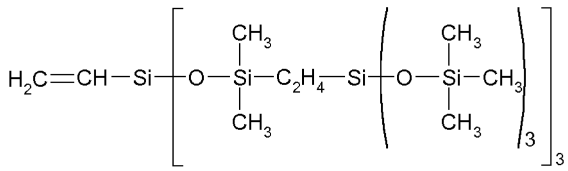

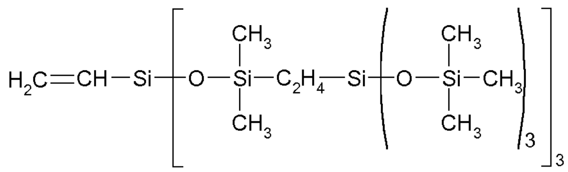

The carbosiloxane dendrimers (B) may be prepared using the process for preparing siloxane/silalkylene branched copolymers described in document EP 1 055 674.

For example, they may be prepared by subjecting organic alkenyl silicone compounds and silicone compounds comprising hydrogen atoms bonded to the silicon, represented by formula (IV) as defined above, to a hydrosilylation reaction.

The copolymerization ratio (by weight) between the monomer (B) and the monomers (M1) and (M2) is preferably within the range of 1:99 to 99:1 and even more preferably within the range of 5:95 to 95:5.

Amino groups may be introduced into the side chains of the vinyl polymer by using, included in component (M2), vinyl monomers containing amino groups, such as dimethylaminoethyl acrylate, dimethylaminoethyl methacrylate, diethylaminoethyl acrylate and diethylaminoethyl methacrylate, followed by performing a modification with potassium acetate monochloride, ammonium acetate monochloride, the aminomethylpropanol salt of monochloroacetic acid, the triethanolamine salt of monobromoacetic acid, sodium monochloropropionate, and other alkali metal salts of halogenated fatty acids.

Alternatively, carboxylic acid groups may be introduced into the side chains of the vinyl polymer by using, included in component (M2), vinyl monomers containing carboxylic acids, such as acrylic acid, methacrylic acid, itaconic acid, crotonic acid, fumaric acid and maleic acid, and the like, followed by neutralizing the product with triethylamine, diethylamine, triethanolamine and other amines.

A fluorinated vinyl polymer may be one of the polymers described in the examples of patent application WO 03/045 337.

According to one preferred embodiment, a vinyl polymer grafted in the sense of the present invention may be conveyed in an oil or a mixture of oils, which is/are preferably volatile, chosen in particular from silicone oils and hydrocarbon-based oils, and mixtures thereof.
According to one particular embodiment, a silicone oil that is suitable for use in the invention may be cyclopentasiloxane.
According to another particular embodiment, a hydrocarbon-based oil that is suitable for use in the invention may be isododecane.
Vinyl polymers grafted with at least one carbosiloxane dendrimer-based unit that may be particularly suitable for use in the present invention are the polymers sold under the names TIB 4-100, TIB 4-101, TIB 4-120, TIB 4-130, TIB 4-200, FA 4002 ID (TIB 4-202), TIB 4-220 and FA 4001 CM (TIB 4-230) by the company Dow Corning.
According to one embodiment, the composition according to the present invention comprises at least one vinyl polymer bearing at least one carbosiloxane dendrimer-based unit in an active material content ranging from 1,5 % to 15 % by weight, in particular 1.5% to 10% by weight and preferably from 3% to 5% by weight, relative to the total weight of the composition.

## NON-VOLATILE OIL

The composition according to the invention also comprises at least one non-volatile oil.
The term "non-volatile oil" means an oil whose vapour pressure at 25°C and atmospheric pressure is non-zero and is less than 0.02 mmHg (2.66 Pa) and better still less than $10^{-3}$ mmHg (0.13 Pa).
As indicated previously, the composition comprises at least one non-volatile oil in a content of at least 2% by weight, preferably ranging from 2% to 30% by weight, more particularly from 3% to 30% by weight, in particular from 3% to 15% by weight, and preferably between 4% and 15% by weight relative to the weight of the composition.
According to one embodiment, the composition comprises at least one non-volatile oil in a content of at least 5% by weight, preferably ranging from 10% to 70% by weight and in particular from 25% to 60% by weight relative to the total weight of volatile and non-volatile oil(s) present in said composition.
In accordance with a particularly advantageous embodiment, at least one of the non-volatile oils is compatible with the film-forming polymer, where appropriate conveyed in an oil or a mixture of oils, which are preferably volatile, used in the composition according to the invention. This especially enables to further improve the stability of the composition.
To check this compatibility, several mixtures of the non-volatile oil to be tested were made with the film-forming polymer, where appropriate conveyed in at least one oil, preferably a volatile oil: 30/70; 50/50; 70/30 (the ratios being expressed by weight, each mixture representing 10 g).
Each mixture is prepared at 25°C with stirring (magnetic bar) for one hour.
If the resulting mixture appears to be one-phase, this mixture is observed with a phase-contrast microscope. If there is no sign of one phase dispersed in the other, then said non-volatile oil and the film-forming polymer in its vehicle (if present) are said to be compatible in the evaluated mixture.
Non-volatile oils that are compatible in all the tested mixtures are more particularly suitable for use. Use may also be made of non-volatile oils that are compatible for some of the tested mixtures, if these oils are used in the composition in a content in which they are compatible. If necessary, on the basis of the results obtained previously, one or more tests may be repeated to check the compatibility at the envisaged content.
Preferably, use is made of at least one non-volatile oil that is compatible with the film-forming polymer/vehicle if present, for the three mixtures described previously.
The non-volatile oil(s) may be chosen from polar or apolar hydrocarbon-based oils, silicone oils and fluoro oils, and mixtures thereof.

### Polar non-volatile hydrocarbon-based oils

The term "*hydrocarbon-based oil*" means an oil formed essentially from, or even consisting of, carbon and hydrogen atoms, and possibly oxygen and nitrogen atoms, and not containing any silicon or fluorine atoms.
It may contain alcohol, ester, ether, carboxylic acid, amine and/or amide groups.
Preferably, the hydrocarbon-based oil, in addition to being free of silicon and fluorine, is free of heteroatoms such as N, S and P. The hydrocarbon-based oil is therefore different from a silicone oil and from a fluoro oil.
In the present case, the non-volatile hydrocarbon-based oil comprises at least one oxygen atom.
In particular, this non-volatile hydrocarbon-based oil comprises at least one alcohol function (it is then an "alcohol oil") and/or at least one ester function (it is then an "ester oil").
The ester oils that may be used in the compositions according to the invention may in particular be hydroxylated.
The composition may comprise one or more non-volatile hydrocarbon-based oils, in particular chosen from:

- $C_{10}$-$C_{26}$ alcohols, preferably monoalcohols.

More particularly, the $C_{10}$-$C_{26}$ alcohols are saturated or unsaturated, and branched or unbranched, and comprise from 10 to 26 carbon atoms.
Preferably, the $C_{10}$-$C_{26}$ alcohols are fatty alcohols, which are preferably branched when they comprise at least 16 carbon atoms.
As examples of fatty alcohols that may be used according to the invention, mention may be made of linear or branched fatty alcohols, of synthetic origin or alternatively of natural origin, for instance alcohols derived from plant material (coconut, palm kernel, palm, etc.) or animal material (tallow, etc.).
Needless to say, other long-chain alcohols may also be used, for instance ether alcohols or alternatively "Guerbet" alcohols.
Finally, use may also be made of certain more or less long fractions of alcohols of natural origin, for instance coconut ($C_{12}$ to $C_{16}$) or tallow ($C_{16}$ to $C_{18}$) or compounds of diol or cholesterol type.
Use is preferably made of a fatty alcohol comprising from 10 to 24 carbon atoms and more preferentially from 12 to 22 carbon atoms.
As particular examples of fatty alcohols that may preferably be used, mention may be made especially of lauryl alcohol, myristyl alcohol, isostearyl alcohol, palmityl alcohol, oleyl alcohol, 2-butyloctanol, 2-undecylpentadecanol, 2-hexyldecyl alcohol, isocetyl alcohol and octyldodecanol, and mixtures thereof.
According to one advantageous embodiment of the invention, the alcohol is chosen from octyldodecanol.

- optionally hydroxylated monoesters, diesters or triesters of a $C_2$-$C_8$ monocarboxylic or polycarboxylic acid and of a $C_2$-$C_8$ alcohol.

In particular:

* optionally hydroxylated monoesters of a $C_2$-$C_8$ carboxylic acid and of a $C_2$-$C_8$ alcohol,
* optionally hydroxylated diesters of a $C_2$-$C_8$ dicarboxylic acid and of a $C_2$-$C_8$ alcohol, such as diisopropyl adipate, 2-diethylhexyl adipate, dibutyl adipate, diisostearyl adipate or 2-diethylhexyl succinate,
* optionally hydroxylated triesters of a $C_2$-$C_8$ tricarboxylic acid and of a $C_2$-$C_8$ alcohol, such as citric acid esters, such as trioctyl citrate, triethyl citrate, acetyl tributyl citrate, tributyl citrate or acetyl tributyl citrate;

- esters of a $C_2$-$C_8$ polyol and of one or more $C_2$-$C_8$ carboxylic acids, such as glycol diesters of monoacids, such as neopentyl glycol diheptanoate, or glycol triesters of monoacids, such as triacetin;

- ester oils, in particular having between 18 and 70 carbon atoms.

Examples that may be mentioned include monoesters, diesters or triesters.
The ester oils may be hydroxylated or non-hydroxylated.
The non-volatile ester oil may for example be chosen from:

* monoesters comprising between 18 and 40 carbon atoms in total, in particular the monoesters of formula $R_1COOR_2$ in which $R_1$ represents a saturated or unsaturated, linear or branched or aromatic fatty acid residue comprising from 4 to 40 carbon atoms and $R_2$ represents a hydrocarbon-based chain, which is in particular branched, containing from 4 to 40 carbon atoms, on condition that $R_1 + R_2 \geq 18$, for instance Purcellin oil (cetostearyl octanoate), isononyl isononanoate, $C_{12}$ to $C_{15}$ alkyl benzoate, 2-ethylhexyl palmitate, octyldodecyl neopentanoate, 2-octyldodecyl stearate, 2-octyldodecyl erucate, isostearyl isostearate, 2-octyldodecyl benzoate, alcohol or polyalcohol octanoates, decanoates or ricinoleates, isopropyl myristate, isopropyl palmitate, butyl stearate, hexyl laurate, 2-ethylhexyl palmitate, 2-hexyldecyl laurate, 2-octyldecyl palmitate or 2-octyldodecyl myristate.

Preferably, they are esters of formula $R_1COOR_2$ in which $R_1$ represents a linear or branched fatty acid residue containing from 4 to 40 carbon atoms and $R_2$ represents a hydrocarbon-based chain that is in particular branched, containing from 4 to 40 carbon atoms, $R_1$ and $R_2$ being such that $R_1 + R_2 \geq 18$.
Even more particularly, the ester comprises between 18 and 40 carbon atoms in total.
Preferred monoesters that may be mentioned include isononyl isononanoate, oleyl erucate and/or 2-octyldodecyl neopentanoate.

* monoesters of a fatty acid, in particular of 18 to 22 carbon atoms, and in particular of lanolic acid, oleic acid, lauric acid or stearic acid, and of diols, for instance propylene glycol monoisostearate;

* diesters, in particular comprising between 18 and 60 carbon atoms in total and in particular between 18 and 50 carbon atoms in total. Use may be made especially of diesters of a dicarboxylic acid and of monoalcohols, preferably such as diisostearyl malate, or glycol diesters of monocarboxylic acids, such as neopentyl glycol diheptanoate, propylene glycol dioctanoate, diethylene glycol diisononanoate or polyglyceryl-2 diisostearate (in particular such as the compound sold under the commercial reference Dermol DGDIS by the company Alzo);
* hydroxylated monoesters and diesters, preferably with a total carbon number ranging from 18 to 70, for instance polyglyceryl-3 diisostearate, isostearyl lactate, octyl hydroxystearate, octyldodecyl hydroxystearate, diisostearyl malate or glyceryl stearate;
* triesters, in particular comprising between 35 and 70 carbon atoms in total, in particular such as triesters of a tricarboxylic acid, such as triisostearyl citrate, or tridecyl trimellitate, or glycol triesters of monocarboxylic acids such as polyglyceryl-2 triisostearate;
* tetraesters, in particular with a total carbon number ranging from 35 to 70, such as pentaerythritol or polyglycerol tetraesters of a monocarboxylic acid, for instance pentaerythrityl tetrapelargonate, pentaerythrityl tetraisostearate, pentaerythrityl tetraisononanoate, glyceryl tris(2-decyl)tetradecanoate, polyglyceryl-2 tetraisostearate or pentaerythrityl tetrakis(2-decyl)tetradecanoate;
* polyesters obtained by condensation of an unsaturated fatty acid dimer and/or trimer and of diol, such as those described in patent application FR 0 853 634, in particular such as dilinoleic acid and 1,4-butanediol. Mention may in particular be made in this respect of the polymer sold by Biosynthis under the name Viscoplast 14436H (INCI name: dilinoleic acid/butanediol copolymer), or else copolymers of polyols and of diacid dimers, and esters thereof, such as Hailucent ISDA;
* esters and polyesters of diol dimer and of monocarboxylic or dicarboxylic acid, such as esters of diol dimer and of fatty acid and esters of diol dimer and of dicarboxylic acid dimer, in particular which may be obtained from a dicarboxylic acid dimer derived in particular from the dimerization of an unsaturated fatty acid especially of $C_8$ to $C_{34}$, especially of $C_{12}$ to $C_{22}$, in particular of $C_{16}$ to $C_{20}$ and more particularly of $C_{18}$, such as esters of dilinoleic diacids and of dilinoleic diol dimers, for instance those sold by the company Nippon Fine Chemical under the trade names Lusplan DD-DA5® and DD-DA7®;
* polyesters resulting from the esterification of at least one triglyceride of hydroxylated carboxylic acid(s) with an aliphatic monocarboxylic acid and with an aliphatic dicarboxylic acid, which is optionally unsaturated, for instance the succinic acid and isostearic acid castor oil sold under the reference Zenigloss by Zenitech;
* hydrocarbon-based plant oils such as fatty acid triglycerides (which are liquid at room temperature), especially of fatty acids containing from 7 to 40 carbon atoms, such as heptanoic or octanoic acid triglycerides or jojoba oil; mention may be made in particular of saturated triglycerides such as caprylic/capric triglyceride and mixtures thereof, for example such as the product sold under the reference Myritol 318 from Cognis, glyceryl triheptanoate, glyceryl trioctanoate, and $C_{18-36}$ acid triglycerides such as those sold under the reference Dub TGI 24 by Stéarineries Dubois, and unsaturated triglycerides such as castor oil, olive oil, ximenia oil or pracaxi oil;

- vinylpyrrolidone/1-hexadecene copolymers, for instance the product sold under the name Antaron V-216 (also known as Ganex V216) by the company ISP (MW = 7300 g/mol);

- $C_{12}$-$C_{26}$ fatty acids, preferably $C_{12}$-$C_{22}$ fatty acids, which are preferably unsaturated, such as oleic acid, linoleic acid or linolenic acid, and mixtures thereof;

- dialkyl carbonates, the 2 alkyl chains possibly being identical or different, such as dicaprylyl carbonate sold under the name Cetiol CC® by Cognis;

- and mixtures thereof.

**Apolar non-volatile hydrocarbon-based oils**

The composition according to the invention may also comprise at least one apolar non-volatile hydrocarbon-based oil.

These oils may be of plant, mineral or synthetic origin.

For the purposes of the present invention, the term "apolar oil" means an oil whose solubility parameter at 25°C, $\delta_a$, is equal to 0 $(J/cm^3)^{1/2}$.

The definition and calculation of the solubility parameters in the Hansen three-dimensional solubility space are described in the article by C.M. Hansen: The three-dimensional solubility parameters, J. Paint Technol. 39, 105 (1967). According to this Hansen space, the parameters $\delta_p$, $\delta_h$, $\delta_D$ and $\delta_a$ are expressed in $(J/cm^3)^{½}$ and are defined as follows:

- $\delta_D$ characterizes the London dispersion forces derived from the formation of dipoles induced during molecular impacts;
- $\delta_p$ characterizes the Debye interaction forces between permanent dipoles and also the Keesom interaction forces between induced dipoles and permanent dipoles;
- $\delta_h$ characterizes the specific interaction forces (such as hydrogen bonding, acid/base, donor/acceptor, etc.); and
- $\delta_a$ is determined by the equation: $\delta_a = (\delta_p^2 + \delta_h^2)^{½}$.

Preferably, the non-volatile apolar hydrocarbon-based oil may be chosen from linear or branched hydrocarbons of mineral or synthetic origin, such as:

- liquid paraffin or derivatives thereof (mineral oil),
- squalane,
- isoeicosane,
- naphthalene oil,
- polybutenes such as for instance Indopol H-100 (molar mass or MW = 965 g/mol), Indopol H-300 (MW = 1340 g/mol) and Indopol H-1500 (MW = 2160 g/mol) sold or manufactured by the company Amoco,
- polyisobutenes, hydrogenated polyisobutenes more particularly such as Parleam® sold by the company Nippon Oil Fats, Panalane H-300 E sold or manufactured by the company Amoco (MW = 1340 g/mol), Viseal 20000 sold or manufactured by the company Synteal (MW = 6000 g/mol) and Rewopal PIB 1000 sold or manufactured by the company Witco (MW = 1000 g/mol), or alternatively Parleam Lite sold by NOF Corporation,
- decene/butene copolymers, polybutene/polyisobutene copolymers, especially Indopol L-14,
- polydecenes and hydrogenated polydecenes for instance such as: Puresyn 10 (MW = 723 g/mol) and Puresyn 150 (MW = 9200 g/mol) sold or manufactured by the company Mobil Chemicals, or alternatively Puresyn 6 sold by ExxonMobil Chemical,
- and mixtures thereof.

**Silicone oils**

The term "*silicone oil*" means an oil containing at least one silicon atom, and in particular containing Si-O groups.
Among the non-volatile silicone oils that may be used in the present invention, examples that may be mentioned include non-volatile non-phenyl silicone oils and non-volatile phenyl silicone oils.
The silicone oils that may be used for the purposes of the invention advantageously have a molecular mass of less than or equal to 150 000 g/mol, preferably less than or equal to 100 000 g/mol and better still less than or equal to 10 000 g/mol.

Non-volatile non-phenyl silicone oils

The term "non-phenyl silicone oil" denotes a silicone oil not comprising any phenyl substituents.
Representative examples of these non-volatile non-phenyl silicone oils which may be mentioned include polydimethylsiloxanes; alkyl dimethicones; vinylmethyl methicones; and also silicones modified with aliphatic groups and/or with functional groups such as hydroxyl, thiol and/or amine groups.
It should be noted that "dimethicone" (INCI name) corresponds to a polydimethylsiloxane (chemical name).
The non-volatile non-phenyl silicone oil is preferably chosen from non-volatile dimethicone oils.
In particular, these oils can be chosen from the following non-volatile oils:

- polydimethylsiloxanes (PDMS),
- PDMSs comprising aliphatic groups, in particular alkyl or alkoxy groups, which are pendent and/or at the end of the silicone chain, these groups each comprising from 2 to 24 carbon atoms. By way of example, mention may be made of the cetyl dimethicone sold under the commercial reference Abil Wax 9801 from Evonik Goldschmidt,
- PDMSs comprising aliphatic groups, or functional groups such as hydroxyl, thiol and/or amine groups,
- polyalkylmethylsiloxanes substituted with functional groups such as hydroxyl, thiol and/or amine groups,
- polysiloxanes modified with fatty acids, fatty alcohols or polyoxyalkylenes, and
- mixtures thereof.

Preferably, these non-volatile non-phenyl silicone oils are chosen from polydimethylsiloxanes; alkyl dimethicones and also PDMSs comprising aliphatic groups, in particular $C_2$-$C_{24}$ alkyl groups, and/or functional groups such as hydroxyl, thiol and/or amine groups.
The non-volatile non-phenyl silicone oil may be chosen in particular from silicones of formula (I):

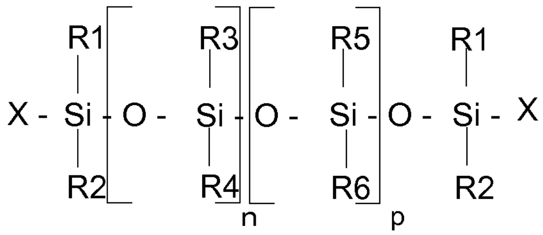

(I)

in which:

- $R_1$, $R_2$, $R_5$ and $R_6$ are, together or separately, an alkyl radical containing from 1 to 6 carbon atoms,
- $R_3$ and $R_4$ are, together or separately, an alkyl radical containing from 1 to 6 carbon atoms, a vinyl radical, an amine radical or a hydroxyl radical,
- X is an alkyl radical containing from 1 to 6 carbon atoms, a hydroxyl radical or an amine radical,
- n and p are integers chosen so as to have a fluid compound, in particular whose viscosity at 25°C is between 8 centistokes (cSt) (8 x $10^{-6}$ $m^2/s$) and 800 000 cSt, and a molecular mass of less than or equal to 150 000 g/mol, preferably less than or equal to 100 000 g/mol and better still less than or equal to 10 000 g/mol.

As non-volatile non-phenyl silicone oils that may be used according to the invention, mention may be made of those for which:

- the substituents $R_1$ to $R_6$ and X represent a methyl group, and p and n are such that the viscosity is 60 000 cSt, for example the product sold under the name Dow Corning 200 Fluid 60 000 CS by the company Dow Corning, and the product sold under the name Wacker Belsil DM 60 000 by the company Wacker,
- the substituents $R_1$ to $R_6$ and X represent a methyl group, and p and n are such that the viscosity is 100 cSt or 350 cSt, for example the products sold respectively under the names Belsil DM100 and Dow Corning 200 Fluid 350 CS by the company Dow Corning, and
- the substituents $R_1$ to $R_6$ represent a methyl group, the group X represents a hydroxyl group, and n and p are such that the viscosity is 700 cSt, for example the product sold under the name Baysilone Fluid T0.7 by the company Momentive.

Non-volatile phenyl silicone oils

The expression "phenyl silicone oil" (or phenylated silicone oil) denotes a silicone oil bearing at least one phenyl substituent.

These non-volatile phenyl silicone oils may be chosen from those also bearing at least one dimethicone fragment, or from those not bearing one. According to the invention, a dimethicone fragment corresponds to the following unit: $-Si(CH_3)_2-O-$.

The non-volatile phenyl silicone oil may thus be chosen from:

- phenyl silicone oils optionally bearing a dimethicone fragment corresponding to formula (I) below:

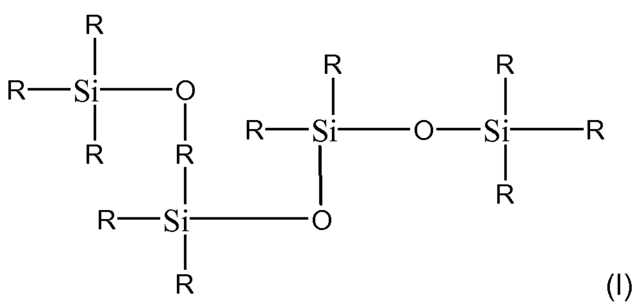

(I)

in which the groups R, which are monovalent or divalent, represent, independently of each other, a methyl, methylene, phenyl or phenylene, with the proviso that at least one group R represents a phenyl.

Preferably, in this formula, the phenyl silicone oil comprises at least three phenyl groups, for example at least four, at least five or at least six.

- phenyl silicone oils optionally bearing a dimethicone fragment corresponding to formula (II) below:

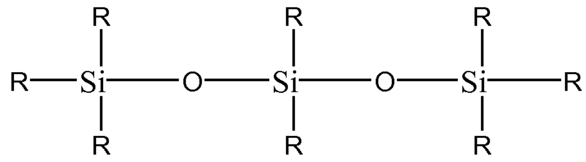

(II)

in which the groups R represent, independently of each other, a methyl or a phenyl, with the proviso that at least one group R represents a phenyl.

Preferably, in this formula, the compound of formula (II) comprises at least three, for example at least four or at least five, phenyl groups.
Mixtures of different phenylorganopolysiloxane compounds described above can be used.
Examples that may be mentioned include mixtures of triphenyl-, tetraphenyl- or pentaphenyl-organopolysiloxanes.
Among the compounds of formula (II), mention may be made more particularly of phenyl silicone oils not bearing any dimethicone fragments, corresponding to formula (II) in which at least 4 or at least 5 radicals R represent a phenyl radical, the remaining radicals representing methyls.
Such non-volatile phenyl silicone oils are preferably trimethylpentaphenyltrisiloxane or tetramethyltetraphenyltrisiloxane. They are in particular sold by Dow Corning under the reference PH-1555 HRI or Dow Corning 555 Cosmetic Fluid (chemical name: 1,3,5-trimethyl-1,1,3,5,5-pentaphenyltrisiloxane; INCI name: trimethylpentaphenyltrisiloxane), or the tetramethyltetraphenyltrisiloxane sold under the reference Dow Corning 554 Cosmetic Fluid by Dow Corning can also be used.
They correspond especially to formulae (III) and (III') below:

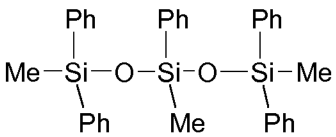

(III)

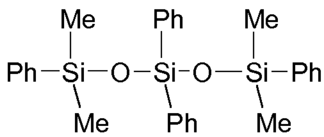

(III')

in which Me represents methyl, and Ph represents phenyl.

- phenyl silicone oils bearing at least one dimethicone fragment corresponding to formula (IV) below:

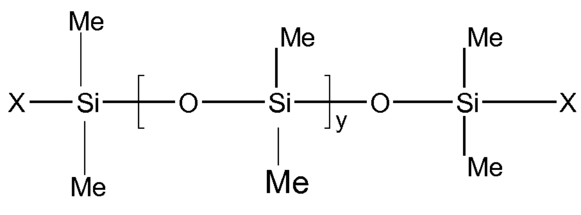

(IV)

in which Me represents methyl, y is between 1 and 1000 and X represents $-CH_2-CH(CH_3)(Ph)$.
- phenyl silicone oils corresponding to formula (V) below, and mixtures thereof:

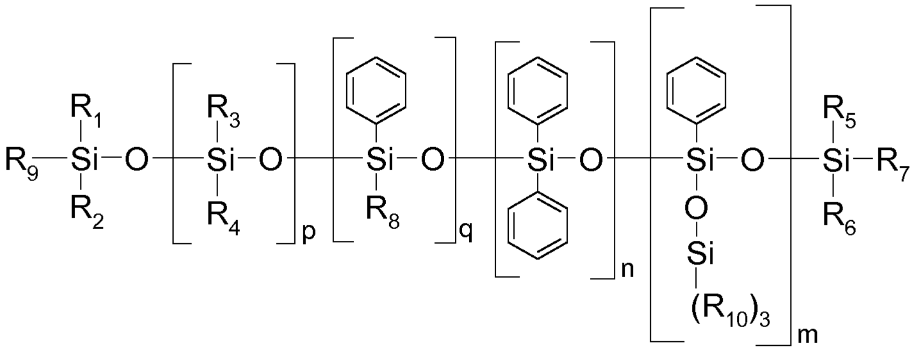

(V)

in which:

- $R_1$ to $R_{10}$, independently of each other, are saturated or unsaturated, linear, cyclic or branched $C_1$-$C_{30}$ hydrocarbon-based radicals,
- m, n, p and q are, independently of each other, integers between 0 and 900, with the proviso that the sum m+n+q is other than 0.

Preferably, the sum m+n+q is between 1 and 100. Advantageously, the sum m+n+p+q is between 1 and 900 and preferably between 1 and 800.
Preferably, q is equal to 0.

More particularly, $R_1$ to $R_{10}$, independently of each other, represent a saturated or unsaturated, preferably saturated, linear or branched $C_1$-$C_{30}$ hydrocarbon-based radical, and in particular a preferably saturated, $C_1$-$C_{20}$, in particular $C_1$-$C_{18}$, hydrocarbon-based radical, or a monocyclic or polycyclic $C_6$-$C_{14}$, and in particular $C_{10}$-$C_{13}$, aryl radical, or an aralkyl radical, the alkyl part of which is preferably $C_1$-$C_3$ alkyl.

Preferably, $R^1$ to $R_{10}$ may each represent a methyl, ethyl, propyl, butyl, isopropyl, decyl, dodecyl or octadecyl radical, or alternatively a phenyl, tolyl, benzyl or phenethyl radical. $R^1$ to $R_{10}$ may in particular be identical, and in addition may be a methyl radical.

As particular embodiments of formula (V), mention may be made of:

o phenyl silicone oils optionally bearing at least one dimethicone fragment corresponding to formula (VI) below, and mixtures thereof:

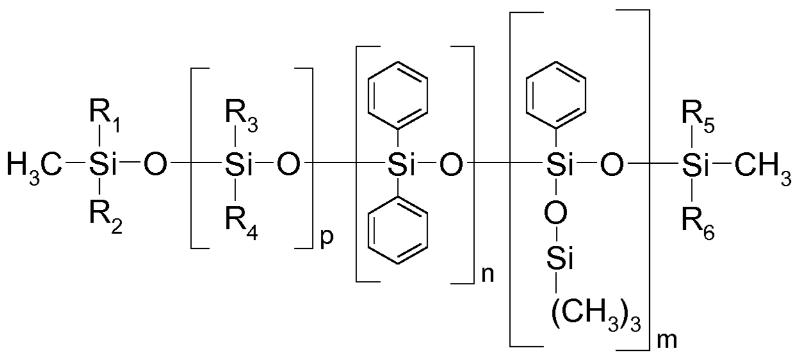

(VI)

in which:

☐ $R_1$ to $R_6$, independently of each other, are saturated or unsaturated, linear, cyclic or branched $C_1$-$C_{30}$ hydrocarbon-based radicals, a preferably $C_6$-$C_{14}$ aryl radical or an aralkyl radical, the alkyl part of which is $C_1$-$C_3$ alkyl,
☐ m, n and p are, independently of each other, integers between 0 and 100, with the proviso that the sum n+m is between 1 and 100.

Preferably, $R_1$ to $R_6$, independently of each other, represent a $C_1$-$C_{20}$, in particular $C_1$-$C_{18}$, hydrocarbon-based,

preferably alkyl, radical, or a $C_6$-$C_{14}$ aryl radical which is monocyclic (preferably $C_6$) or polycyclic and in particular $C_{10}$-$C_{13}$, or an aralkyl radical (preferably the aryl part is $C_6$ aryl; the alkyl part is $C_1$-$C_3$ alkyl).

Preferably, $R_1$ to $R_6$ may each represent a methyl, ethyl, propyl, butyl, isopropyl, decyl, dodecyl or octadecyl radical, or alternatively a phenyl, tolyl, benzyl or phenethyl radical.

$R_1$ to $R_6$ may in particular be identical, and in addition may be a methyl radical. Preferably, m = 1 or 2 or 3, and/or n = 0 and/or p = 0 or 1 can be applied, in formula (VI).

According to one particular embodiment, the non-volatile phenyl silicone oil is chosen from phenyl silicone oils bearing at least one dimethicone fragment.

Preferably, such oils correspond to compounds of formula (VI) in which:

☐ m=0 and n and p are, independently of each other, integers between 1 and 100.

Preferably, $R_1$ to $R_6$ are methyl radicals.

According to this embodiment, the silicone oil is preferably chosen from a diphenyl dimethicone such as KF-54 from Shin-Etsu (400 cSt), KF54HV from Shin-Etsu (5000 cSt), KF-50-300CS from Shin-Etsu (300 cSt), KF-53 from Shin-Etsu (175 cSt) or KF-50-100CS from Shin-Etsu (100 cSt).

☐ p is between 1 and 100, the sum n+m is between 1 and 100, and n=0.

These phenyl silicone oils optionally bear at least one dimethicone fragment correspond more particularly to formula (VII) below:

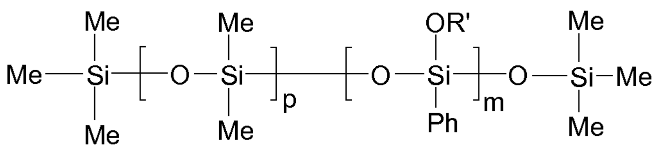

(VII)

in which Me is methyl and Ph is phenyl, OR' represents a group $-OSiMe_3$ and p is 0 or is between 1 and 1000, and m is between 1 and 1000. In particular, m and p are such that the compound (VII) is a non-volatile oil.
According to a first embodiment of non-volatile phenyl silicone bearing at least one dimethicone fragment, p is between 1 and 1000 and m is more particularly such that compound (VII) is a non-volatile oil. Trimethylsiloxyphenyldimethicone, sold in particular under the reference Belsil PDM 1000 by the company Wacker, may, for example, be used.

According to a second embodiment of non-volatile phenyl silicone not bearing a dimethicone fragment, p is equal to 0 and m is between 1 and 1000, and in particular is such that the compound (VII) is a non-volatile oil.

Phenyltrimethylsiloxytrisiloxane, sold in particular under the reference Dow Corning 556 Cosmetic Grade Fluid (DC556), may, for example, be used.

o non-volatile phenyl silicone oils not bearing a dimethicone fragment corresponding to formula (VIII) below, and mixtures thereof:

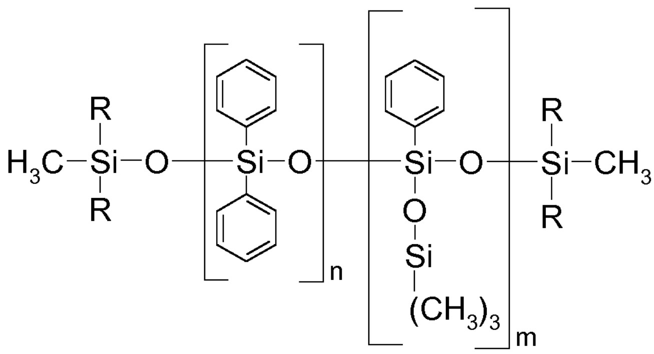

(VIII)

in which:

- R, independently of each other, are saturated or unsaturated, linear, cyclic or branched $C_1$-$C_{30}$ hydrocarbon-based radicals, preferably R is a $C_1$-$C_{30}$ alkyl radical, a preferably $C_6$-$C_{14}$ aryl radical, or an aralkyl radical, the alkyl part of which is $C_1$-$C_3$ alkyl,
- m and n are, independently of each other, integers between 0 and 100, with the proviso that the sum n+m is between 1 and 100.

Preferably, R, independently of each other, represent a saturated or unsaturated, preferably saturated, linear or branched $C_1$-$C_{30}$ hydrocarbon-based radical, and in particular a preferably saturated, $C_1$-$C_{20}$, in particular $C_1$-$C_{18}$ and more particularly $C_4$-$C_{10}$, hydrocarbon-based radical, a monocyclic or polycyclic $C_6$-$C_{14}$, and in particular $C_{10}$-$C_{13}$, aryl radical, or an aralkyl radical of which preferably the aryl part is $C_6$ aryl and the alkyl part is $C_1$-$C_3$ alkyl.
Preferably, the groups R may each represent a methyl, ethyl, propyl, butyl, isopropyl, decyl, dodecyl or octadecyl radical, or alternatively a phenyl, tolyl, benzyl or phenethyl radical.
The groups R may in particular be identical, and in addition may be a methyl radical.
Preferably, m = 1 or 2 or 3, and/or n = 0 and/or p = 0 or 1 can be applied, in formula (VIII).
According to a preferred embodiment, n is an integer between 0 and 100 and m is an integer between 1 and 100, with the proviso that the sum n+m is between 1 and 100, in formula (VIII). Preferably, R is a methyl radical.
According to one embodiment, a phenyl silicone oil of formula (VIII) with a viscosity at 25°C of between 5 and 1500 $mm^2/s$ (i.e. 5 to 1500 cSt), and preferably with a viscosity of between 5 and 1000 $mm^2/s$ (i.e. 5 to 1000 cSt), may be used.
According to this embodiment, the non-volatile phenyl silicone oil is preferably chosen from phenyl trimethicones (when n=0) such as DC556 from Dow Corning (22.5 cSt), or else from diphenylsiloxyphenyl trimethicone oil (when m and n are between 1 and 100) such as KF56 A from Shin-Etsu, or the Silbione 70663V30 oil from Rhône-Poulenc (28 cSt). The values in parentheses represent the viscosities at 25°C.

- phenyl silicone oils optionally bearing at least one dimethicone fragment corresponding to the following formula, and mixtures thereof:

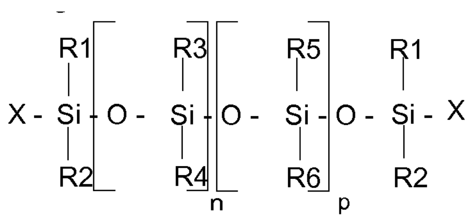

(IX)

in which:

$R_1$, $R_2$, $R_5$ and $R_6$, which may be identical or different, are an alkyl radical containing 1 to 6 carbon atoms,
$R_3$ and $R_4$, which may be identical or different, are an alkyl radical containing from 1 to 6 carbon atoms or an aryl radical (preferably $C_6$-$C_{14}$), with the proviso that at least one of $R_3$ and $R_4$ is a phenyl radical,
X is an alkyl radical containing from 1 to 6 carbon atoms, a hydroxyl radical or a vinyl radical, n and p being an integer greater than or equal to 1, chosen so as to give the oil a weight-average molecular weight preferably less than 150 000 g/mol and more preferably less than 100 000 g/mol.

- and a mixture thereof.

**Non-volatile fluoro oils**

The term "fluoro oil" means an oil containing at least one fluorine atom.
Among the non-volatile fluoro oils that may be used in the present invention, mention may be made in particular of fluorosilicone oils, fluoro polyethers and fluorosilicones especially as described in document EP-A-847 752, and perfluoro compounds.
The term "perfluoro compounds" means compounds in which all the hydrogen atoms have been replaced with fluorine atoms.
According to one embodiment, the fluoro oil is chosen from perfluoro oils. As examples of perfluoro oils, mention may be made of perfluorodecalins and perfluoroperhydrophenanthrenes.

According to one embodiment, the fluoro oil is chosen from perfluoroperhydrophenanthrenes, and especially the Fiflow® products sold by the company Creations Couleurs. In particular, use may be made of the fluoro oil whose INCI name is perfluoroperhydrophenanthrene, sold under the reference Fiflow 220 by the company F2 Chemicals.

The composition according to the invention preferably comprises at least one polar or apolar non-volatile hydrocarbon-based oil, at least one non-volatile silicone oil, which is preferably phenylated, and mixtures thereof. In accordance with an even more particular embodiment, the composition according to the invention comprises at least one non-volatile silicone oil, which is preferably phenylated.

Preferably, said non-volatile phenyl silicone oils are chosen from phenyl silicones not bearing any dimethicone fragments. More particularly, the non-volatile phenyl silicone oils not having a dimethicone fragment are chosen from (I), with radicals R such that the silicone has no dimethicone fragment; (II) with radicals R such that the silicone has no dimethicone fragment, in particular formulae (III) and (III'); (V) with p = 0; (VI) with p=0; (VII) with p=0; (VIII); (IX) with radicals R such that the silicone has no dimethicone fragment; or mixtures thereof.

Furthermore, preferably, the non-volatile phenylated silicone oils are chosen from those of formula (II), more particularly non-volatile phenyl silicone oils of formula (III) or (III').

In addition, the non-volatile hydrocarbon-based oil(s) are more particularly chosen from polar non-volatile oils, such as for example $C_{10}$-$C_{26}$ alcohols, or ester oils; from apolar oils; and mixtures thereof.

Preferably, the composition comprises at least one polar oil chosen from $C_{10}$-$C_{26}$ alcohols; hydroxylated mono-noesters and diesters; monoesters comprising between 18 and 40 carbon atoms in total; triesters comprising between 35 and 70 carbon atoms in total, or mixtures thereof, or at least one apolar oil chosen from liquid paraffin or derivatives thereof, hydrogenated or non-hydrogenated poly(iso)butenes, and also mixtures thereof.

Preferably, the composition comprises at least one polar oil, more particularly octyldodecanol.

## SILICONE GUM

According to one embodiment of the invention, the composition comprises at least one silicone gum chosen from polyorganosiloxanes with a weight-average molecular mass of greater than or equal to 400 000 g/mol.

The weight-average molecular masses are measured in a manner that is conventional in the field, for example using gel permeation chromatography coupled to static light scattering (GPC-MALLS).

Preferably, the viscosity of the silicone gum is greater than 800 000 cSt and more particularly less than or equal to 10 000 000 cSt (at 25°C, measured by standard ASTM D-445), more particularly between 1 000 000 and 5 000 000 cSt (at 25°C, measured by standard ASTM D-445).

The term "silicone gum" means more particularly polyorganosiloxanes, in particular linear non-crosslinked, optionally hydroxylated, phenylated or vinyl polydimethylsiloxanes, or combinations thereof. It should be noted that the silicone gums used according to the invention are not silicone elastomers.

According to a preferred embodiment of the invention, the silicone gum corresponds to the following formula:

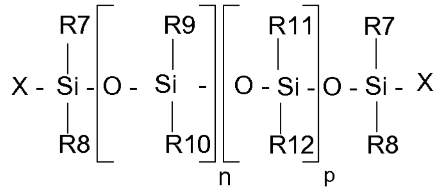

in which:

- R7, R8, R11 and R12 are identical or different, and each is chosen from alkyl radicals comprising from 1 to 6 carbon atoms,
- R9 and R10 are identical or different, and each is chosen from an alkyl radical comprising from 1 to 6 carbon atoms, an aryl radical, a hydroxyl radical, a vinyl radical, preferably an alkyl radical comprising from 1 to 6 carbon atoms, a hydroxyl radical;
- X is chosen from an alkyl radical comprising from 1 to 6 carbon atoms, a hydroxyl radical, a vinyl radical, preferably an alkyl radical comprising from 1 to 6 carbon atoms, a hydroxyl radical;
- n and p are chosen so as to give the silicone gum a molecular mass of greater than or equal to 400 000 g/mol.

In general, n and p may each take values ranging from 0 to 5000 and more particularly from 0 to 3000, given that n and p are not simultaneously zero.

According to a particular embodiment, the silicone gum is a polydimethylsiloxane gum optionally comprising

at least one aryl radical, a dimethiconol gum, or mixtures thereof, and preferably a dimethiconol gum.

The silicone gum(s) may be used alone or as a mixture, especially with a solvent chosen from volatile silicones, polydimethylsiloxane oils, polyphenylmethylsiloxane oils, isoparaffins, in particular of $C_8$-$C_{16}$, methylene chloride, pentane, dodecane, tridecane and tetradecane, or mixtures thereof.

If the silicone gums are sold in predissolved form, the proportion of gum usually represents from 5% to 20% by weight and preferably from 10% to 15% by weight in a linear or cyclic, volatile or non-volatile polydimethylsiloxane of low molecular weight.

Among the silicone gums that may be used, mention may be made of those for which:

- R7 to R12 represent a methyl group, the substituent X represents a hydroxyl group, the coefficients n and p being such that the molecular mass is greater than or equal to 400 000 g/mol:
    - ◦ product sold or manufactured under the name Xiameter® PMX-1401 Fluid by the company Dow Corning, in the form of a solution at 13% in cyclopentasiloxane,
    - ◦ product sold or manufactured under the name Xiameter® PMX-1501 Fluid by the company Dow Corning, in the form of a solution at 14-15 % in cyclopentasiloxane,
    - ◦ product sold or manufactured under the name Xiameter® PMX-1503 Fluid by the company Dow Corning, in the form of a solution at 12% in polydimethylsiloxane,
    - ◦ product sold or manufactured under the name Xiameter® PMX-1403 Fluid by the company Dow Corning, in the form of a solution at 13% in polydimethylsiloxane,
- R7, R8, R11, R12 and X represent a methyl group and the substituents R9 and R10 represent an aryl group, the coefficients n and p being such that the molecular mass is greater than or equal to 400 000 g/mol:
    - ◦ product sold or manufactured under the name Mirasil® C-DPDM by the company Bluestar.

According to a preferred embodiment of the invention, the silicone gum is a polydimethylsiloxane gum optionally comprising at least one aryl radical, a dimethiconol gum, or mixtures thereof, and preferably a dimethiconol gum.

In accordance with a particularly advantageous embodiment of the invention, the silicone gum, where appropriate in predissolved form, is incompatible with the film-forming polymer, the latter being, where appropriate, conveyed in at least one oil, preferably a volatile oil.

To check this absence of compatibility, several mixtures of the silicone gum, where appropriate predissolved, were made with the film-forming polymer, where appropriate conveyed in at least one oil, preferably a volatile oil: 30/70; 50/50; 70/30 (the ratios being expressed by weight, each mixture representing 10 g).

Each mixture is prepared at 25°C with stirring (magnetic bar) for one hour.

If the resulting mixture appears to be one-phase, this mixture is observed with a phase-contrast microscope. If one phase is seen to be dispersed in the other, then said optionally predissolved silicone gum and the film-forming polymer in its vehicle (if present) are said to be incompatible in the evaluated mixture.

Silicone gums that are incompatible in all the tested mixtures are more particularly suitable for use. Use may also be made of gums that are incompatible for some of the tested mixtures, if these gums are used in the composition in a content in which they are incompatible. If necessary, on the basis of the results obtained previously, one or more tests may be repeated to check the compatibility at the envisaged content.

Preferably, use is made of at least one silicone gum that is incompatible with the film-forming polymer/vehicle if present, for the three mixtures described previously.

According to a preferred embodiment, the composition according to the invention comprises at least one silicone gum in an active material content ranging from 0.1% to 10% by weight, preferably from 0.2% to 8% by weight, in particular from 0.3% to 6% by weight, relative to the total weight of the composition.

## SURFACTANTS

According to a particular embodiment of the invention, the composition comprises at least one surfactant.

The content of surfactant(s) usually ranges between 0.05% and 15% by weight and preferably between 0.5% and 10% by weight relative to the weight of the composition.

The suitable surfactant(s) may be chosen from nonionic, anionic, cationic and amphoteric surfactants, and mixtures thereof; and preferably from nonionic or anionic hydrocarbon-based or silicone surfactants, and mixtures thereof.

For the choice of these surfactants, reference may be made to the document "Encyclopaedia of Chemical Technology, Kirk-Othmer", volume 22, pp. 333-432, 3rd edition, 1979, Wiley, for the definition of the properties and functions (emulsifying) of surfactants, in particular pp. 347-377 of this reference, for anionic and nonionic surfactants.

In particular, at least one emulsifying surfactant having at 25°C an HLB (hydrophilic-lipophilic balance) within the Griffin sense of greater than or equal to 8 may be used. The Griffin HLB value is defined in J. Soc. Cosm. Chem. 1954 (volume 5), pages 249-256.

An emulsifying surfactant having at 25°C an HLB (hydrophilic-lipophilic balance) within the Griffin sense of less than 8 may also optionally be used.

A person skilled in the art will select the surfactant(s) as a function of the type of emulsion that he wishes to obtain, a water-in-oil or oil-in-water emulsion, on the basis of his general knowledge.

*Nonionic surfactants*

The nonionic surfactants may be chosen especially from alkyl and polyalkyl esters of poly(ethylene oxide), (poly)oxyalkylenated alcohols, alkyl and polyalkyl ethers of poly(ethylene oxide), optionally polyoxyethylenated alkyl and polyalkyl esters of sorbitan, optionally polyoxyethylenated alkyl and polyalkyl ethers of sorbitan, alkyl and polyalkyl glycosides or polyglycosides, in particular alkyl and polyalkyl glucosides or polyglucosides, alkyl and polyalkyl esters of sucrose, optionally polyoxyethylenated alkyl and polyalkyl esters of glycerol, and optionally polyoxyethylenated alkyl and polyalkyl ethers of glycerol, and mixtures thereof.

1) ($C_8$-$C_{30}$)Alkyl and poly($C_8$-$C_{30}$)alkyl esters of poly(ethylene oxide) that are preferably used are those with a number of ethylene oxide (EO) units ranging from 2 to 200. Examples that may be mentioned include stearate 40 EO, stearate 50 EO, stearate 100 EO, laurate 20 EO, laurate 40 EO and distearate 150 EO.
2) ($C_8$-$C_{30}$)Alkyl and poly($C_8$-$C_{30}$)alkyl ethers of poly(ethylene oxide) that are preferably used are those with a number of ethylene oxide (EO) units ranging from 2 to 200. Examples that may be mentioned include cetyl ether 23 EO, oleyl ether 50 EO, phytosterol 30 EO, steareth 40, steareth 100 and beheneth 100.
3) (Poly)oxyalkylenated, in particular oxyethylenated and/or oxypropylenated, alcohols that are preferably used are those that can comprise from 1 to 150 oxyethylene and/or oxypropylene units, in particular containing from 20 to 100 oxyethylene units, in particular ethoxylated fatty alcohols, in particular of $C_8$-$C_{24}$ and preferably of $C_{12}$-$C_{18}$, such as stearyl alcohol ethoxylated with 20 oxyethylene units (CTFA name Steareth-20), for instance Brij 78 sold by the company Uniqema, cetearyl alcohol ethoxylated with 30 oxyethylene units (CTFA name Ceteareth-30), and the mixture of $C_{12}$-$C_{15}$ fatty alcohols comprising 7 oxyethylene units (CTFA name $C_{12-15}$ Pareth-7), for instance the product sold under the name Neodol 25-7® by Shell Chemicals; or in particular oxyalkylenated (oxyethylenated and/or oxypropylenated) alcohols containing from 1 to 15 oxyethylene and/or oxypropylene units, in particular $C_8$-$C_{24}$ and preferably $C_{12}$-$C_{18}$ fatty alcohols, such as stearyl alcohol ethoxylated with 2 oxyethylene units (CTFA name Steareth-2), for instance Brij 72 sold by the company Uniqema.
4) Optionally polyoxyethylenated ($C_8$-$C_{30}$)alkyl and poly($C_8$-$C_{30}$)alkyl esters of sorbitan that are preferably used are those with a number of ethylene oxide (EO) units ranging from 0 to 100. Examples that may be mentioned include sorbitan laurate 4 or 20 EO, in particular polysorbate 20 (or polyoxyethylene (20) sorbitan monolaurate) such as the product Tween 20 sold by the company Uniqema, sorbitan palmitate 20 EO, sorbitan stearate 20 EO, sorbitan oleate 20 EO, or the Cremophor products (RH 40, RH 60, etc.) from BASF.
5) Optionally polyoxyethylenated ($C_8$-$C_{30}$)alkyl and poly($C_8$-$C_{30}$)alkyl ethers of sorbitan that are preferably used are those with a number of ethylene oxide (EO) units ranging from 0 to 100.
6) Alkyl and polyalkyl glucosides or polyglucosides that are preferably used are those containing an alkyl group comprising from 6 to 30 carbon atoms and preferably from 6 to 18 or even from 8 to 16 carbon atoms, and containing a glucoside group preferably comprising from 1 to 5 and in particular 1, 2 or 3 glucoside units. The alkylpolyglucosides may be chosen, for example, from decylglucoside (alkyl-$C_9$/$C_{11}$-polyglucoside (1.4)), for instance the product sold under the name Mydol 10® by the company Kao Chemicals or the product sold under the name Plantacare 2000 UP® by the company Henkel and the product sold under the name Oramix NS 10® by the company SEPPIC; caprylyl/capryl glucoside, for instance the product sold under the name Plantacare KE 3711® by the company Cognis or Oramix CG 110® by the company SEPPIC; laurylglucoside, for instance the product sold under the name Plantacare 1200 UP® by the company Henkel or Plantaren 1200 N® by the company Henkel; cocoglucoside, for instance the product sold under the name Plantacare 818 UP® by the company Henkel; caprylylglucoside, for instance the product sold under the name Plantacare 810 UP® by the company Cognis; and mixtures thereof.
7) Examples of ($C_8$-$C_{30}$)alkyl and poly($C_8$-$C_{30}$)alkyl esters of sucrose that may be mentioned include Crodesta F150, sucrose monolaurate sold under the name Crodesta SL 40, and the products sold by Ryoto Sugar Ester, for instance sucrose palmitate sold under the reference Ryoto Sugar Ester P1670, Ryoto Sugar Ester LWA1695 or Ryoto Sugar Ester 01570.
8) Optionally polyoxyethylenated ($C_8$-$C_{30}$)alkyl and poly($C_8$-$C_{30}$)alkyl esters of glycerol that are preferably used are those with a number of ethylene oxide (EO) units ranging from 0 to 100 and a number of glycerol units ranging from 1 to 30. Examples that may be mentioned include hexaglyceryl monolaurate, polyglyceryl-4 isostearate, for instance

the product sold under the name Isolan Gl 34 by the company Evonik Goldschmidt, and PEG-30 glyceryl stearate.
9) Optionally polyoxyethylenated ($C_8$-$C_{30}$)alkyl and poly($C_8$-$C_{30}$)alkyl ethers of glycerol that are preferably used are those with a number of ethylene oxide (EO) units ranging from 0 to 100 and a number of glycerol units ranging from 1 to 30. Examples that may be mentioned include Nikkol Batyl Alcohol 100 and Nikkol Chimyl Alcohol 100.

*Anionic surfactants*

The anionic surfactants may be chosen from alkyl ether sulfates, carboxylates, amino acid derivatives, sulfonates, isethionates, taurates, sulfosuccinates, alkylsulfoacetates, phosphates and alkyl phosphates, polypeptides, metal salts of $C_{10}$-$C_{30}$ and in particular $C_{12}$-$C_{20}$ fatty acids, in particular metal stearates, and mixtures thereof.

1) Examples of ($C_8$-$C_{30}$)alkyl ether sulfates that may be mentioned include sodium lauryl ether sulfate (70/30 $C_{12}$-$C_{14}$) (2.2 EO) sold under the names Sipon AOS225 or Texapon N702 by the company Henkel, ammonium lauryl ether sulfate (70/30 $C_{12}$-$C_{14}$) (3 EO) sold under the name Sipon LEA 370 by the company Henkel, ammonium ($C_{12}$-$C_{14}$) alkyl ether (9 EO) sulfate sold under the name Rhodapex AB/20 by the company Rhodia Chimie, and the mixture of sodium magnesium lauryl oleyl ether sulfate sold under the name Empicol BSD 52 by the company Albright & Wilson.
2) Examples of carboxylates that may be mentioned include salts (for example alkali metal salts) of N-acylamino acids, glycol carboxylates, amido ether carboxylates (AECs) and polyoxyethylenated carboxylic acid salts.

The surfactant of glycol carboxylate type may be chosen from alkyl glycol carboxylics or 2-(2-hydroxyalkyloxy acetate), salts thereof and mixtures thereof. These alkyl glycol carboxylics comprise a linear or branched, saturated or unsaturated aliphatic and/or aromatic alkyl chain containing from 8 to 18 carbon atoms. These carboxylics may be neutralized with mineral bases such as potassium hydroxide or sodium hydroxide.
Examples of surfactants of glycol carboxylic type that may be mentioned include sodium lauryl glycol carboxylate or sodium 2-(2-hydroxyalkyloxy acetate) such as the product sold under the name Beaulight Shaa® by the company Sanyo, Beaulight LCA-25N® or the corresponding acid form Beaulight Shaa (Acid form)®.
An example of an amido ether carboxylate (AEC) that may be mentioned is sodium lauryl amido ether carboxylate (3 EO) sold under the name Akypo Foam 30® by the company Kao Chemicals.
Examples of polyoxyethylenated carboxylic acid salts that may be mentioned include oxyethylenated (6 EO) sodium lauryl ether carboxylate (65/25/10 $C_{12\text{-}14\text{-}16}$) sold under the name Akypo Soft 45 NV® by the company Kao Chemicals, polyoxyethylenated and carboxymethylated fatty acids of olive oil origin sold under the name Olivem 400® by the company Biologia e Tecnologia, and oxyethylenated (6 EO) sodium tridecyl ether carboxylate sold under the name Nikkol ECTD-6 NEX® by the company Nikkol.

3) Amino acid derivatives that may especially be mentioned include alkali metal salts of amino acids, such as:

- ($C_8$-$C_{30}$)alkoyl sarcosinates, for instance the sodium lauroyl sarcosinate sold under the name Sarkosyl NL 97® by the company Ciba or sold under the name Oramix L30® by the company SEPPIC, sodium myristoyl sarcosinate sold under the name Nikkol Sarcosinate MN® by the company Nikkol, and sodium palmitoyl sarcosinate sold under the name Nikkol Sarcosinate PN® by the company Nikkol;
- ($C_8$-$C_{30}$)alkoyl alaninates, for instance sodium N-lauroyl N-methyl amidopropionate sold under the name Sodium Nikkol Alaninate LN30® by the company Nikkol, or sold under the name Alanone ALE® by the company Kawaken, and triethanolamine N-lauroyl N-methyl alanine sold under the name Alanone Alta® by the company Kawaken;
- ($C_8$-$C_{30}$)alkoyl glutamates, for instance triethanolamine monococoyl glutamate sold under the name Acylglutamate CT-12® by the company Ajinomoto, or triethanolamine lauroyl glutamate sold under the name Acylglutamate LT-12® by the company Ajinomoto;
- ($C_8$-$C_{30}$)alkoyl aspartates, for instance the mixture of triethanolamine N-lauroyl aspartate and of triethanolamine N-myristoyl aspartate, sold under the name Asparack® by the company Mitsubishi;
- glycine derivatives (($C_8$-$C_{30}$)alkoyl glycinates), for instance the sodium N-cocoyl glycinate sold under the names Amilite GCS-12® and Amilite GCK 12 by the company Ajinomoto;
- citrates such as citric acid monoesters of $C_8$-$C_{30}$ alcohols, such as the oxyethylenated (9 mol) citric monoester of cocoyl alcohols sold under the name Witconol EC 1129 by the company Goldschmidt;
- ($C_8$-$C_{30}$)alkyl galacturonates, such as the sodium dodecyl-D-galactoside uronate sold by the company Soliance.

4) Examples of sulfonates that may be mentioned include α-olefin sulfonates, for instance the sodium α-olefin sulfonate ($C_{14\text{-}16}$) sold under the name Bio-Terge AS-40® by the company Stepan, sold under the names Witconate AOS Protégé® and Sulframine AOS PH 12® by the company Witco or sold under the name Bio-Terge AS-40 CG®

by the company Stepan, and the sodium secondary olefin sulfonate sold under the name Hostapur SAS 30® by the company Clariant.

5) ($C_8$-$C_{30}$)Alkoyl isethionates that may be mentioned include acylisethionates, for instance sodium cocoyl isethionate, such as the product sold under the name Jordapon CI P® by the company Jordan.

6) ($C_8$-$C_{30}$)Alkoyl taurates that may be mentioned include the sodium salt of palm kernel oil methyltaurate sold under the name Hostapon CT Pate® by the company Clariant; N-acyl N-methyltaurates, for instance the sodium N-cocoyl N-methyltaurate sold under the name Hostapon LT-SF® by the company Clariant or sold under the name Nikkol CMT-30-T® by the company Nikkol, and the sodium palmitoyl methyltaurate sold under the name Nikkol PMT® by the company Nikkol.

7) Examples of sulfosuccinates that may be mentioned include the oxyethylenated (3 EO) lauryl alcohol monosulfosuccinate (70/30 $C_{12}$/$C_{14}$) sold under the names Setacin 103 Special® and Rewopol SB-FA 30 K 4® by the company Witco, the disodium salt of a $C_{12}$-$C_{14}$ alkyl hemisulfosuccinate, sold under the name Setacin F Special Paste® by the company Zschimmer Schwarz, the oxyethylenated (2 EO) disodium oleamidosulfosuccinate sold under the name Standapol SH 135® by the company Henkel, the oxyethylenated (5 EO) laurylamide monosulfosuccinate sold under the name Lebon A-5000® by the company Sanyo, the oxyethylenated (10 EO) disodium salt of lauryl citrate monosulfosuccinate sold under the name Rewopol SB CS 50® by the company Witco, and the ricinoleic monoethanolamide monosulfosuccinate sold under the name Rewoderm S 1333® by the company Witco. Polydimethylsiloxane sulfosuccinates may also be used, such as disodium PEG-12 dimethicone sulfosuccinate sold under the name Mackanate-DC30 by the company MacIntyre.

8) Examples of ($C_8$-$C_{30}$)alkyl sulfoacetates that may be mentioned include the mixture of sodium lauryl sulfoacetate and disodium lauryl ether sulfosuccinate, sold under the name Stepan-Mild LSB by the company Stepan.

9) Examples of phosphates and ($C_8$-$C_{30}$)alkyl phosphates that may be mentioned include monoalkyl phosphates and dialkyl phosphates, such as the lauryl monophosphate sold under the name MAP 20® by the company Kao Chemicals, the potassium salt of dodecylphosphoric acid, the mixture of monoester and diester (predominantly diester) sold under the name Crafol AP-31® by the company Cognis, the mixture of octylphosphoric acid monoester and diester sold under the name Crafol AP-20® by the company Cognis, the mixture of ethoxylated (7 mol of EO) phosphoric acid monoester and diester of 2-butyloctanol, sold under the name Isofol 12 7 EO-Phosphate Ester® by the company Condea, the potassium or triethanolamine salt of mono($C_{12}$-$C_{13}$)alkyl phosphate sold under the references Arlatone MAP 230K-40® and Arlatone MAP 230T-60® by the company Uniqema, the potassium lauryl phosphate sold under the name Dermalcare MAP XC-99/09® by the company Rhodia Chimie, and the potassium cetyl phosphate sold under the name Arlatone MAP 160K by the company Uniqema.

10) The polypeptides are obtained, for example, by condensation of a fatty chain onto amino acids from cereals and in particular from wheat and oat. Examples of polypeptides that may be mentioned include the potassium salt of hydrolysed lauroyl wheat protein, sold under the name Aminofoam W OR by the company Croda, the triethanolamine salt of hydrolysed cocoyl soybean protein, sold under the name May-Tein SY by the company Maybrook, the sodium salt of lauroyl oat amino acids, sold under the name Proteol Oat by the company SEPPIC, collagen hydrolysate grafted onto coconut fatty acid, sold under the name Geliderm 3000 by the company Deutsche Gelatine, and soybean proteins acylated with hydrogenated coconut acids, sold under the name Proteol VS 22 by the company SEPPIC.

11) As metal salts of $C_{10}$-$C_{30}$ and in particular $C_{12}$-$C_{20}$ fatty acids, mention may be made in particular of metal stearates, such as sodium stearate and potassium stearate, and also polyhydroxystearates.

*Silicone surfactants*

As regards the silicone surfactants, mention may be made of dimethicone copolyols, which are more particularly oxypropylenated and/or oxyethylenated polydimethyl methyl siloxanes.

As examples of dimethicone copolyols, use may be made of those corresponding more particularly to formula (II) below:

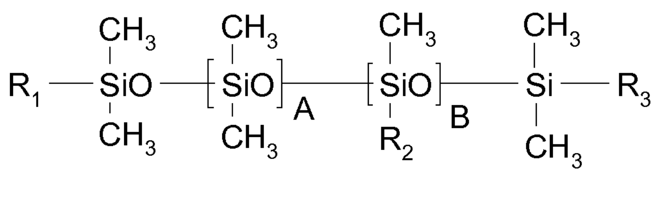

(II)

in which:

- $R_1$, $R_2$ and $R_3$, independently of each other, represent a $C_1$-$C_6$ alkyl radical or a radical $-(CH_2)_x-(OCH_2CH_2)_y-(OCH_2CH_2CH_2)_z-OR_4$, at least one radical $R_1$, $R_2$ or $R_3$ not being an alkyl radical; $R_4$ being a hydrogen, a $C_1$-$C_3$ alkyl radical or a $C_2$-$C_4$ acyl radical;
- A is an integer ranging from 0 to 200;
- B is an integer ranging from 0 to 50; on condition that A and B are not simultaneously equal to zero;
- x is an integer ranging from 1 to 6;
- y is an integer ranging from 1 to 30; and
- z is an integer ranging from 0 to 5.

Dimethicone copolyols that may be used include those sold under the names DC 5329, DC 7439-146, DC 2-5695 and Q4-3667 by the company Dow Corning; KF-6013, KF-6015, KF-6016, KF-6017 (PEG-10 dimethicone) and KF-6028 (PEG-polydimethylsiloxyethyl dimethicone) by the company Shin-Etsu.

Use may also be made of:

- dimethicone copolyol, such as the product sold under the name Q2-5220® by the company Dow Corning;
- the mixture of cyclomethicone/dimethicone copolyol sold under the name Q2-3225C® by the company Dow Corning; and
- the mixture of dimethicone and PEG/PPG-18/18 dimethicone sold under the name X-22-6711D by the company Shin-Etsu;
- the mixture of cyclomethicone and dimethicone copolyol sold under the name DC-5225C by the company Dow Corning;
- alkyl dimethicone copolyols such as the laurylmethicone copolyol sold under the name Dow Corning 5200 Formulation Aid by the company Dow Corning; cetyl dimethicone copolyol such as the product sold under the name Abil EM 90 by the company Evonik Goldschmidt, and the mixture of cetyl dimethicone copolyol, polyglyceryl (4 mol) isostearate and hexyl laurate sold under the name Abil WE 09 by the company Evonik Goldschmidt, or alternatively phosphate-based surfactants.

Use may also be made of dimethicone copolyol benzoate, such as the product sold under the names Finsolv SLB 101® and 201® by the company Finetex.

Emulsion surfactants that may also be mentioned include, in particular for water-in-oil emulsions, crosslinked elastomeric solid organopolysiloxanes comprising at least one oxyalkylene group, such as the products obtained according to the procedure of Examples 3, 4 and 8 of document US-A-5 412 004 and the examples of document US-A-5 811 487, especially the product of Example 3 (synthetic example) of patent US-A-5 412 004, and such as the product sold under the reference KSG 21 by the company Shin-Etsu.

One or more coemulsifiers, which may be chosen advantageously from the group comprising polyol alkyl esters, may also be added thereto. Polyol alkyl esters that may especially be mentioned include polyethylene glycol esters, for instance PEG-30 dipolyhydroxystearate, such as the product sold under the name Arlacel P135 by the company ICI.

According to a particular embodiment of the invention, and irrespective of the sense of the emulsion, the surfactant(s) are chosen from nonionic surfactants and silicone surfactants, or mixtures thereof.

According to a particularly preferred embodiment, an emulsion according to the invention, in particular a water-in-oil emulsion, comprises at least one silicone surfactant chosen from (alkyl)dimethicone copolyols, alone or as mixtures.

## AQUEOUS PHASE

The composition according to the invention comprises an aqueous phase.

The aqueous phase comprises water and optionally at least one water-soluble solvent.

In the present invention, the term "water-soluble solvent" means a compound that is liquid at room temperature and atmospheric pressure, and that is water-miscible (miscibility with water of greater than 50% by weight at 25°C and atmospheric pressure).

The composition according to the invention preferentially comprises at least 10% by weight, in particular from 20% to 70% by weight and especially from 25% to 60% by weight of aqueous phase, relative to the total weight of the composition.

The composition according to the invention preferentially comprises at least 10% by weight, in particular from 20% to 65% by weight and especially from 25% to 55% by weight of water, relative to the total weight of the composition.

According to one embodiment, the composition may comprise at least 60% by weight, preferably at least 70% by weight and in particular at least 75% by weight of water, relative to the total weight of the aqueous phase.

According to a preferred embodiment, the composition comprises a total content of water and volatile oil(s) of greater than or equal to 40% by weight and in particular greater than or equal to 50% by weight relative to the total

weight of the composition.

Among the water-soluble solvents that may be used in the composition in accordance with the invention, mention may be made especially of monoalcohols containing from 1 to 5 carbon atoms, such as ethanol and isopropanol, ethylene glycol, ketones containing three or four carbon atoms, and aldehydes containing from two to four carbon atoms.

## VOLATILE OIL

The composition according to the invention may comprise at least one volatile oil.

The term "volatile oil" means an oil that can evaporate on contact with the skin or the lips in less than one hour, at room temperature and atmospheric pressure. The volatile oils of the invention are volatile cosmetic oils that are liquid at room temperature, with a non-zero vapour pressure, at room temperature and atmospheric pressure, ranging in particular from 0.13 Pa to 40 000 Pa, in particular ranging from 1.3 Pa to 13 000 Pa and more particularly ranging from 1.3 Pa to 1300 Pa.

According to one embodiment, the composition comprises at least one volatile oil in a content ranging from 0.1% to 30% by weight, preferably from 0.5% to 25% by weight and in particular from 1% to 20% by weight relative to the total weight of the composition.

According to one embodiment, the composition comprises at least one volatile oil in a content of at least 10% by weight, preferably ranging from 25% to 90% by weight and in particular from 35% to 80% by weight relative to the total weight of volatile and non-volatile oil(s) present in said composition.

According to one embodiment, the composition comprises a mixture of volatile oil(s) and of non-volatile oil(s) in respective weight contents such that the ratio of the content of volatile oil(s) to the content of non-volatile oil(s) ranges from 0.7 to 3 and preferably ranges from 0.8 to 2.

The volatile oil(s) may be chosen from hydrocarbon-based oils, silicone oils and fluoro oils, and mixtures thereof.

The term "hydrocarbon-based oil" means an oil formed essentially from, or even consisting of, carbon and hydrogen atoms, and optionally oxygen and nitrogen atoms, and not comprising any silicon or fluorine atoms. A hydrocarbon-based oil may comprise, for example, alcohol, ester, carboxylic acid, amine and/or amide groups.

The term "fluoro oil" means an oil containing at least one fluorine atom.

The volatile hydrocarbon-based oil(s), which are preferably apolar, may have a flash point ranging from 40°C to 102°C, preferably ranging from 40°C to 55°C and preferentially ranging from 40°C to 50°C.

The hydrocarbon-based volatile oil(s) may be chosen from hydrocarbon-based volatile oils containing from 8 to 16 carbon atoms, and mixtures thereof, and especially:

- branched $C_8$-$C_{16}$ alkanes such as $C_8$-$C_{16}$ isoalkanes (also known as isoparaffins), isododecane, isodecane and isohexadecane, and, for example, the oils sold under the trade name Isopar or Permethyl,
- linear alkanes, for instance n-dodecane (C12) and n-tetradecane (C14) sold by Sasol under the respective references Parafol 12-97 and Parafol 14-97, and also mixtures thereof, the undecane-tridecane mixture (Cetiol UT), the mixtures of n-undecane (C11) and of n-tridecane (C13) obtained in Examples 1 and 2 of patent application WO 2008/155 059 from the company Cognis, and
- mixtures thereof.

The volatile silicone oil(s) may be chosen from silicone oils with a flash point ranging from 40°C to 102°C, preferably with a flash point of greater than 55°C and less than or equal to 95°C, and preferentially ranging from 65°C to 95°C.

As volatile silicone oils that may be used in the invention, mention may be made of linear or cyclic silicones with a viscosity at 25°C of less than 8 centistokes (cSt) ($8 \times 10^{-6}$ $m^2/s$), and in particular containing from 2 to 10 silicon atoms and in particular from 2 to 7 silicon atoms, these silicones optionally comprising alkyl or alkoxy groups containing from 1 to 10 carbon atoms.

As volatile silicone oils that may be used in the invention, mention may be made especially of dimethicones with viscosities of 2, 5 and 6 cSt, octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane and dodecamethylpentasiloxane, and mixtures thereof.

The volatile oil(s) may also be a fluoro oil, such as nonafluoromethoxybutane or perfluoromethylcyclopentane, and mixtures thereof.

## PASTY COMPOUND

The composition according to the invention also comprises at least one hydrocarbon-based or silicone-based compound that is pasty at 23°C.

For the purposes of the present invention, the term "pasty compound" means a water-immiscible compound with a reversible solid/liquid change of state, having in the solid state an anisotropic crystal organization, and comprising at a temperature of 23°C a liquid fraction and a solid fraction. In other words, the starting melting point of the pasty compound can be less than 23°C. The liquid fraction of the pasty compound, measured at 23°C, can represent from 9% to 97% by weight of the pasty compound. This liquid fraction at 23°C preferably represents between 15% and 85% and more preferably between 40% and 85% by weight.

For the purposes of the invention, the melting point corresponds to the temperature of the most endothermic peak observed in thermal analysis (DSC) as described in the standard ISO 11357-3; 1999. The melting point of a pasty compound may be measured using a differential scanning calorimeter (DSC), for example the calorimeter sold under the name MDSC 2920 by the company TA Instruments.

The measuring protocol is as follows: a sample of 5 mg of pasty compound placed in a crucible is subjected to a first temperature rise ranging from -20°C to 100°C, at a heating rate of 10°C/minute, is then cooled from 100°C to -20°C at a cooling rate of 10°C/minute and is finally subjected to a second temperature rise ranging from -20°C to 100°C at a heating rate of 5°C/minute. During the second temperature rise, the variation in the difference in power absorbed by the empty crucible and by the crucible containing the sample of pasty fatty substance is measured as a function of the temperature. The melting point of the pasty compound is the temperature value corresponding to the top of the peak of the curve representing the variation in the difference in power absorbed as a function of the temperature.

The liquid fraction by weight of the pasty compound at 23°C is equal to the ratio of the heat of fusion consumed at 23°C to the heat of fusion of the pasty compound.

The heat of fusion (expressed in J/g) of the pasty compound is the amount of energy required to make the compound change from the solid state to the liquid state. The pasty compound is said to be in the solid state when all of its mass is in crystalline solid form. The pasty compound is said to be in the liquid state when all of its mass is in liquid form. The heat of fusion consumed at 23°C is the amount of energy absorbed by the sample to change from the solid state to the state that it has at 2°C, consisting of a liquid fraction and a solid fraction.

The heat of fusion of the pasty compound is equal to the area under the curve of the thermogram obtained using a differential scanning calorimeter (DSC), such as the calorimeter sold under the name MDSC 2920 by the company TA Instrument, with a temperature rise of 5°C or 10°C per minute, according to the standard ISO 11357-3; 1999.

The liquid fraction of the pasty compound measured at 32°C preferably represents from 30% to 100% by weight of the pasty compound, preferably from 50% to 100% and more preferably from 60% to 100% by weight of the pasty compound. When the liquid fraction of the pasty compound measured at 32°C is equal to 100%, the temperature of the end of the melting range of the pasty compound is less than or equal to 32°C.

The liquid fraction of the pasty compound measured at 32°C is equal to the ratio of the heat of fusion consumed at 32°C to the heat of fusion of the pasty compound. The heat of fusion consumed at 32°C is calculated in the same way as the heat of fusion consumed at 23°C.

The pasty compound(s) may in particular be chosen from synthetic pasty compounds and fatty substances of plant origin. The pasty compound(s) may be hydrocarbon-based or silicone-based.

The pasty compound(s) may be chosen in particular from:

- lanolin and derivatives thereof, such as lanolin alcohol, oxyethylenated lanolins, acetylated lanolin, lanolin esters such as isopropyl lanolate, and oxypropylenated lanolins,
- petroleum jelly (also known as petrolatum),
- polyol ethers chosen from $C_2$-$C_4$ polyalkylene glycol pentaerythrityl ethers, fatty alcohol ethers of sugars, and mixtures thereof. For example, mention may be made of polyethylene glycol pentaerythrityl ether comprising 5 oxyethylene units (5 OE) (CTFA name: PPG-5 Pentaerythrityl Ether), polypropylene glycol pentaerythrityl ether comprising five oxypropylene (5 OP) units (CTFA name: PPG-5 Pentaerythrityl Ether) and mixtures thereof, and more especially the mixture PEG-5 Pentaerythrityl Ether, PPG-5 Pentaerythrityl Ether and soybean oil, sold under the name Lanolide by the company Vevy, which is a mixture in which the constituents are in a 46/46/8 weight ratio: 46% PEG-5 Pentaerythrityl Ether, 46% PPG-5 Pentaerythrityl Ether and 8% soybean oil,
- polymeric or non-polymeric silicone compounds,
- polymeric or non-polymeric fluoro compounds,
- vinyl polymers, especially:

  - ◦ olefin homopolymers and copolymers,
  - ◦ hydrogenated diene homopolymers and copolymers,
  - ◦ linear or branched oligomers, which are homopolymers or copolymers of alkyl (meth)acrylates preferably containing a $C_8$-$C_{30}$ alkyl group,
  - ◦ oligomers, which are homopolymers and copolymers of vinyl esters containing $C_8$-$C_{30}$ alkyl groups, and
  - ◦ oligomers, which are homopolymers and copolymers of vinyl esters containing $C_8$-$C_{30}$ alkyl groups,

- liposoluble polyethers resulting from the polyetherification between one or more $C_2$-$C_{100}$ and preferably $C_2$-$C_{50}$ diols. Among the liposoluble polyethers that are particularly considered are copolymers of ethylene oxide and/or of propylene oxide with $C_6$-$C_{30}$ long-chain alkylene oxides, more preferably such that the weight ratio of the ethylene oxide and/or of the propylene oxide to the alkylene oxides in the copolymer is from 5:95 to 70:30. In this family, mention will be made especially of copolymers such as long-chain alkylene oxides arranged in blocks with an average molecular weight from 1000 to 10 000, for example a polyoxyethylene/polydodecyl glycol block copolymer such as the ethers of dodecanediol (22 mol) and of polyethylene glycol (45 OE) sold under the brand name Elfacos ST9 by Akzo Nobel,
- esters and polyesters. Among the esters, the following are especially considered:

    ◦ esters of a glycerol oligomer, especially diglycerol esters, with linear or branched, saturated or unsaturated, preferably saturated, $C_6$-$C_{20}$, optionally hydroxylated monocarboxylic acids, and/or linear or branched, saturated or unsaturated, preferably saturated, $C_6$-$C_{10}$ dicarboxylic acids, in particular condensates of adipic acid and of diglycerol, for which some of the hydroxyl groups of the glycerols have reacted with a mixture of fatty acids such as stearic acid, capric acid, isostearic acid and 12-hydroxystearic acid, for instance bis-diglyceryl polyacyladipate-2 sold under the reference Softisan® 649 by the company Sasol,
    ◦ vinyl ester homopolymers bearing $C_8$-$C_{30}$ alkyl groups, such as polyvinyl laurate (sold especially under the reference Mexomer PP by the company Chimex),
    ◦ the arachidyl propionate sold under the brand name Waxenol 801 by Alzo,
    ◦ phytosterol esters,
    ◦ fatty acid triglycerides and derivatives thereof, in particular optionally hydrogenated (totally or partially), optionally monohydroxylated or polyhydroxylated, $C_6$-$C_{30}$ and more particularly $C_8$-$C_{18}$, linear or branched, saturated or unsaturated fatty acid triglycerides; for example Softisan 100® sold by the company Sasol,
    ◦ pentaerythritol esters,
    ◦ aliphatic esters resulting from the esterification of an aliphatic hydroxycarboxylic acid ester with an aliphatic carboxylic acid. More particularly, the aliphatic carboxylic acid is of $C_4$-$C_{30}$ and preferably of $C_8$-$C_{30}$. It is preferably chosen from hexanoic, heptanoic, octanoic, 2-ethylhexanoic, nonanoic, decanoic, undecanoic, dodecanoic, tridecanoic, tetradecanoic, pentadecanoic, hexadecenoic, hexyldecanoic, heptadecanoic, octadecanoic, isostearic, nonadecanoic, eicosanoic, isoarachidic, octyldodecanoic, heneicosanoic and docosanoic acids, and mixtures thereof. The aliphatic carboxylic acid is preferably branched. The hydroxy carboxylic acid ester is advantageously derived from a $C_2$-$C_{40}$, preferably $C_{10}$-$C_{34}$ and even more preferentially $C_{12}$-$C_{28}$ hydroxylated carboxylic acid; the number of hydroxyl groups being between 1 and 20, more particularly between 1 and 10 and preferably between 1 and 6.

Said hydroxy carboxylic acid esters are preferably chosen from:

a) total or partial esters of saturated, linear and monohydroxylated aliphatic monocarboxylic acids;
b) total or partial esters of saturated, monohydroxylated aliphatic monocarboxylic acids;
c) total or partial esters of saturated, monohydroxylated aliphatic polycarboxylic acids;
d) total or partial esters of saturated, polyhydroxylated aliphatic polycarboxylic acids;
e) partial or total esters of $C_2$-$C_{16}$ aliphatic polyols with a monohydroxylated or polyhydroxylated aliphatic monocarboxylic or polycarboxylic acid;
f) mixtures thereof;

    o esters of a diol dimer and of a diacid dimer, where appropriate esterified on their free alcohol or acid function(s) with acid or alcohol radicals, especially dimer dilinoleate esters; such esters may be chosen especially from the esters having the following INCI nomenclature: bis-behenyl/isostearyl/phytosteryl dimer dilinoleyl dimer dilinoleate (Plandool G), phytosteryl/isosteryl/cetyl/stearyl/behenyl dimer dilinoleate (Plandool H or Plandool S), and mixtures thereof,
    ◦ hydrogenated esters of rosin (Lusplan DD-DHR or DD-DHR from Nippon Fine Chemical);

    - butters of plant origin, such as mango butter, such as the product sold under the reference Lipex 203 by the company Aarhuskarlshamn, shea butter, in particular the product whose INCI name is Butyrospermum Parkii Butter, such as the product sold under the reference Sheasoft® by the company Aarhuskarlshamn, cupuacu butter (Rain Forest RF3410 from the company Beraca Sabara), murumuru butter (Rain Forest RF3710 from the company Beraca Sabara), cocoa butter; babassu butter such as the product sold under the name Cropure Babassu SS-(LK) by Croda, and also orange wax, for instance the product sold under the reference Orange Peel Wax by the company Koster Keunen,

- totally or partially hydrogenated plant oils, for instance hydrogenated soybean oil, hydrogenated coconut oil, hydrogenated rapeseed oil, mixtures of hydrogenated plant oils such as the mixture of hydrogenated soybean, coconut, palm and rapeseed plant oil, for example the mixture sold under the reference Akogel® by the company Aarhuskarlshamn (INCI name Hydrogenated Vegetable Oil), the trans-isomerized partially hydrogenated jojoba oil manufactured or sold by the company Desert Whale under the commercial reference Iso-Jojoba-50®, partially hydrogenated olive oil, for instance the compound sold under the reference Beurrolive by the company Soliance,
- hydrogenated castor oil esters, such as hydrogenated castor oil dimer dilinoleate, for example Risocast DA-L sold by Kokyu Alcohol Kogyo, and hydrogenated castor oil isostearate, for example Salacos HCIS (V-L) sold by Nisshin Oil,
- and mixtures thereof.

Preferably, the pasty compound(s) that are suitable for use in the invention are chosen from hydrocarbon-based compounds, in particular chosen from petroleum jelly, polyol ethers, vinyl polymers, liposoluble polyethers resulting from polyetherification between one or more $C_2$-$C_{50}$ diols, esters and polyesters, butters of plant origin, totally or partially hydrogenated plant oils, and mixtures thereof.

In accordance with an even more preferred embodiment of the invention, the pasty compound(s) are chosen from:

- petroleum jelly;
- pentaerythrityl ethers of $C_2$-$C_4$ polyalkylene glycol;
- fatty acid ethers of sugars;
- copolymers of ethylene oxide and/or of propylene oxide with $C_6$-$C_{30}$ long-chain alkylene oxides;
- esters of a glycerol oligomer, especially esters of diglycerol, with linear or branched, saturated or unsaturated, preferably saturated, $C_6$-$C_{20}$, optionally hydroxylated monocarboxylic acids, and/or linear or branched, saturated or unsaturated, preferably saturated, $C_6$-$C_{10}$ dicarboxylic acids;
- vinyl ester homopolymers bearing $C_8$-$C_{30}$ alkyl groups;
- arachidyl propionate;
- saturated or unsaturated, linear or branched, optionally monohydroxylated or polyhydroxylated, $C_6$-$C_{30}$, more particularly $C_8$-$C_{18}$, optionally hydrogenated fatty acid triglycerides;
- pentaerythritol esters;
- non-crosslinked esters obtained by condensation of a linear or branched $C_4$-$C_{50}$ dicarboxylic or polycarboxylic acid and of a $C_2$-$C_{50}$ diol or polyol, the aliphatic esters obtained by reaction of an ester of a hydroxycarboxylic acid and of an aliphatic carboxylic acid, the carboxylic acid advantageously being $C_4$-$C_{30}$;
- esters of a diol dimer and of a diacid dimer, for instance dilinoleate dimer esters;
- butters of plant origin;
- totally or partially hydrogenated plant oils;
- and mixtures thereof.

According to one embodiment, the composition according to the invention comprises at least one pasty compound in a content of greater than 0.5% by weight, preferably in a content ranging from 2% to 20% by weight, in particular from 2% to 15% by weight and more advantageously from 2% to 10% by weight, relative to the total weight of the composition.

## POLYOLS

The composition according to the invention may comprise at least one polyol, more particularly a saturated or unsaturated, linear or branched $C_2$-$C_8$ and preferably $C_3$-$C_6$ polyol that is liquid at room temperature, comprising from 2 to 6 hydroxyl groups.

Preferably, the polyol is chosen from glycerol, diglycerol, linear or branched, saturated $C_3$-$C_8$ glycols, in particular propylene glycol, butylene glycol, pentylene glycol, caprylyl glycol or dipropylene glycol, and also mixtures thereof, and preferably glycerol, propylene glycol or butylene glycol, and mixtures thereof.

In accordance with a preferred embodiment of the invention, the second oily phase comprises at least glycerol as polyol.

If it comprises any, the composition according to the invention has a content of polyol(s) particularly between 4% and 10% by weight and preferably from 6% to 8% by weight relative to the weight of the composition.

## COLOURING SUBSTANCES

The composition according to the invention may also comprise at least one colouring substance chosen especially from water-soluble or liposoluble dyestuffs, pigments and nacres, and mixtures thereof. Advantageously, the amount of such colouring substance(s) is lower or equal to 10 % by weight relative to the weight of the composition, if present therein.

The term "colouring substance" means a compound that is capable of producing a coloured optical effect when it is formulated in sufficient amount in a suitable cosmetic medium.

The water-soluble colouring substances used according to the invention are more particularly water-soluble dyes.

For the purposes of the invention, the term "water-soluble dye" means any natural or synthetic, generally organic compound, which is soluble in an aqueous phase or water-miscible solvents and which is capable of imparting colour. In particular, the term "water-soluble" is intended to characterize the capacity of a compound to be dissolved in water, measured at 25°C, to a concentration at least equal to 0.1 g/L (production of a macroscopically isotropic, transparent, coloured or colourless solution). This solubility is in particular greater than or equal to 1 g/L.

As water-soluble dyes that are suitable for use in the invention, mention may be made especially of synthetic or natural water-soluble dyes, for instance FDC Red 4 (CI: 14700), DC Red 6 (Lithol Rubine Na; CI: 15850), DC Red 22 (CI: 45380), DC Red 27 (CI: 45410 Na salt), DC Red 30 (CI: 73360), DC Red 33 (CI: 17200), DC Orange 4 (CI: 15510), FDC Yellow 5 (CI: 19140), FDC Yellow 6 (CI: 15985), DC Yellow 8 (CI: 45350 Na salt), FDC Green 3 (CI: 42053), DC Green 5 (CI: 61570), FDC Blue 1 (CI: 42090).

As non-limiting illustrations of sources of water-soluble colouring substance(s) that may be used in the context of the present invention, mention may be made in particular of those of natural origin, such as extracts of cochineal carmine, of beetroot, of grape, of carrot, of tomato, of annatto, of paprika, of henna, of caramel and of curcumin.

Thus, the water-soluble colouring substances that are suitable for use in the invention are especially carminic acid, betanin, anthocyans, enocyanins, lycopene, β-carotene, bixin, norbixin, capsanthin, capsorubin, flavoxanthin, lutein, cryptoxanthin, rubixanthin, violaxanthin, riboflavin, rhodoxanthin, cantaxanthin and chlorophyll, and mixtures thereof.

They may also be copper sulfate, iron sulfate, water-soluble sulfopolyesters, rhodamine, betaine, methylene blue, the disodium salt of tartrazine and the disodium salt of fuchsin.

Some of these water-soluble colouring substances are in particular permitted for food use. Representatives of these dyes that may be mentioned more particularly include dyes of the carotenoid family, referenced under the food codes E120, E162, E163, E160a-g, E150a, E101, E100, E140 and E141.

According to a particularly preferred embodiment, the water-soluble colouring substance(s) are chosen from the disodium salt of brilliant yellow FCF sold by the company LCW under the name DC Yellow 6, the disodium salt of fuchsin acid D sold by the company LCW under the name DC Red 33, and the trisodium salt of Rouge Allura sold by the company LCW under the name FD & C Red 40.

The colouring substance(s) used in the context of the present invention may also be chosen from pigments.

The term "pigments" should be understood as meaning white or coloured, inorganic (mineral) or organic particles, which are insoluble in the liquid organic phase, and which are intended to colour and/or opacify the composition and/or the deposit produced with the composition.

The pigments may be chosen from mineral pigments, organic pigments and composite pigments (i.e. pigments based on mineral and/or organic materials).

The pigments may be chosen from monochromatic pigments, lakes, nacres, and pigments with an optical effect, for instance reflective pigments and goniochromatic pigments.

The coated or uncoated mineral pigments may be chosen from metal oxide pigments, chromium oxides, iron oxides, titanium dioxide, zinc oxides, cerium oxides, zirconium oxides, manganese violet, Prussian blue, ultramarine blue and ferric blue, and mixtures thereof.

Organic lakes are organic pigments formed from a dye attached to a substrate. The lakes, which are also known as organic pigments, may be chosen from:

- cochineal carmine;
- organic pigments of azo dyes, anthraquinone dyes, indigoid dyes, xanthene dyes, pyrene dyes, quinoline dyes, triphenylmethane dyes or fluoran dyes. Among the organic pigments that may in particular be mentioned are those known under the following names: D&C Blue No. 4, D&C Brown No. 1, D&C Green No. 5, D&C Green No. 6, D&C Orange No. 4, D&C Orange No. 5, D&C Orange No. 10, D&C Orange No. 11, D&C Red No. 6, D&C Red No. 7, D&C Red No. 17, D&C Red No. 21, D&C Red No. 22, D&C Red No. 27, D&C Red No. 28, D&C Red No. 30, D&C Red No. 31, D&C Red No. 33, D&C Red No. 34, D&C Red No. 36, D&C Violet No. 2, D&C Yellow No. 7, D&C Yellow No. 8, D&C Yellow No. 10, D&C Yellow No. 11, FD&C Blue No. 1, FD&C Green No. 3, FD&C Red No. 40, FD&C Yellow No. 5, FD&C Yellow No. 6;

- insoluble sodium, potassium, calcium, barium, aluminium, zirconium, strontium or titanium salts of acid dyes such as azo, anthraquinone, indigoid, xanthene, pyrene, quinoline, triphenylmethane or fluoran dyes, these dyes possibly comprising at least one carboxylic or sulfonic acid group; and
- mixtures thereof.

The organic lakes may also be supported on an organic support such as rosin or aluminium benzoate, for example.

Among the organic lakes, mention may be made in particular of those known under the following names: D&C Red No. 2 Aluminium lake, D&C Red No. 3 Aluminium lake, D&C Red No. 4 Aluminium lake, D&C Red No. 6 Aluminium lake, D&C Red No. 6 Barium lake, D&C Red No. 6 Barium/Strontium lake, D&C Red No. 6 Strontium lake, D&C Red No. 6 Potassium lake, D&C Red No. 7 Aluminium lake, D&C Red No. 7 Barium lake, D&C Red No. 7 Calcium lake, D&C Red No. 7 Calcium/Strontium lake, D&C Red No. 7 Zirconium lake, D&C Red No. 8 Sodium lake, D&C Red No. 9 Aluminium lake, D&C Red No. 9 Barium lake, D&C Red No. 9 Barium/Strontium lake, D&C Red No. 9 Zirconium lake, D&C Red No. 10 Sodium lake, D&C Red No. 19 Aluminium lake, D&C Red No. 19 Barium lake, D&C Red No. 19 Zirconium lake, D&C Red No. 21 Aluminium lake, D&C Red No. 21 Zirconium lake, D&C Red No. 22 Aluminium lake, D&C Red No. 27 Aluminium lake, D&C Red No. 27 Aluminium/Titanium/Zirconium lake, D&C Red No. 27 Barium lake, D&C Red No. 27 Calcium lake, D&C Red No. 27 Zirconium lake, D&C Red No. 28 Aluminium lake, D&C Red No. 30 lake, D&C Red No. 31 Calcium lake, D&C Red No. 33 Aluminium lake, D&C Red No. 34 Calcium lake, D&C Red No. 36 lake, D&C Red No. 40 Aluminium lake, D&C Blue No. 1 Aluminium lake, D&C Green No. 3 Aluminium lake, D&C Orange No. 4 Aluminium lake, D&C Orange No. 5 Aluminium lake, D&C Orange No. 5 Zirconium lake, D&C Orange No. 10 Aluminium lake, D&C Orange No. 17 Barium lake, D&C Yellow No. 5 Aluminium lake, D&C Yellow No. 5 Zirconium lake, D&C Yellow No. 6 Aluminium lake, D&C Yellow No. 7 Zirconium lake, D&C Yellow No. 10 Aluminium lake, FD&C Blue No. 1 Aluminium lake, FD&C Red No. 4 Aluminium lake, FD&C Red No. 40 Aluminium lake, FD&C Yellow No. 5 Aluminium lake, FD&C Yellow No. 6 Aluminium lake.

Mention may also be made of liposoluble dyes, such as, for example, Sudan Red, DC Red 17, DC Green 6, β-carotene, soybean oil, Sudan Brown, DC Yellow 11, DC Violet 2, DC Orange 5 and quinoline yellow.

The chemical substances corresponding to each of the organic colouring substances cited above are mentioned in the publication "International Cosmetic Ingredient Dictionary and Handbook", 1997 edition, pages 371 to 386 and 524 to 528, published by The Cosmetic, Toiletries and Fragrance Association, the content of which is incorporated into the present patent application by way of reference.

The pigments may also have been subjected to a hydrophobic treatment.

The hydrophobic treatment agent may be chosen from silicones such as methicones, dimethicones, alkoxylsilanes and perfluoroalkylsilanes; fatty acids such as stearic acid; metal soaps such as aluminium dimyristate, the aluminium salt of hydrogenated tallow glutamate, perfluoroalkyl phosphates, perfluoroalkylsilanes, perfluoroalkylsilazanes, polyhexafluoropropylene oxides, polyorganosiloxanes comprising perfluoroalkyl perfluoropolyether groups and amino acids; N-acylamino acids or salts thereof; lecithin, isopropyl triisostearyl titanate, and mixtures thereof.

The N-acylamino acids can comprise an acyl group containing from 8 to 22 carbon atoms, for instance a 2-ethylhexanoyl, caproyl, lauroyl, myristoyl, palmitoyl, stearoyl or cocoyl group. The salts of these compounds can be the aluminium, magnesium, calcium, zirconium, zinc, sodium or potassium salts. The amino acid may be, for example, lysine, glutamic acid or alanine.

The term "alkyl" mentioned in the compounds cited above especially denotes an alkyl group containing from 1 to 30 carbon atoms and preferably containing from 5 to 16 carbon atoms.

Hydrophobic-treated pigments are described especially in patent application EP-A-1 086 683.

For the purposes of the present patent application, the term "nacre" means coloured particles of any form, which may or may not be iridescent, especially produced by certain molluscs in their shell, or alternatively synthesized, and which have a colour effect via optical interference.

Examples of nacres that may be mentioned include nacreous pigments such as titanium mica coated with an iron oxide, mica coated with bismuth oxychloride, titanium mica coated with chromium oxide, titanium mica coated with an organic dye in particular of the abovementioned type, and also nacreous pigments based on bismuth oxychloride. They may also be mica particles, at the surface of which are superposed at least two successive layers of metal oxides and/or of organic dyestuffs.

The nacres may more particularly have a yellow, pink, red, bronze, orangey, brown, gold and/or coppery colour or tint.

As illustrations of nacres that may be introduced as interference pigments into the first composition, mention may be made of the gold-coloured nacres sold in particular by the company Engelhard under the name Brilliant gold 212G (Timica), Gold 222C (Cloisonne), Sparkle gold (Timica), Gold 4504 (Chromalite) and Monarch gold 233X (Cloisonne); the bronze nacres sold in particular by the company Merck under the name Bronze fine (17384) (Colorona) and Bronze (17353) (Colorona) and by the company Engelhard under the name Super bronze (Cloisonne); the orange nacres

sold in particular by the company Engelhard under the name Orange 363C (Cloisonne) and Orange MCR 101 (Cosmica) and by the company Merck under the name Passion orange (Colorona) and Matte orange (17449) (Microna); the brown nacres sold in particular by the company Engelhard under the name Nu-antique copper 340XB (Cloisonne) and Brown CL4509 (Chromalite); the nacres with a copper tint sold in particular by the company Engelhard under the name Copper 340A (Timica); the nacres with a red tint sold in particular by the company Merck under the name Sienna fine (17386) (Colorona); the nacres with a yellow tint sold in particular by the company Engelhard under the name Yellow (4502) (Chromalite); the red nacres with a gold tint sold in particular by the company Engelhard under the name Sunstone G012 (Gemtone); the pink nacres sold in particular by the company Engelhard under the name Tan opale G005 (Gemtone); the black nacres with a gold tint sold in particular by the company Engelhard under the name Nu antique bronze 240 AB (Timica), the blue nacres sold in particular by the company Merck under the name Matte blue (17433) (Microna), the white nacres with a silvery tint sold in particular by the company Merck under the name Xirona Silver, and the golden-green pink-orange nacres sold in particular by the company Merck under the name Indian summer (Xirona), and mixtures thereof.

## ADDITIVES

The composition according to the invention may also comprise any additive chosen by a person skilled in the art such that the advantageous properties intrinsically associated with the compositions in accordance with the invention are not, or not substantially, adversely affected by the envisaged addition(s).

As additives that may be incorporated into the compositions in accordance with the invention, mention may be made especially of hydrophilic thickeners, hydrophobic thickeners, fillers of organic or mineral nature, emollients, humectants other than the abovementioned polyols, stabilizers, preserving agents, pigment dispersers, film-forming agents other than those according to the invention, and organic solvents.

As examples of hydrophilic thickening polymers, mention may be made more particularly of:

- acrylic or methacrylic acid homopolymers or copolymers or salts and esters thereof and in particular the products sold under the names Versicol F or Versicol K by the company Allied Colloid, Ultrahold 8 by the company Ciba-Geigy, and polyacrylic acids of Synthalen K type, and salts, in particular sodium salts, of polyacrylic acid (corresponding to the INCI name sodium acrylate copolymer) and more particularly a crosslinked sodium polyacrylate (corresponding to the INCI name sodium acrylate copolymer (and) caprylic/capric triglyceride) sold under the name Luvigel EM by the company BASF,
- copolymers of acrylic acid and of acrylamide sold in the form of the sodium salt thereof under the name Reten by the company Hercules, the sodium polymethacrylate sold under the name Darvan No. 7 by the company Vanderbilt, and the sodium salts of polyhydroxycarboxylic acids sold under the name Hydagen F by the company Henkel,
- polyacrylic acid/alkyl acrylate copolymers, preferably modified or unmodified carboxyvinyl polymers, particularly acrylate/$C_{10}$-$C_{30}$-alkylacrylate copolymers (INCI name: Acrylates/$C_{10}$-$C_{30}$ Alkyl acrylate Crosspolymer) such as the products sold by the company Lubrizol under the trade names Pemulen TR1, Pemulen TR2, Carbopol 1382 and Carbopol EDT 2020, and even more preferentially Pemulen TR-2,
- polyacrylamidomethylpropanesulfonic acid partially neutralized with aqueous ammonia and highly crosslinked, sold by the company Clariant,
- acrylamidopropanesulfonic/acrylamide copolymers of Sepigel or Simulgel type sold by the company SEPPIC,
- polyoxyethylenated acrylamidopropanesulfonic/alkyl methacrylate copolymers (crosslinked or non-crosslinked) of the Aristoflex HMS type sold by the company Clariant,
- copolymers of hydroxyalkylacrylic acid or salts thereof and of acryloyldimethyl taurate monomers such as the product Sepinov EMT 10 sold by the company SEPPIC,
- and mixtures thereof.

Other examples of hydrophilic gelling polymers that may be mentioned include:

- anionic, cationic, amphoteric or nonionic chitin or chitosan polymers;
- cellulose polymers, for instance alkylcelluloses such as hydroxyethylcellulose, hydroxypropylcellulose, hydroxymethylcellulose, ethylhydroxyethylcellulose and carboxymethylcellulose, and also quaternized cellulose derivatives;
- vinyl polymers, for instance polyvinylpyrrolidones, copolymers of methyl vinyl ether and of malic anhydride, the copolymer of vinyl acetate and of crotonic acid, copolymers of vinylpyrrolidone and of vinyl acetate; copolymers of vinylpyrrolidone and of caprolactam; polyvinyl alcohol;
- optionally modified polymers of natural origin, such as galactomannans and derivatives thereof, for instance konjac gum, gellan gum, locust bean gum, fenugreek gum, karaya gum, gum tragacanth, gum arabic, acacia gum, guar

gum, hydroxypropyl guar, hydroxypropyl guar modified with sodium methylcarboxylate groups (Jaguar XC97-1, Rhodia), hydroxypropyltrimethylammonium guar chloride, and xanthan gum and derivatives thereof;
- alginates and carrageenans;
- muccopolysaccharides such as hyaluronic acid;
- and mixtures thereof.

If the composition comprises any, the content of hydrophilic thickener ranges from 0.01% to 3% by weight, preferably from 0.05% to 2% by weight and more advantageously from 0.1% to 1% by weight relative to the weight of the composition.

The term "filler" should be understood as meaning colourless or white solid particles of any shape, which are in a form that is insoluble and dispersed in the medium of the composition. They are different from the dyestuffs and make it possible especially to thicken the lipophilic phase of the composition according to the invention.

Among the fillers that may be used in the compositions according to the invention, mention may be made, for instance, of silica, kaolin, bentone, starch, lauroyllysine, and fumed silica particles, optionally hydrophilically or hydrophobically treated, and mixtures thereof.

A composition used according to the invention may comprise one or more fillers in a content ranging from 0.1% to 15% by weight and in particular from 1% to 10% by weight relative to the total weight of the composition.

## DEVICE

The composition according to the invention may be conditioned in any type of device that is common for fluid cosmetic compositions intended especially to be applied to the lips.

It may thus be envisaged to use devices containing a container comprising an applicator equipped with a ball (roll-on), a container of dispensing pen type, terminated with an end provided with at least one orifice through which the composition may be expelled, or alternatively terminated with a felt or with a flocked tip, or with a brush; a container comprising a dip applicator, for instance a brush.

Such devices may or may not be provided with a mechanism for dispensing the composition making it possible to expel said composition from the container to the application member, or to the support. It should be noted that this mechanism may advantageously comprise a means for metering out the composition.

According to a particularly advantageous embodiment of the invention, the composition is packaged in a container and is applied by means of an application member having a porous application surface.

The application member may be entirely porous, i.e. the composition may cross from an internal surface of the application member to an external surface of the application member, or vice versa.

The application member may in particular consist of one or more open-cell or semi-open-cell foams.

Thus, the application member may be made up of one of at least two portions of foam, of different compressibilities, which can be adhesively bonded together.

The foam may be non-crosslinked or preferentially crosslinked.

Still preferentially, the foam(s) are elastically compressible. The term "elastically compressible" is intended to mean that, starting from a position deformed by a pressure exerted on its surface, the foam returns to its initial shape when the pressure is released.

With such materials, the releasing of the composition on the surface to be treated takes place either by capillary action on contact with the lips, or by expulsion of the composition, from the pores of the applicator, in response to a slight deformation (by pressure) of said applicator on the surface to be treated.

The application member may have a cylindrical shape and may have a circular cross section. As a variant, the application member may have any other shape, for example conical, flattened-cone, nose-cone or prism shape, and can have an oval, rectangular or polygonal cross section. It may also comprise a bevelled portion and/or a concave portion forming a hollow on the application surface. The application member may be symmetrical or asymmetrical relative to a longitudinal plane of said application member.

The visible part of the application member may have a cross section which falls within a circle advantageously having a diameter of between 2 mm and 20 mm, and preferably between 5 mm and 15 mm. The visible part of the application member, in the non-compressed position, may be between 2 mm and 20 mm in height.

The application member may consist of several different materials, in particular of a stack of foams having different characteristics.

By way of example, the application member may consist of one or more foams chosen from polyether, polyester, polyurethane, polyester-polyurethane, NBR (natural butadiene rubber), SBR (synthetic butadiene rubber) or PVC (polyvinyl chloride) foams, or mixtures thereof, and quite particularly polyester-polyurethane, in particular the product S90 from Crest Foam Industries.

The density of the foam(s) forming the application member, measured according to standard ASTM D 3574-05,

may advantageously be between 0.02 $g.cm^{-3}$ and 0.05 $g.cm^{-3}$, for example 0.03 $g.cm^{-3}$. This density makes it possible to release an appropriate amount of impregnated composition.

The average number of pores or cells of the foam(s) may advantageously be between 25 and 50 pores per centimetre, for example equal to 35 pores per centimetre. The average pore size may advantageously be between 0.2 mm and 0.5 mm. It should be noted that the evaluation of the average number of pores is conventionally carried out visually by counting. Preferably, the cells or pores communicate with one another omnidirectionally.

The hardness of the application surface of the applicator member, measured by means of an F-type durometer from Asker, may advantageously be between 10 Asker F and 70 Asker F.

At least one part of the surface of the application member may be covered with a flock, in particular based on polyamide, rayon, cotton, viscose or nylon fibres. The flock contributes to creating a store of product, immediately in the neighbourhood of the application surface. In addition, it makes it possible to impart more softness on application, in particular when the application member is made of wide-cell foam. Furthermore, the flock may contribute to the homogenization of the spreading of the composition to form a thin film.

The flock may consist of a mixture of fibres of various lengths and/or nature and/or diameters.

The length of the fibres may advantageously be between 0.2 mm and 1 mm, for example equal to 0.75 mm.

The fibre count grading unit may advantageously be between 0.3 dtex and 3.3 dtex, for example equal to 1.7 dtex.

As a variant, the application surface can be covered with a permeable coating of textile, perforated plastic or felt type.

In accordance with one advantageous variant of the invention, the device comprises a composition-dispensing mechanism which makes it possible to expel said composition from the container to the application member.

According to this variant, said dispensing mechanism advantageously comprises a composition-metering means.

Application devices that are particularly suited to this composition will be described with reference to the appended drawings, in which:

- Figure 1 is a sectional view of an application device according to a first embodiment;
- Figure 2 is an exploded view of an application device according to a second embodiment;
- Figure 3 is a sectional view of the device of Figure 2.

According to a first embodiment represented in Figure 1, the device 1 comprises a cylindrical polypropylene body 2 having a longitudinal axis X. The body 2 denotes a first housing 3 (container), delimited by a cylindrical skirt 19, a first end 18 of which is open, and a second end of which, opposite the first, is closed by a transverse wall 16 comprising passages which emerge to form a grille 42. The transverse wall 16 separates the first housing 3 from a second housing 6, located above the first housing 3.

The upper housing 6 has a bottom 7, in the shape of a hemisphere, into which the passages forming the grille 42 emerge. One end of the housing 6, opposite the bottom 7, forms a free edge 8 delimiting an opening 9. The external surface of the housing 6 comprises a screw thread 10 intended to cooperate with a corresponding screw thread 11 provided for on the internal surface of a skirt 12 of a stopper 13. As a variant, the stopper is reversibly snap-fastened onto the external surface of the housing 6.

The stopper 13 is rigidly connected to an applicator 14 correspondingly made to match the hemispherical profile defined by the bottom 7 of the housing 6. The application member 14 may be adhesively bonded, welded or crimped onto the cap 13.

The application member consists of an open-cell polyurethane foam. The density of the foam forming the application member, measured according to standard ASTM D 3574-05, is advantageously between 0.02 $g.cm^{-3}$ and 0.05 $g.cm^{-3}$, for example equal to 0.03 $g.cm^{-3}$.

According to one preferred variant, at least one part of the surface 14 of the application member is covered with a flock 15, in particular based on rayon, cotton, viscose or nylon fibres. The length of the fibres is advantageously between 0.2 mm and 1 mm, for example 0.75 mm. The fibre count grading unit is advantageously between 0.3 dtex and 3.3 dtex, for example equal to 1.7 dtex.

The application member 14 is proportioned relative to the housing 6 such that, when the stopper 13 is in the closed position, at least one portion of the application surface of the application member 14 is in contact with the bottom 7.

The application member 14 is proportioned such that, in this position, it is not appreciably axially compressed.

The composition of the invention is here referred to as product P. This product P is contained inside the housing 3 forming a container, the open end 18 of the housing 3 being closed via a dispensing mechanism 30. The latter is mounted by clip-fastening onto the body 2 of the housing 3, after filling with the product P inside the housing 3 through its open end 18. The mechanism 30 comprises an actuating wheel 31 mounted to rotate freely with respect to the body 2, via a bulge/groove arrangement. The wheel 31 is rigidly connected to a threaded rod 32 capable of axially driving a threaded piston 33, incapable of rotating inside the container, for example by virtue of a rib/notch assembly which

prevents the piston from rotating inside the container. The mechanism may also comprise a metering means, such as a ratchet system, capable of periodically generating an audible sound, so as to inform the user of the amount of product dispensed.

As a variant, the mechanism associated with the first housing, for causing the product to exit, may be different; for example, the walls of the body 2 may be deformable in order to apply, by crimping, an overpressure in the container so as to expel the product through the grille.

To use this device, the user turns the actuating wheel through half or one turn, with the cap 13 in the closed position over the opening 9 of the housing 6, so as to cause a corresponding amount of product P to pass from the container to the applicator 14 contained in the housing 6, via the passage of the grille 42. The product is taken up by the applicator 14, in particular by capillary action. All that then remains is for the cap 13 to be unscrewed so that the application member can be extracted therefrom and for the product P to be applied by moving the application surface of the application member 14 over lips, in order to deposit the product impregnated in the application member.

According to a variant which is not illustrated, the application member 14 is axially oversized with respect to the housing 6. Thus, after having applied all the product contained on the application member 14, it is possible to reload the latter, without having to rescrew the cap fully onto the device 1, simply by introducing the application member 14 into the housing 6 through the opening 9, and picking up either residual product resulting from the previous actuating of the wheel 31, or product resulting from a further actuating of the wheel 31, in the absence of the application member 14.

According to a second embodiment represented in Figures 2 and 3, the device is in the form of an applicator bottle for a product P, and comprises mainly a container or reservoir 110 consisting of a body 111, one end of which is closed by a bottom 112. The other end of the reservoir 110 is surmounted by an application head which comprises an intermediate element 130 intended for mounting the head on the reservoir and an application member 120 housed in the intermediate element.

The intermediate element 130 comprises, on its external surface, means 132 (of screw thread or snap-fitting bead type) for enabling the removable mounting of a stopper 140 capable of covering the application member 120.

As a variant, a dispensing mechanism can be combined with the reservoir, for example a piston mechanism as previously described, or flexible walls making it possible to generate an overpressure in the reservoir in order to expel the product. Likewise, a ball 160 can be placed inside the reservoir so as to homogenize the product in order to facilitate the flow thereof and/or to facilitate the conveying thereof to the application member.

The internal wall of the intermediate element 130 defines a cylindrical internal housing 133 which rotates about an axis X. This housing 133 comprises a side wall 134 and a planar transverse wall 135 constituting the bottom of the housing. A fitting skirt 136 extends the side wall beyond the transverse wall, on the opposite side from the opening 131 of the intermediate element. The fitting skirt 136 is attached, by snap-fitting, onto the body of the reservoir, at the top of which a radial projection 114 is provided for, on the opposite side from the bottom 112.

This snap-fitting system can of course be replaced with any other system of attachment, in particular a screw attachment system. A sealing skirt 137 is advantageously provided for on the transverse wall 135 of the housing so as to come into leaktight contact with the inside of the reservoir.

The body 111 of the reservoir, the intermediate element 130 and also the stopper 140 are made of rigid material, for example of polyethylene. It is obvious that these three independent elements can each be made of a different material. It is thus possible to envisage using a flexible material for at least one part of the reservoir.

The housing 133 communicates with the inside of the reservoir via a passage 139 which passes through a shaft 115 extending along the axis of the housing. The shaft is made up of a single part with the transverse wall 135 from which it extends to a free end, located inside the housing 133. The shaft has a circular internal cross section, which is constant throughout its axial height, whereas its external cross section decreases to the free end.

The diameter of the shaft is chosen according to the product contained in the reservoir, in such a way that the product coming from the reservoir can flow in the shaft, for example by simply shaking the assembly.

The application member 120 is mounted inside the housing 133 around the shaft 115.

The application member is in the form of a block of porous material(s), at least one part of which is elastically compressible.

According to one preferred embodiment, the application member is made up of a block of open-cell foam, in particular a block of polyurethane foam. Alternatively, the application member 120 may be made up of an axial succession of at least two portions of foam, of different compressibilities, which can be adhesively bonded together.

According to the example represented, the application member has a cylindrical shape and has a circular cross section. It is obvious that the application member can have any other shape, and can have any other cross section.

The application member 120 has a side wall 121, one end 122 of which constitutes a dome-shaped application surface.

Where appropriate, the application surface 122 can be covered with a flock. In this case, the flock may consist of hairs of various diameters and/or of various natures and/or of various heights, or of a mixture of such hairs.

On the opposite side from this application surface 122, the application member ends with a second open end

124 which comes into contact with the transverse wall 135 of the housing. The end 124 can be permanently attached to the intermediate element 130. Advantageously, this end is removably attached so as to make it possible to easily remove the application member in order, for example, to clean it. To this effect, the end 124 of the application member is covered with a permanent adhesive, for example an acrylic adhesive, which adheres more to the application member than to the wall 135.

A portion 123 of the side wall located on the side of the open end 124 acts as a support for the application member. The portion 123 acting as a support for the application member is separated from the rest of the application member by an annular groove 150 which defines a zone of smaller cross section. The annular groove 150 provided for on the periphery of the application member enables the zone of smaller cross section to have a greater compressibility than the rest of the application member. Thus, when a pressure is exerted on the application surface, the maximum compression of the zone of smaller cross section is obtained before obtaining the maximum compression of the application member.

When the application member is mounted in the housing 133, it occupies approximately the entire housing, the application member having a shape approximately complementary to the shape of the housing. The application member 120 has in particular an axial recess 125, the shape of which is adjusted so that the application member comes to press against the wall of the shaft, without being substantially laterally compressed by said shaft. Alternatively, the side wall of the application member can be at a distance from the shaft. When the application member is in the relaxed position, the axial recess 125 has an axial height which is substantially greater than the axial height of the shaft 115 so as to define an internal cavity inside the application member, between an internal surface 126 located facing the shaft and the free end of the shaft. The portion 127 of the application member located above the cavity has an axial thickness which is smaller than the thickness of the lateral edge 121 of the application member, measured perpendicular to the axis X. The cavity can thus constitute a store of product in proximity to the application surface, it being possible for the product to come into contact with the application member only in the cavity.

Typically, the application member has a diameter of between 2 mm and 20 mm, and preferably between 5 mm and 15 mm. Its height, in the non-compressed position, can range between 2 mm and 20 mm.

Generally, the application member 120 comprises pores or open cells which have an average size of between 0.3 mm and 0.5 mm. Preferably, the pores or cells communicate with one another omnidirectionally.

In order to use the application assembly according to the invention, the user shakes the packaging and application assembly in order to bring the product into the shaft and into the application member. As a variant, the user actuates a dispensing mechanism in order to bring the product to the application member. Product is then kept inside the block of foam 120 of the application member by capillary action. All that is then needed is to bring the application member 120 into contact with the area to be treated; a slight pressure can be applied so as to place the product present in the cells of the foam in proximity to the application surface 122. Product is then spread by passing the application surface 122 over the support to be treated, by simple capillary contact, so as to draw the product in the form of a film, under the action of the affinity of the product that is exerted between the application surface and the support to be treated, without the slightest pressure being appreciably exerted on the application assembly.

In the detailed description above, reference was made to preferred embodiments of the invention. It is obvious that variants can be introduced therein without departing from the spirit of the invention as claimed hereinafter.

In particular, the shape of the applicator may be different from the shape illustrated with reference to the embodiments that have just been described. Generally, the shape of the applicator is chosen according to the area to be treated. For example, in the case of an applicator for the lips, an applicator approximately in the shape of a cone, a nose-cone or a hemisphere will more particularly be used, the applicator optionally comprising a bevelled face.

Likewise, the applicator can be dissociated from a container containing the product without a dispensing mechanism. The device is then used by dipping the applicator in the container and then by applying the product to the lips by means of the applicator.

The example below is presented as a non-limiting illustration of the field of the invention.

<u>Protocol for measuring the film thickness:</u>

This protocol is an *in vitro* measurement.

A square of Bioskin® synthetic skin of 3 cm × 4 cm was prepared and weighed accurately. The composition was applied by means of the device so as to obtain an even deposit covering the entire surface of the skin square. The skin square thus made up was weighed accurately.

The film thickness was calculated according to the formula:

$$\text{Thickness (cm)} = \text{volume of composition applied (cm}^3\text{)/surface area of the skin square}$$

with volume of the composition applied = mass of the deposit of composition (g)/density of the composition (g/cm$^3$)

The density of the composition is 1.

The average thickness is given with three separate measurements.

Protocol for evaluating the cosmeticity of the composition:

The composition is applied to the lips. The evaluation of the cosmeticity of the composition is visual. The tack is evaluated during drying. The gloss is evaluated 10 minutes after applying the composition to the lips and the migration is evaluated 1 hour after application.

## EXAMPLES

### EXAMPLES 1

The two compositions below are prepared:
In the table below, the amounts are expressed by weight of starting material.

| | | Amount (%) | |
|---|---|---|---|
| Phase | Ingredients | A | B |
| A | Dimethicone (and) dimethiconol (sold under the name Xiameter PMX-1503 Fluid by Dow Corning) | 4.0 | 4.0 |
| | Trimethylpentaphenyltrisiloxane (viscosity: 175 cSt) (sold under the name Dow Corning PH-1555 HRI Cosmetic Fluid by Dow Corning) | 5.0 | 5.0 |
| | Dimethicone (viscosity 2 cSt) | 3.9 | 3.9 |
| | Isohexadecane | 3.9 | 3.9 |
| | Sodium polyacrylate (sold under the name Cosmedia SP by Cognis) | 0.5 | 0.5 |
| B | Disteardimonium hectorite (sold under the name Bentone 38 VCG by Elementis) | 1.0 | 1.0 |
| | Propylene carbonate (sold under the name Jeffsol propylene carbonate by Huntsman) | 0.3 | 0.3 |
| | Dimethicone (viscosity 2 cSt) | 3.8 | 3.8 |
| C | Cetyl PEG/PPG-10/1 dimethicone (sold under the name Abil EM 90 by Evonik Goldschmidt) | 3.0 | 3.0 |
| | Dimethicone (and) PEG/PPG-18/18 dimethicone (sold under the name X-22-6711D by Shin-Etsu) | 1.0 | 1.0 |
| | Polyglyceryl-4 isostearate (sold under the name Isolan GI 34 by Evonik Goldschmidt) | 1.0 | 1.0 |
| | White mineral oil | 4.0 | 4.0 |
| | Bis-behenyl/isostearyl/phytosteryl dimer dilinoleyl dimer dilinoleate (sold under the name Plandool G-7 by Nippon Fine Chemical) | 5.0 | - |
| | PEG-45/dodecyl glycol copolymer (sold under the name Elfacos ST 9 by Akzo Nobel) | - | 5.0 |
| D | Acrylates/polytrimethylsiloxy methacrylate copolymer (sold under the name Dow Corning FA 4002 ID Silicone Acrylate by Dow Corning, as a 40% by weight mixture in isododecane) | 10.0 | 10.0 |
| E | 2-Octyldodecan-1-ol | 2.5 | 2.5 |
| | Pigments | 1.5 | 1.5 |
| | Polyhydroxystearic acid (sold under the name Dispersun DSP OL-300 by Innospec Active Chemicals) | 0.2 | 0.2 |

(continued)

| | | Amount (%) | |
|---|---|---|---|
| Phase | Ingredients | A | B |
| F | 1,3-Butylene glycol | 6.0 | 6.0 |
| | Magnesium sulfate | 0.7 | 0.7 |
| | Preserving agent | qs | qs |
| | Glycerol | 2.0 | 2.0 |
| | Water | qs | qs |
| G | Ethanol | 5.0 | 5.0 |

Preparation protocol:

A ground pigmentary material (phase E) is prepared by dispersing the pigments in the preheated 2-octyldodecan-1-ol and polyhydroxystearic acid, by means of an Exakt three-roll machine (at least three treatments).
Phase A is prepared by mixing the ingredients in a TM DAC 150.1 speed mixer from Hauschild, at 3500 rpm for 6 minutes.
Phase B is prepared by mixing the components in the speed mixer at 3500 rpm for 3 minutes.
Phase C is prepared by mixing the components (preheated pasty compound) in the speed mixer at 3500 rpm for 6 minutes.
Phase D is added to phase C and the mixture is homogenized in the speed mixer at 3500 rpm for 3 minutes.
Phase F is prepared by mixing with magnetic stirring in a beaker.
Phase B is added to phase A with stirring using a Moritz rotor-stator. After 10 minutes, phases C and D obtained are added, and, after homogenization, the ground material E is added, followed by phase F (slowly and after homogenization) and finally phase G, after homogenization.
The two compositions thus obtained are homogeneous and stable after a storage during 2 months at 25°C and 37°C.
The compositions are applied using the following two devices:

Device 1: application member consisting of S90NR foam from the company Crest Foam Industries, covered with a flock from the company ERZI Flock Technik (0.75 mm; 1.7 dtex).
Device 2: applicator for compositions of lip gloss type (flexible flocked applicator 14030; GEKA GMBH).

Protocol for measuring the film thickness:

This protocol is an *in vitro* measurement.
A square of Bioskin® synthetic skin of 3 cm x 4 cm is prepared and weighed accurately. The composition is applied by means of the device so as to obtain an even deposit covering the entire surface of the skin square. The skin square thus made up is weighed accurately.
The film thickness is calculated according to the formula:

$$\text{Thickness (cm)} = \text{volume of composition applied (cm}^3\text{)/surface area of the skin square}$$

with volume of the composition applied = mass of the deposit of composition (g)/density of the composition (g/cm$^3$)
The density of the composition is 1.
The average thickness is given with three separate measurements.

Protocol for evaluating the cosmeticity of the composition:

The composition is applied to the lips. The evaluation of the cosmeticity of the composition is visual. The tack is evaluated during drying. The gloss is evaluated 10 minutes after applying the composition to the lips and the migration is evaluated 1 hour after application.

Results:

A very thin deposit is obtained in both cases, the deposit being thinner with device 2 than with device 1, and the variation in thickness of the deposit between t0 and t1h is low.
The deposit is uniform in both cases, has a satiny appearance (slightly shiny) and the colour is homogeneous with appropriate resistance.
The application is gentle and easy, not drying too fast, giving a sensation of moisturization on application, with no tack. The deposit is comfortable during application and after (no pulling sensation).
The deposits migrate little (device 1) or not at all (device 2).

**EXEMPLE 2**

The following composition is prepared (the contents are expressed as weight of starting material).

| Phase | Ingredients | Amount (%) |
|---|---|---|
| A | Dimethicone (and) dimethiconol (sold under the name Xiameter PMX-1503 Fluid by Dow Corning) | 2.5 |
| | Trimethylpentaphenyltrisiloxane (viscosity: 175 cSt) (sold under the name Dow Corning PH-1555 HRI Cosmetic Fluid by Dow Corning) | 5.0 |
| | Dimethicone (viscosity 2 cSt) | 3.1 |
| | Isododecane | 2.7 |
| | Isohexadecane | 5.0 |
| B | Kaydol white mineral oil from Sonneborn | 4.0 |
| | Cetyl PEG/PPG-10/1 dimethicone (sold under the name Abil EM 90 by Evonik Goldschmidt) | 3.0 |
| | Dimethicone (and) PEG/PPG-18/18 dimethicone (sold under the name X-22-6711D by Shin-Etsu) | 1.0 |
| | Polyglyceryl-4 isostearate (sold under the name Isolan GI 34 by Evonik Goldschmidt) | 1.0 |
| C | Disteardimonium hectorite (sold under the name Bentone 38 VCG by Elementis) | 1.0 |
| | Dimethicone (viscosity 2 cSt) | 3.8 |
| | Propylene carbonate (sold under the name Jeffsol propylene carbonate by Huntsman) | 0.3 |
| D | Acrylates/polytrimethylsiloxy methacrylate copolymer (sold under the name Dow Corning FA 4002 ID Silicone Acrylate by Dow Corning, as a 40% by weight mixture in isododecane) | 10.0 |
| E | 2-Octyldodecan-1-ol | 2.5 |
| | Pigments | 1.5 |
| | Polyhydroxystearic acid (sold under the name Dispersun DSP OL-300 by Innospec Active Chemicals) | 0.2 |
| F | 1,3-Butylene glycol | 6.0 |
| | Magnesium sulfate | 0.7 |
| | Preserving agent | qs |
| | Glycerol | 2.0 |
| | Acacia honey | 1.0 |
| | Water | qs |
| G | Ethanol | 5.00 |

A ground pigmentary material (phase E) is prepared by dispersing the pigments in the preheated 2-octyldodecan-1-ol and polyhydroxystearic acid, by means of an Exakt three-roll machine (at least three treatments).

Phases A and B are prepared separately, by mixing the ingredients in a TM DAC 150.1 speed mixer from Hauschild, at 3500 rpm for 3 minutes.
Phase F is prepared by mixing with magnetic stirring in a beaker.
Phase B is mixed with phase A with stirring using a Moritz rotor-stator. After 10 minutes, phase C, phase E, then phase D are added successively, and after homogenization between each introduction. Phase F and finally phase G are then added slowly, after homogenization.
The compositions are homogeneous and stable (storage during 2 months at 25°C and 37°C).

Devices used for the application:

Device 1: application member consisting of S90NR foam from the company Crest Foam Industries covered with flocking from the company ERZI Flock Technik (0.75 mm; 1.7dtex).
Device 2: applicator for compositions of lip gloss type (flexible flocked applicator 14030; GEKA GmbH).
Very fine, homogeneous and comfortable (supple leaving the lips with a great freedom of movement, no pulling sensation) deposits are obtained, with a sensation of moisturization on application. The deposits have a satiny appearance (slightly shiny) and their colour is homogeneous with appropriate resistance.
The deposits migrate little and are sparingly tacky/non-tacky.

**EXAMPLE 3**

The following composition is prepared (the contents are expressed as weight of starting material).

| Phase | Ingredients | Amount (%) |
|---|---|---|
| A | Dimethicone (and) dimethiconol (sold under the name Xiameter PMX-1503 Fluid by Dow Corning) | 4.0 |
| | Trimethylpentaphenyltrisiloxane (viscosity: 175 cSt) (sold under the name Dow Corning PH-1555 HRI Cosmetic Fluid by Dow Corning) | 5.0 |
| | Dimethicone (viscosity 2 cSt) | 3.1 |
| | Isododecane | 2.7 |
| | Isohexadecane | 5.0 |
| B | Cetyl PEG/PPG-10/1 dimethicone (sold under the name Abil EM 90 by Evonik Goldschmidt) | 3.0 |
| | Dimethicone (and) PEG/PPG-18/18 dimethicone (sold under the name X-22-6711D by Shin-Etsu) | 1.0 |
| | Polyglyceryl-4 isostearate (sold under the name Isolan GI 34 by Evonik Goldschmidt) | 1.0 |
| C | Petroleum jelly (White Protopet 1s Petrolatum from Sonneborn) | 4.0 |
| D | Disteardimonium hectorite (sold under the name Bentone 38 VCG by Elementis) | 1.0 |
| | Dimethicone (viscosity 2 cSt) | 3.8 |
| | Propylene carbonate (sold under the name Jeffsol propylene carbonate by Huntsman) | 0.3 |
| E | Acrylates/polytrimethylsiloxy methacrylate copolymer (sold under the name Dow Corning FA 4002 ID Silicone Acrylate by Dow Corning, as a 40% by weight mixture in isododecane) | 10.0 |
| F | 2-Octyldodecan-1-ol | 2.5 |
| | Pigments | 1.5 |
| | Polyhydroxystearic acid (sold under the name Dispersun DSP OL-300 by Innospec Active Chemicals) | 0.2 |

(continued)

| Phase | Ingredients | Amount (%) |
|---|---|---|
| G | 1,3-Butylene glycol | 6.0 |
| | Magnesium sulfate | 0.7 |
| | Preserving agent | qs |
| | Glycerol | 2.0 |
| | Acacia honey | 1 |
| | Polyacrylamidomethylpropanesulfonic acid partially neutralized with aqueous ammonia (Hostacerin AMPS sold by Clariant) | 0.5 |
| | Water | qs |
| H | Ethanol | 5.0 |

A ground pigmentary material (phase D) is prepared by dispersing the pigments in the preheated 2-octyldodecan-1-ol and polyhydroxystearic acid, by means of an Exakt three-roll machine (at least three treatments).
Phases A, B and D are prepared separately, by mixing the ingredients in a TM DAC 150.1 speed mixer from Hauschild, at 3500 rpm for 3 minutes.
Phase G is prepared by mixing with magnetic stirring in a beaker.
Phase B is mixed with phase A with stirring using a Moritz rotor-stator. After 10 minutes, preheated phase C, then phase D, phase F and phase E are added successively, and after homogenization between each introduction. Phase G and finally phase H are then added slowly, after homogenization.
The composition obtained is homogeneous and stable (storage during 2 months at 25°C and 37°C).
It is applied using device 1.
The application is gentle and easy, not drying too fast,
**[0410]** The obtained deposit is very thin, non-tacky, uniform and comfortable (supple deposit on the lips leaving them with a great freedom of movement, no pulling sensation) It has a satiny appearance (slightly shiny) and the colour is homogeneous with appropriate resistance. Furthermore, the deposit remains located where it was applied, during and after the application (precise outline of the lips; no migration) and shows a good staying power.

**EXEMPLE 4**

The composition below is prepared:
In the table below, the amounts are expressed by weight of starting material.

| Phase | Ingredients | Amount (%) |
|---|---|---|
| A | Dimethicone (and) dimethiconol (sold under the name Xiameter PMX-1503 Fluid by Dow Corning) | 4.0 |
| | Trimethylpentaphenyltrisiloxane (viscosity: 175 cSt) (sold under the name Dow Corning PH-1555 HRI Cosmetic Fluid by Dow Corning) | 5.0 |
| | Dimethicone (viscosity 2 cSt) | 2.8 |
| | Isohexadecane | 5 |
| | Sodium polyacrylate (sold under the name Cosmedia SP by Cognis) | 0.6 |
| B | Disteardimonium hectorite (sold under the name Bentone 38 VCG by Elementis) | 1.6 |
| | Propylene carbonate (sold under the name Jeffsol propylene carbonate by Huntsman) | 0.48 |
| | Dimethicone (viscosity 2 cSt) | 3.75 |

(continued)

| Phase | Ingredients | Amount (%) |
|---|---|---|
| C | Cetyl PEG/PPG-10/1 dimethicone (sold under the name Abil EM 90 by Evonik Goldschmidt) | 3.0 |
| | Dimethicone (and) PEG/PPG-18/18 dimethicone (sold under the name X-22-6711D by Shin-Etsu) | 1.0 |
| | Polyglyceryl-4 isostearate (sold under the name Isolan GI 34 by Evonik Goldschmidt) | 1.0 |
| | White mineral oil | 4.0 |
| | Bis-behenyl/isostearyl/phytosteryl dimer dilinoleyl dimer dilinoleate (sold under the name Plandool G-7 by Nippon Fine Chemical) | 2.5- |
| | Orbignya Oleifera seed oil (CROPURE™ BABASSU-SS-(LK), by Croda) | 2 |
| D | Acrylates/polytrimethylsiloxy methacrylate copolymer (sold under the name Dow Corning FA 4002 ID Silicone Acrylate by Dow Corning, as a 40% by weight mixture in isododecane) | 10.0 |
| E | Pentaerythrityl tetraisostearate | 4.5 |
| | Pigments | 1.5 |
| F | 1,3-Butylene glycol | 6.0 |
| | Magnesium sulfate | 0.7 |
| | Preserving agent | qs |
| | Glycerol | 2.0 |
| | Water | qs |
| G | Ethanol | 5.0 |

The composition is prepared according to the protocol of example 1.

The composition obtained is homogeneous and stable (storage during 2 months at 25°C and 37°C).

The composition is applied with Device 1 (application member consisting of S90NR foam from the company Crest Foam Industries covered with flocking from the company ERZI Flock Technik (0.75 mm; 1.7dtex)).

The application is gentle and easy, not drying too fast,

The obtained deposit is very thin, non-tacky, uniform and comfortable (supple deposit on the lips leaving them with a great freedom of movement, no pulling sensation) It has a satiny appearance (slightly shiny) and the colour is homogeneous with appropriate resistance. Furthermore, the deposit remains located where it was applied, during and after the application (precise outline of the lips; no migration) and shows a good staying power.

**Claims**

1. Makeup and/or care composition in the form of an emulsion comprising:

   a. at least one film-forming polymer chosen from vinyl polymers comprising at least one carbosiloxane dendrimer-based unit, present in an active material content ranging from 1.5% to 15% by weight, relative to the total weight of the composition,
   b. at least 2% by weight of at least one non-volatile oil,
   c. at least one silicone gum chosen from polyorganosiloxanes with a weight-average molecular mass of at least 400 000 g/mol,
   d. water.

2. Composition according to Claim 1, in which the vinyl polymer comprises at least one carbosiloxane dendrimer-based unit of general formula:

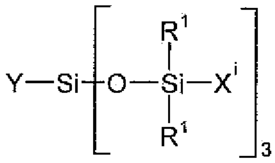

(I)

in which:

- $R^1$ represents an aryl group containing from 5 to 10 carbon atoms or an alkyl group containing from 1 to 10 carbon atoms;
- $X^i$ represents a silylalkyl group which, when i = 1, is represented by formula (II):

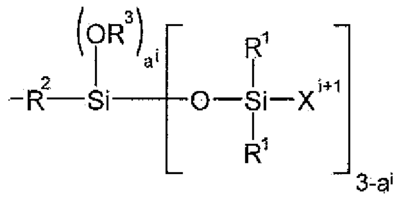

(II)

in which:

• $R^1$ is as defined above in formula (I),
• $R^2$ represents an alkylene radical containing from 2 to 10 carbon atoms,
• $R^3$ represents an alkyl group containing from 1 to 10 carbon atoms,
• $X^{i+i}$ is chosen from: a hydrogen atom, an alkyl group containing from 1 to 10 carbon atoms, an aryl group containing from 5 to 10 carbon atoms and a silylalkyl group defined above of formula (II) with i = i + 1,
• i is an integer from 1 to 10 which represents the generation of said silylalkyl group, and
• $a^i$ is an integer from 0 to 3;

- Y represents a radical-polymerizable organic group chosen from:

• organic groups containing a methacrylic group or an acrylic group, said organic groups being represented by the formulae:

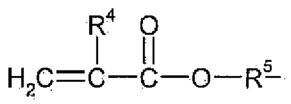

or

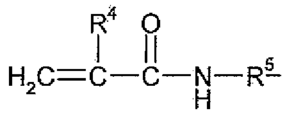

in which:

* $R^4$ represents a hydrogen atom or an alkyl group containing from 1 to 10 carbon atoms; and
* $R^5$ represents an alkylene group containing from 1 to 10 carbon atoms; and

• organic groups containing a styryl group of formula:

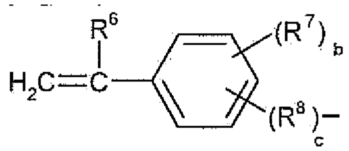

in which:

* $R^6$ represents a hydrogen atom or an alkyl group containing from 1 to 10 carbon atoms;

* $R^7$ represents an alkyl group containing from 1 to 10 carbon atoms;
* $R^8$ represents an alkylene group containing from 1 to 10 carbon atoms;
* b is an integer from 0 to 4; and
* c is 0 or 1, such that if c is 0, $-(R^8)c-$ represents a bond.

**3.** Composition according to any one of Claims 1 and 2, in which the carbosiloxane dendrimer is represented by the formula:

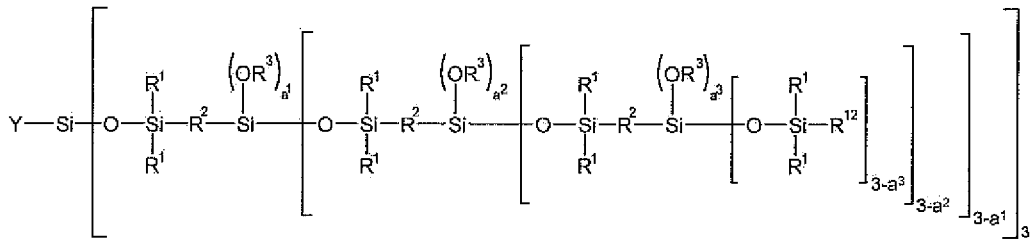

in which:

- Y, $R^1$, $R^2$, $R^3$, $a^1$, $a^2$ and $a^3$ are as defined in the preceding claim; and
- $R^{12}$ represents a hydrogen atom, an aryl group containing from 5 to 10 carbon atoms or an alkyl group containing from 1 to 10 carbon atoms.

**4.** Composition according to any one of Claims 1, 2 and 3, in which the carbosiloxane dendrimer is represented by one of the following formulae:

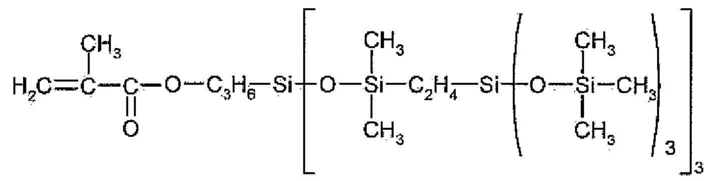

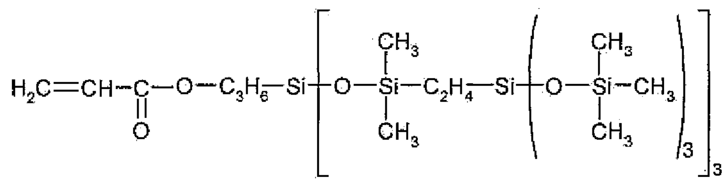

**5.** Composition according to any one of the preceding claims, in which the film-forming polymer chosen from vinyl polymers comprising at least one carbosiloxane dendrimer-based unit is present in an active material content ranging from 1.5% to 10% by weight and preferably from 3% to 5% by weight, relative to the total weight of the composition.

**6.** Composition according to any one of the preceding claims, **characterized in that** the non-volatile oil(s) are chosen from non-volatile silicone oils, which may or may not be phenylated, non-volatile fluoro oils, polar or apolar non-volatile hydrocarbon-based oils, or mixtures thereof; and preferably from non-volatile phenyl silicone oils, and from polar or apolar non-volatile hydrocarbon-based oils, and also mixtures thereof.

**7.** Composition according to any one of the preceding claims, **characterized in that** it comprises at least one non-volatile silicone oil, which is preferably phenylated, said non-volatile phenyl silicone oil preferably not bearing at least one dimethicone fragment.

**8.** Composition according to any one of the preceding claims, **characterized in that** the content of non-volatile oil(s) represents at least 2% by weight, more particularly from 3% to 30% by weight and even more particularly from 3% to 15% by weight, relative to the weight of the composition.

**9.** Composition according to any one of the preceding claims, **characterized in that** it comprises at least one volatile

oil chosen from hydrocarbon-based, silicone and fluoro volatile oils, and in particular chosen from branched $C_8$-$C_{16}$ alkanes, linear alkanes and linear or cyclic silicones.

10. Composition according to any one of the preceding claims, **characterized in that** the silicone gum is of the following formula:

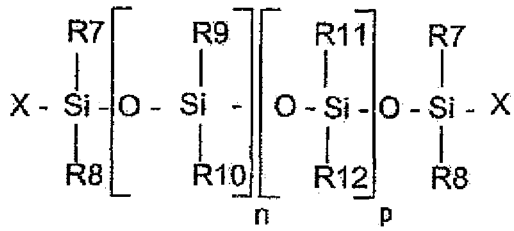

in which:

- R7, R8, R11 and R12 are identical or different, and each is chosen from alkyl radicals comprising from 1 to 6 carbon atoms,
- R9 and R10 are identical or different, and each is chosen from an alkyl radical comprising from 1 to 6 carbon atoms, an aryl radical, a hydroxyl radical, a vinyl radical, preferably an alkyl radical comprising from 1 to 6 carbon atoms, a hydroxyl radical;
- X is chosen from an alkyl radical comprising from 1 to 6 carbon atoms, a hydroxyl radical, a vinyl radical, preferably an alkyl radical comprising from 1 to 6 carbon atoms, a hydroxyl radical;
- n and p are chosen so as to give the silicone gum a molecular mass of greater than or equal to 400 000 g/mol, and in particular may each take values ranging from 0 to 5000, given that n and p are not simultaneously zero.

11. Composition according to any one of the preceding claims, **characterized in that** the silicone gum is a polydimethylsiloxane gum optionally comprising at least one aryl radical, a dimethiconol gum, or mixtures thereof, and preferably a dimethiconol gum.

12. Composition according to any one of the preceding claims, in which the silicone gum is present in an active material content ranging from 0.1% to 10% by weight, preferably from 0.2% to 8% by weight, in particular from 0.3% to 6% by weight, and more, relative to the total weight of the composition.

13. Composition according to any one of the preceding claims, **characterized in that** it comprises at least one surfactant chosen from nonionic or anionic, hydrocarbon-based or silicone surfactants, preferably from nonionic hydrocarbon-based surfactants, silicone surfactants particularly chosen from dimethicone copolyols, and alkyl dimethicone copolyols, and mixtures thereof.

14. Composition according to any one of the preceding claims, in which the composition comprises a water content of at least 10% by weight, preferably from 20% to 65% and more preferentially from 25% to 55% by weight relative to the total weight of the composition.

15. Composition according to any one of the preceding claims, **characterized in that** the composition comprises at least one saturated or unsaturated, linear or branched $C_2$-$C_8$ and preferably $C_3$-$C_6$ polyol that is liquid at room temperature (25°C), comprising from 2 to 6 hydroxyl groups.

16. Composition according to any one of the preceding claims, **characterized in that** the composition is in the form of a water-in-oil emulsion and is preferably liquid.

17. Composition according to any one of the preceding claims, **characterized in that** the composition comprises at least a pasty compound(s) chosen from hydrocarbon-based or silicon compounds, preferably from hydrocarbon-based compounds, in particular from lanolin and derivatives thereof, petroleum jelly, polyol ethers, vinyl polymers, liposoluble polyethers resulting from polyetherification between one or more $C_2$-$C_{100}$ diols, esters and polyesters, butters of plant origin, totally or partially hydrogenated plant oils, esters of hydrogenated castor oil, and mixtures thereof.

**18.** Composition according the preceding claim, in which the pasty compound(s) is/are present in a content of greater than 0.5% by weight, preferably in a content ranging from 2% to 20% by weight and in particular from 2% to 15% by weight relative to the total weight of the composition.

**19.** Process for making up and/or caring for the lips, comprising the application to said lips of a composition according to any one of the preceding claims.

**20.** Device comprising a container (3; 110) in which is stored the composition according to any one of the preceding claims and comprising an application member with a porous application surface (14; 120).

**21.** Device according to any one of the preceding claims, **characterized in that** it comprises a composition-dispensing mechanism (30) for expelling said composition from the container to the application member.

**Patentansprüche**

**1.** Schmink- und/oder Pflegezusammensetzung in Form einer Emulsion, umfassend:

a. mindestens ein filmbildendes Polymer, ausgewählt aus Vinylpolymeren mit mindestens einer auf Carbosiloxandendrimer basierenden Einheit, die in einem Wirkstoffgehalt im Bereich von 1,5 bis 15 Gew.-%, bezogen auf das Gesamtgewicht der Zusammensetzung, vorliegt,
b. mindestens 2 Gew.-% mindestens eines nichtflüchtigen Öls,
c. mindestens ein Silikongummi, das aus Polyorganosiloxanen mit einer gewichtsmittleren Molmasse von mindestens 400.000 g/mol ausgewählt ist,
d. Wasser.

**2.** Zusammensetzung nach Anspruch 1, wobei das Vinylpolymer mindestens eine auf Carbosiloxandendrimer basierende Einheit der allgemeinen Formel:

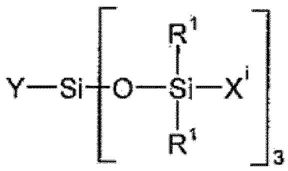
(I)

umfasst, wobei:

- $R^1$ für eine Arylgruppe mit 5 bis 10 Kohlenstoffatomen oder eine Alkylgruppe mit 1 bis 10 Kohlenstoffatomen steht;
- $X^1$ für eine Silylalkylgruppe steht, die im Fall von i = 1 durch Formel (II) wiedergegeben wird:

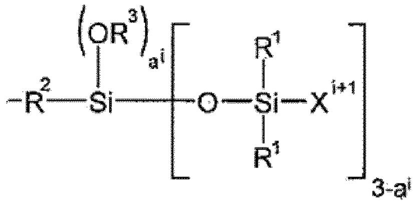
(II)

wobei:

• $R^1$ wie oben in Formel (I) definiert ist;
• $R^2$ für einen Alkylenrest mit 2 bis 10 Kohlenstoffatomen steht,
• $R^3$ für eine Alkylgruppe mit 1 bis 10 Kohlenstoffatomen steht,
• $X^{i+1}$ ausgewählt ist aus: einem Wasserstoffatom, einer Alkylgruppe mit 1 bis 10 Kohlenstoffatomen, einer

Arylgruppe mit 5 bis 10 Kohlenstoffatomen und einer oben definierten Silylalkylgruppe der Formel (II) mit i = i + 1,

- i eine ganze Zahl von 1 bis 10 ist, die die Generation der Silylalkylgruppe wiedergibt, und
- $a^i$ eine ganze Zahl von 0 bis 3 ist;

- Y für eine radikalisch polymerisierbare organische Gruppe steht, die aus:

• organischen Gruppen mit einer Methacrylgruppe oder einer Acrylgruppe, wobei die organischen Gruppen durch die Formeln:

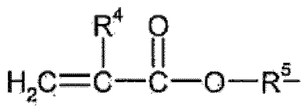
oder
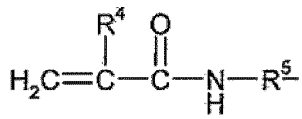

wiedergegeben werden, wobei:

* $R^4$ für ein Wasserstoffatom oder eine Alkylgruppe mit 1 bis 10 Kohlenstoffatomen steht und
* $R^5$ für eine Alkylengruppe mit 1 bis 10 Kohlenstoffatomen steht; und

• organischen Gruppen mit einer Styrylgruppe der Formel:

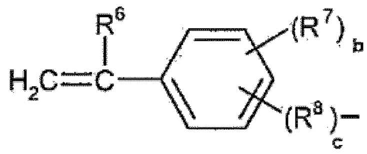

wobei:

* $R^6$ für ein Wasserstoffatom oder eine Alkylgruppe mit 1 bis 10 Kohlenstoffatomen steht;
* $R^7$ für eine Alkylgruppe mit 1 bis 10 Kohlenstoffatomen steht;
* $R^8$ für eine Alkylengruppe mit 1 bis 10 Kohlenstoffatomen steht;
* b eine ganze Zahl von 0 bis 4 ist und
* c für 0 oder 1 steht, so dass dann, wenn c gleich 0 ist, $-(R^8)_c-$ für eine Bindung steht; ausgewählt ist.

**3.** Zusammensetzung nach einem der Ansprüche 1 und 2, wobei das Carbosiloxandendrimer durch die folgende Formel wiedergegeben wird:

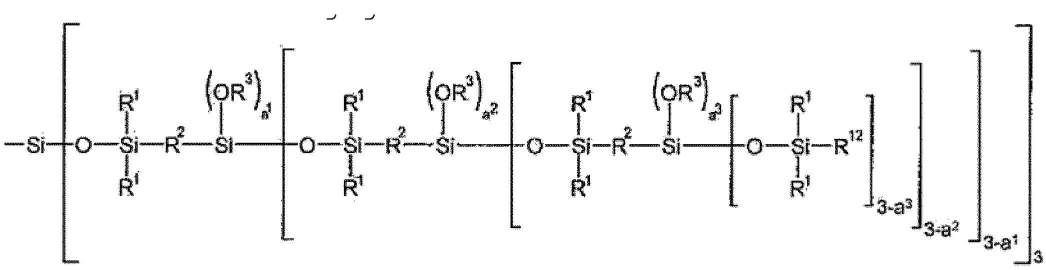

wobei:

- Y, $R^1$, $R^2$, $R^3$, $a^1$, $a^2$ und $a^3$ wie im vorhergehenden Anspruch definiert sind und
- $R^{12}$ für ein Wasserstoffatom, eine Arylgruppe mit 5 bis 10 Kohlenstoffatomen oder eine Alkylgruppe mit 1 bis 10 Kohlenstoffatomen steht.

**4.** Zusammensetzung nach einem der Ansprüche 1, 2 und 3, wobei das Carbosiloxandendrimer durch eine der folgenden Formeln wiedergegeben wird:

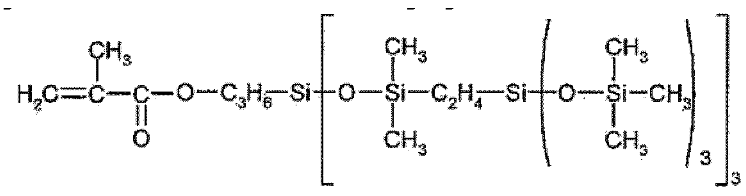

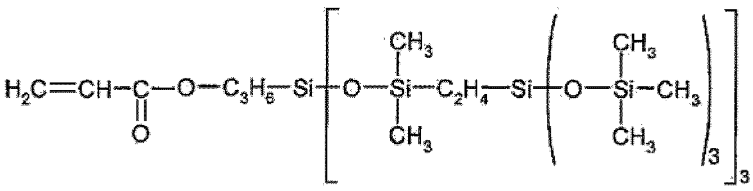

5. Zusammensetzung nach einem der vorhergehenden Ansprüche, wobei das filmbildende Polymer, das aus Vinylpolymeren mit mindestens einer auf Carbosiloxandendrimer basierenden Einheit ausgewählt ist, in einem Wirkstoffgehalt im Bereich von 1,5 bis 10 Gew.-% und vorzugsweise von 3 bis 5 Gew.-%, bezogen auf das Gesamtgewicht der Zusammensetzung, vorliegt.

6. Zusammensetzung nach einem der vorhergehenden Ansprüche, **dadurch gekennzeichnet, dass** das nichtflüchtige Öl bzw. die nichtflüchtigen Öle aus nichtflüchtigen Silikonölen, die gegebenenfalls phenyliert sind, nichtflüchtigen Fluorölen, polaren oder unpolaren nichtflüchtigen Ölen auf Kohlenwasserstoffbasis oder Mischungen davon und vorzugsweise aus nichtflüchtigen Phenylsilikonölen und polaren oder unpolaren nichtflüchtigen Ölen auf Kohlenwasserstoffbasis sowie Mischungen davon ausgewählt ist bzw. sind.

7. Zusammensetzung nach einem der vorhergehenden Ansprüche, **dadurch gekennzeichnet, dass** sie mindestens ein nichtflüchtiges Silikonöl, das vorzugsweise phenyliert ist, umfasst, wobei das nichtflüchtige Phenylsilikonöl vorzugsweise nicht mindestens ein Dimethicon-Fragment trägt.

8. Zusammensetzung nach einem der vorhergehenden Ansprüche, **dadurch gekennzeichnet, dass** der Gehalt an nichtflüchtigem Öl bzw. nichtflüchtigen Ölen mindestens 2 Gew.-%, spezieller 3 bis 30 Gew.-% und noch spezieller 3 bis 15 Gew.-%, bezogen auf das Gewicht der Zusammensetzung, ausmacht.

9. Zusammensetzung nach einem der vorhergehenden Ansprüche, **dadurch gekennzeichnet, dass** sie mindestens ein flüchtiges Öl umfasst, das aus flüchtigen Ölen auf Kohlenwasserstoffbasis und flüchtigen Silikon- und Fluorölen und insbesondere aus verzweigten $C_8$-$C_{16}$-Alkanen, linearen Alkanen und linearen oder cyclischen Silikonen ausgewählt ist.

10. Zusammensetzung nach einem der vorhergehenden Ansprüche, **dadurch gekennzeichnet, dass** das Silikongummi die folgende Formel aufweist:

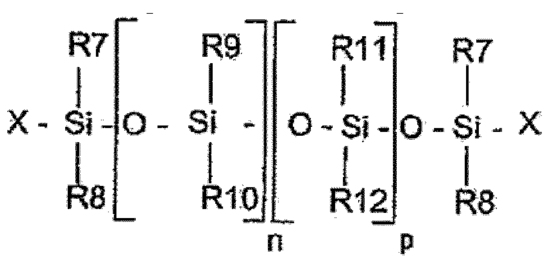

wobei:

- R7, R8, R11 und R12 gleich oder verschieden sind und jeweils aus Alkylresten mit 1 bis 6 Kohlenstoffatomen ausgewählt sind;
- R9 und R10 gleich oder verschieden sind und jeweils aus einem Alkylrest mit 1 bis 6 Kohlenstoffatomen, einem Arylrest, einem Hydroxylrest und einem Vinylrest, vorzugsweise einem Alkylrest mit 1 bis 6 Kohlenstoffatomen und einem Hydroxylrest, ausgewählt sind;
- X aus einem Alkylrest mit 1 bis 6 Kohlenstoffatomen, einem Hydroxylrest und einem Vinylrest, vorzugsweise einem Alkylrest mit 1 bis 6 Kohlenstoffatomen und einem Hydroxylrest, ausgewählt ist;
- n und p so gewählt sind, dass das Silikongummi eine Molmasse größer oder gleich 400.000 g/mol aufweist, und insbesondere jeweils Werte im Bereich von 0 bis 5000 annehmen können, mit der Maßgabe, dass n und p nicht gleichzeitig gleich null sind.

**11.** Zusammensetzung nach einem der vorhergehenden Ansprüche, **dadurch gekennzeichnet, dass** es sich bei dem Silikongummi um ein Polydimethylsiloxangummi, das gegebenenfalls mindestens einen Arylrest umfasst, ein Dimethiconolgummi oder Mischungen davon und vorzugsweise ein Dimethiconolgummi handelt.

**12.** Zusammensetzung nach einem der vorhergehenden Ansprüche, wobei das Silikongummi in einem Wirkstoffgehalt im Bereich von 0,1 bis 10 Gew.-%, vorzugsweise von 0,2 bis 8 Gew.-%, insbesondere von 0,3 bis 6 Gew.-%, und mehr, bezogen auf das Gesamtgewicht der Zusammensetzung, vorliegt.

**13.** Zusammensetzung nach einem der vorhergehenden Ansprüche, **dadurch gekennzeichnet, dass** sie mindestens ein Tensid umfasst, das aus nichtionischen oder anionischen Tensiden, Tensiden auf Kohlenwasserstoffbasis oder Silikontensiden, vorzugsweise aus nichtionischen Tensiden auf Kohlenwasserstoffbasis, Silikontensiden, die insbesondere aus Dimethiconcopolyolen und Alkyldimethiconcopolyolen ausgewählt sind, und Mischungen davon ausgewählt ist.

**14.** Zusammensetzung nach einem der vorhergehenden Ansprüche, wobei die Zusammensetzung einen Wassergehalt von mindestens 10 Gew.-%, vorzugsweise von 20 bis 65 Gew.-% und weiter bevorzugt von 25 bis 55 Gew.-%, bezogen auf das Gesamtgewicht der Zusammensetzung, umfasst.

**15.** Zusammensetzung nach einem der vorhergehenden Ansprüche, **dadurch gekennzeichnet, dass** die Zusammensetzung mindestens ein bei Raumtemperatur (25 °C) flüssiges gesättigtes oder ungesättigtes, lineares oder verzweigtes $C_2$-$C_8$- und vorzugsweise $C_3$-$C_6$-Polyol mit 2 bis 6 Hydroxylgruppen umfasst.

**16.** Zusammensetzung nach einem der vorhergehenden Ansprüche, **dadurch gekennzeichnet, dass** die Zusammensetzung in Form einer Wasser-in-Öl-Emulsion vorliegt und vorzugsweise flüssig ist.

**17.** Zusammensetzung nach einem der vorhergehenden Ansprüche, **dadurch gekennzeichnet, dass** die Zusammensetzung mindestens eine pastöse Verbindung umfasst, die aus Verbindungen auf Kohlenwasserstoffbasis oder Siliciumverbindungen, vorzugsweise aus Verbindungen auf Kohlenwasserstoffbasis, insbesondere aus Lanolin und Derivaten davon, Vaseline, Polyethern, Vinylpolymeren, fettlöslichen Polyethern, die sich aus der Polyveretherung zwischen einem oder mehreren $C_2$-$C_{100}$-Diolen ergeben, Estern und Polyestern, Buttern pflanzlicher Herkunft, vollständig oder teilweise hydrierten Pflanzenölen, Estern von hydriertem Ricinusöl und Mischungen davon, ausgewählt sind.

**18.** Zusammensetzung nach dem vorhergehenden Anspruch, wobei die pastöse Verbindung bzw. die pastösen Verbindungen in einem Gehalt von mehr als 0,5 Gew.-%, vorzugsweise in einem Gehalt im Bereich von 2 bis 20 Gew.-% und insbesondere von 2 bis 15 Gew.-%, bezogen auf das Gesamtgewicht der Zusammensetzung, vorliegt bzw. vorliegen.

**19.** Verfahren zum Schminken und/oder Pflegen für die Lippen, bei dem man auf die Lippen eine Zusammensetzung nach einem der vorhergehenden Ansprüche aufbringt.

**20.** Vorrichtung mit einem Behälter (3; 110), in dem die Zusammensetzung nach einem der vorhergehenden Ansprüche aufbewahrt wird, und mit einem Applikationselement mit einer porösen Applikationsoberfläche (14; 120).

**21.** Vorrichtung nach einem der vorhergehenden Ansprüche, **dadurch gekennzeichnet, dass** sie einen Zusammensetzungsabgabemechanismus (30) zum Ausstoßen der Zusammensetzung aus dem Behälter zum Applikationselement aufweist.

## Revendications

**1.** Composition de maquillage et/ou de soin sous forme d'une émulsion comprenant :

a. au moins un polymère filmogène choisi parmi les polymères vinyliques comprenant au moins un motif dérivé de dendrimère carbosiloxane, présent en une teneur en matière active allant de 1,5 à 15% en poids, par rapport au poids total de la composition,
b. au moins 2 % en poids d'au moins une huile non volatile,
c. au moins une gomme de silicone choisie parmi les polyorganosiloxanes de masse moléculaire moyenne en poids d'au moins 400 000 g/mol,
d. de l'eau.

**2.** Composition selon la revendication 1, dans laquelle le polymère vinylique comprend au moins un motif dérivé de dendrimère carbosiloxane de formule générale :

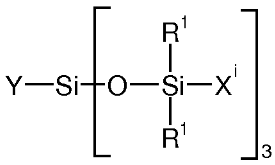

(I)

dans laquelle :

- $R^1$ représente un groupe aryle de 5 à 10 atomes de carbone ou un groupe alkyle de 1 à 10 atomes de carbone ;
- $X^i$ représente un groupe silylalkyle qui, lorsque i = 1, est représenté par la formule (II) :

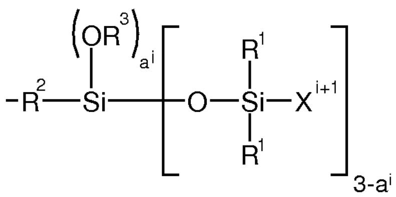

(II)

dans laquelle :

• $R^1$ est tel que défini ci-dessus dans la formule (I),
• $R^2$ représente un radical alkylène de 2 à 10 atomes de carbone,
• $R^3$ représente un groupe alkyle de 1 à 10 atomes de carbone,
• $X^{i+1}$ est choisi parmi : un atome d'hydrogène, un groupe alkyle de 1 à 10 atomes de carbone, un groupe aryle de 5 à 10 atomes de carbone et un groupe silylalkyle défini ci-dessus de formule (II) avec i = i + 1,
• i est un nombre entier de 1 à 10 qui représente la génération dudit groupe silylalkyle, et
• $a^i$ est un nombre entier de 0 à 3 ;

- Y représente un groupe organique polymérisable par voie radicalaire choisi parmi :

• des groupes organiques contenant un groupe méthacrylique ou un groupe acrylique, lesdits groupes organiques étant représentés par les formules :

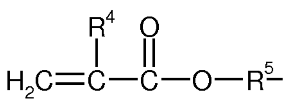

ou

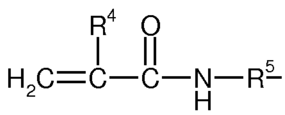

dans lesquelles :

* $R^4$ représente un atome d'hydrogène ou un groupe alkyle de 1 à 10 atomes de carbone ; et
* $R^5$ représente un groupe alkylène de 1 à 10 atomes de carbone ; et

• des groupes organiques contenant un groupe styryle de formule :

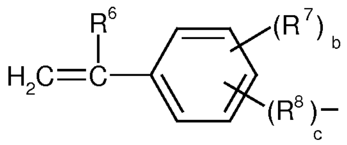

dans laquelle :

* $R^6$ représente un atome d'hydrogène ou un groupe alkyle de 1 à 10 atomes de carbone ;
* $R^7$ représente un groupe alkyle de 1 à 10 atomes de carbone ;
* R8 représente un groupe alkylène de 1 à 10 atomes de carbone ;
* b est un nombre entier de 0 à 4 ; et
* c vaut 0 ou 1, de sorte que si c vaut 0, -($R^8$)c-représente une liaison.

**3.** Composition selon l'une quelconque des revendications 1 ou 2, dans laquelle le dendrimère carbosiloxane est représenté par la formule :

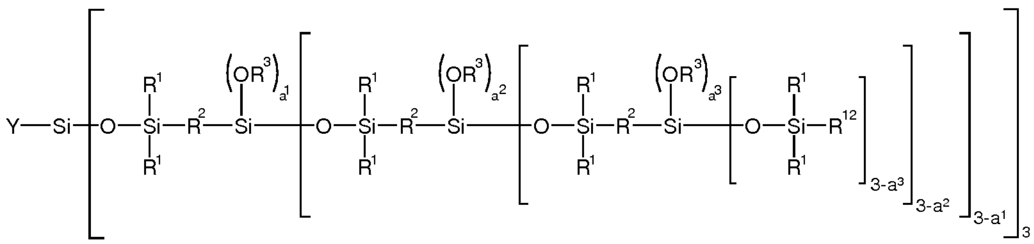

dans laquelle :

- Y, $R^1$, $R^2$, $R^3$, $a^1$, $a^2$ et $a^3$ sont tels que définis dans la revendication précédente ; et
- $R^{12}$ représente un atome d'hydrogène, un groupe aryle ayant 5 à 10 atomes de carbone ou un groupe alkyle ayant 1 à 10 atomes de carbone.

**4.** Composition selon l'une quelconque des revendications 1, 2 ou 3, dans laquelle le dendrimère carbosiloxane est représenté par l'une des formules suivantes :

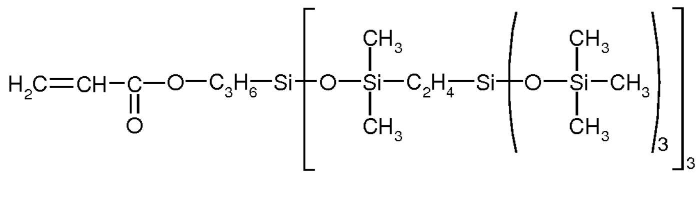

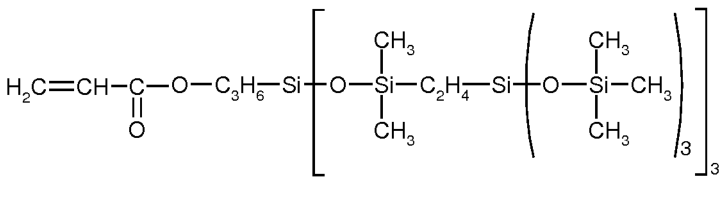

**5.** Composition selon l'une quelconque des revendications précédentes, dans laquelle le polymère filmogène choisi parmi les polymères vinyliques comprenant au moins un motif dérivé de dendrimère carbosiloxane est présent en une teneur en matière active allant de 1,5 à 10% en poids, et de préférence de 3 à 5% en poids, par rapport au poids total de la composition.

**6.** Composition selon l'une quelconque des revendications précédentes, **caractérisée en ce que** la ou les huiles non volatiles sont choisies parmi les huiles siliconées non volatiles, phénylées ou non ; les huiles fluorées non volatiles ; les huiles hydrocarbonées non volatiles, polaires ou apolaires, ou leurs mélanges ; et de préférence parmi les huiles siliconées non volatiles phénylées, parmi les huiles hydrocarbonées non volatiles polaires ou apolaires, ainsi que leurs mélanges.

**7.** Composition selon l'une quelconque des revendications précédentes, **caractérisée en ce qu'**elle comprend au moins une huile de silicone non volatile qui est de préférence phénylée, ladite huile de silicone non volatile phénylée ne portant de préférence pas au moins un fragment diméthicone.

**8.** Composition selon l'une quelconque des revendications précédentes, **caractérisée en ce que** la teneur en huile(s) non volatile(s) représente au moins 2 % en poids, plus particulièrement de 3 à 30% en poids, encore plus particulièrement de 3 à 15 % en poids, par rapport au poids de la composition.

**9.** Composition selon l'une quelconque des revendications précédentes, **caractérisée en ce qu'**elle comprend au moins une huile volatile choisie parmi les huiles volatiles hydrocarbonées, siliconées ou fluorées, et en particulier choisies parmi les alcanes ramifiés en $C_8$-$C_{16}$, les alcanes linéaires, les silicones linéaires ou cycliques.

**10.** Composition selon l'une quelconque des revendications précédentes, **caractérisée en ce que** la gomme de silicone est de formule suivante :

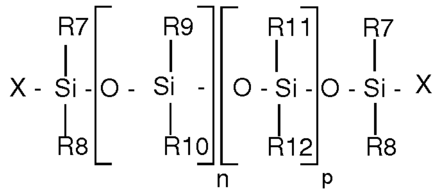

dans laquelle :

- R7, R8, R11 et R12 sont identiques ou différents, et chacun est choisi parmi les radicaux alkyle comprenant de 1 à 6 atomes de carbone,
- R9 et R10 sont identiques ou différents, et chacun est choisi parmi un radical alkyle comprenant de 1 à 6 atomes de carbone, un radical aryle, un radical hydroxyle, un radical vinyle, de préférence un radical alkyle comprenant de 1 à 6 atomes de carbone, ou un radical hydroxyle ;
- X est choisi parmi un radical alkyle comprenant de 1 à 6 atomes de carbone, un radical hydroxyle, un radical vinyle, de préférence un radical alkyle comprenant de 1 à 6 atomes de carbone, ou un radical hydroxyle ;
- n et p sont choisis de façon à conférer à la gomme de silicone une masse moléculaire supérieure ou égale à 400 000 g/mol, et en particulier peuvent chacun prendre des valeurs allant de 0 à 5000, sachant que n et p ne sont pas nuls simultanément.

**11.** Composition selon l'une quelconque des revendications précédentes, **caractérisée en ce que** la gomme de silicone est une gomme de polydiméthylsiloxane comprenant éventuellement au moins un radical aryle, une gomme diméthiconol, ou leurs mélanges, et de préférence une gomme diméthiconol.

**12.** Composition selon l'une quelconque des revendications précédentes, dans laquelle la gomme de silicone est présente en une teneur en matière active allant de 0,1 à 10% en poids, de préférence de 0,2 à 8% en poids, en particulier de 0,3 à 6% en poids, et plus, par rapport au poids total de la composition.

**13.** Composition selon l'une quelconque des revendications précédentes, **caractérisée en ce qu'**elle comprend au moins un tensioactif choisi parmi les tensioactifs non ioniques ou anioniques, hydrocarbonés ou siliconés, de préférence parmi les tensioactifs hydrocarbonés non ioniques, les tensioactifs siliconés particulièrement choisis parmi les diméthicones copolyols, les alkyldiméthicones copolyols, ou leurs mélanges.

**14.** Composition selon l'une quelconque des revendications précédentes, dans laquelle la composition comprend une teneur en eau d'au moins 10% en poids, de préférence de 20 à 65% et plus préférentiellement de 25 à 55% en poids, par rapport au poids total de la composition.

**15.** Composition selon l'une quelconque des revendications précédentes, **caractérisée en ce que** la composition comprend au moins un polyol liquide à température ambiante (25°C), en $C_2$-$C_8$, de préférence en $C_3$-$C_6$, saturé ou non, linéaire ou ramifié, comprenant de 2 à 6 groupements hydroxyle.

**16.** Composition selon l'une quelconque des revendications précédentes, **caractérisée en ce que** la composition se présente sous la forme d'une émulsion eau dans huile, et est de préférence liquide.

**17.** Composition selon l'une quelconque des revendications précédentes, **caractérisée en ce que** la composition comprend au moins un composé pâteux choisi(s) parmi des composés à base d'hydrocarbures ou de silicone, de préférence des composés à base d'hydrocarbures, en particulier parmi la lanoline et ses dérivés, la gelée de pétrole, les poyoléthers, les polymères vinyliques, les polyéthers liposolubles résultant de la polyétherification entre un ou plusieurs diols $C_2$-$C_{100}$, des esters et des polyesters, des beurres d'origine végétale, des huiles végétales entièrement ou partiellement hydrogénées, des esters d'huile de ricin hydrogénée et leurs mélanges.

**18.** Composition selon la revendication précédente, dans laquelle le ou les composés pâteux est/sont présent(s) dans une teneur supérieure à 0,5% en poids, de préférence dans une teneur comprise entre 2% et 20% en poids et en particulier entre 2% et 15% en poids par rapport au poids total de la composition.

**19.** Procédé de maquillage et/ou de soin des lèvres comprenant l'application sur lesdites lèvres d'une composition selon l'une quelconque des revendications précédentes.

**20.** Dispositif comprenant un récipient (3 ; 110) dans lequel est stockée la composition selon l'une quelconque des revendications précédentes et comprenant un organe d'application présentant une surface d'application poreuse (14 ; 120).

**21.** Dispositif selon l'une quelconque des revendications précédentes, **caractérisé en ce qu'**il comporte un mécanisme de distribution (30) de la composition permettant d'expulser ladite composition du récipient vers l'organe d'application.

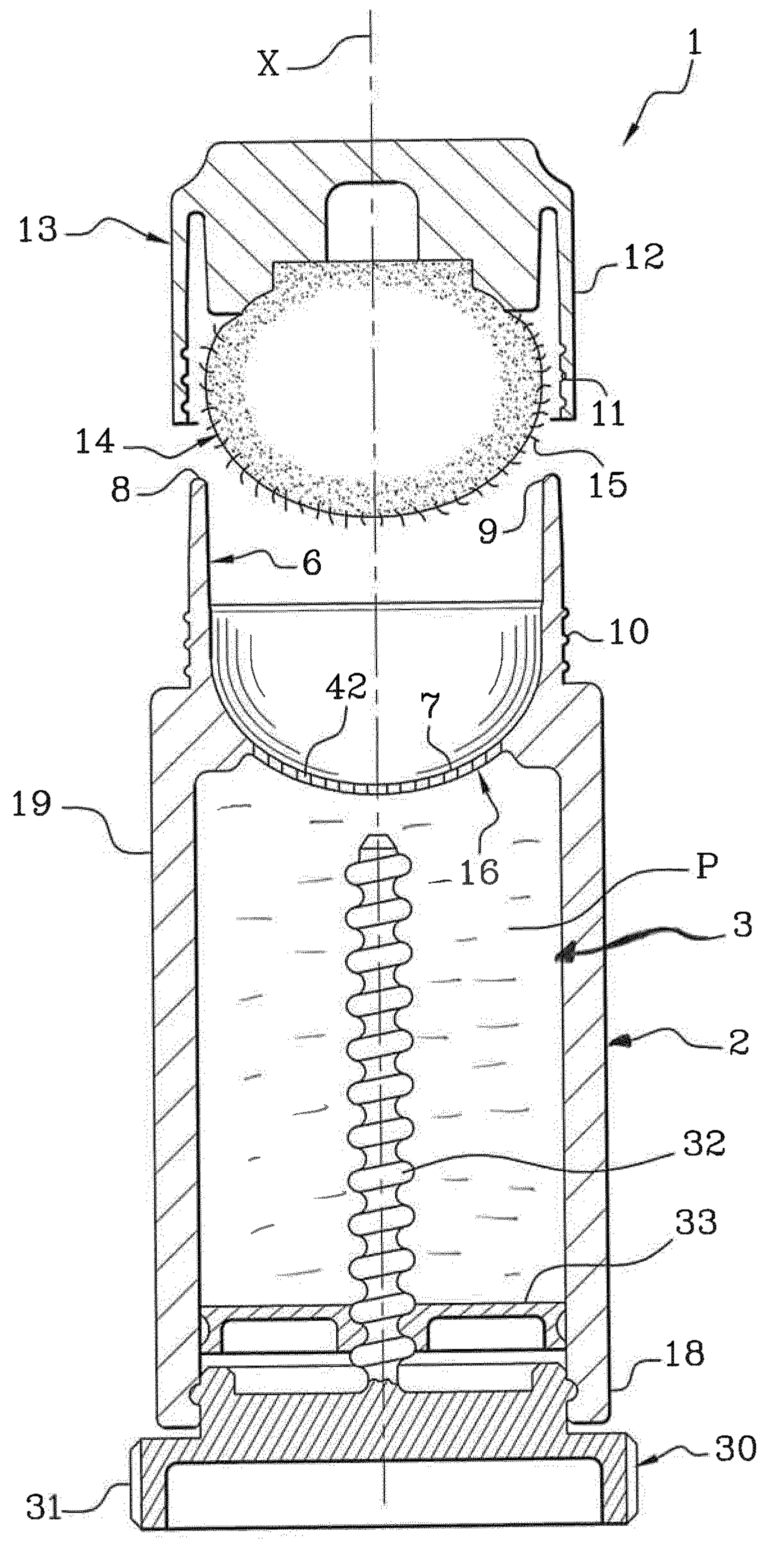


Fig. 1

Fig. 2

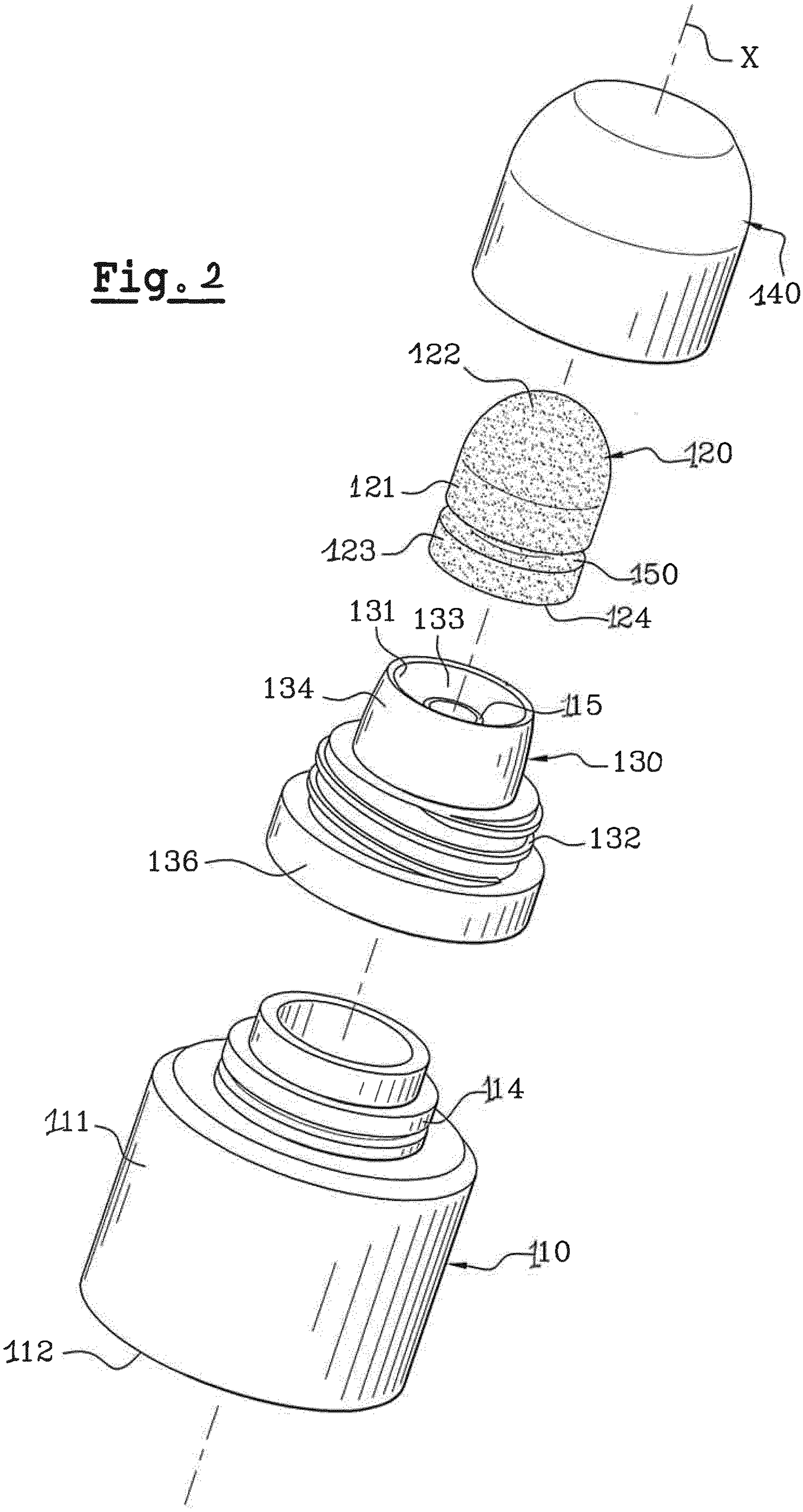
X
140
122
120
121
123
150
124
131
133
134
15
130
132
136
114
111
110
112

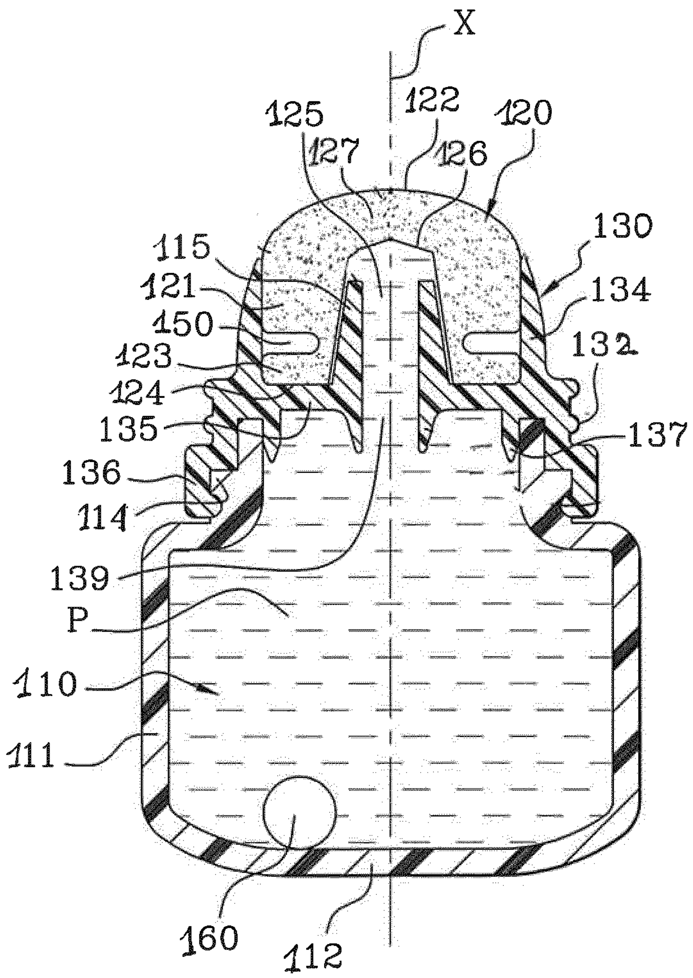
X
125
122
120
127
126
130
115
134
121
150
132
123
124
137
135
136
114
139
P
110
111
160
112

Fig. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 9171154 A
- JP HEI9171154 B
- EP 0963751 A
- EP 1055674 A
- WO 03045337 A
- FR 0853634
- EP 847752 A
- US 5412004 A
- US 5811487 A
- WO 2008155059 A
- EP 1086683 A


### Non-patent literature cited in the description


- **C.M. HANSEN.** The three-dimensional solubility parameters. *J. Paint Technol.,* 1967, vol. 39, 105
- Encyclopaedia of Chemical Technology, Kirk-Othmer. Wiley, 1979, vol. 22, 333-432
- *J. Soc. Cosm. Chem.,* 1954, vol. 5, 249-256
- International Cosmetic Ingredient Dictionary and Handbook. The Cosmetic, Toiletries and Fragrance Association, 1997, 371-386, 524-528